# EUROPEAN PATENT SPECIFICATION

(11) **EP 1 851 250 B1**
(45) Date of publication and mention of the grant of the patent: **06.06.2012**
(21) Application number: 06735489.4
(22) Date of filing: 17.02.2006
(51) Int. Cl.: C07K 16/28, A61P 13/08

(54) **HUMAN MONOCLONAL ANTIBODY TO PROSTATE SPECIFIC MEMBRANE ANTIGEN (PSMA)**
MENSCHLICHER MONOKLONALER ANTIKÖRPER GEGEN EIN PROSTATASPEZIFISCHES MEMBRANANTIGEN
ANTICORPS MONOCLONAL HUMAIN DIRIGE CONTRE L'ANTIGENE D'ENVELOPPE SPECIFIQUE DE LA PROSTATE

(30) Priority: 18.02.2005 US 654125 P; 26.09.2005 US 720499 P; 08.12.2005 US 748417 P
(43) Date of publication of application: 07.11.2007
(73) Proprietor: Medarex, Inc., Princeton, NJ 08543-4000 (US)
(72) Inventor: HUANG, Haichun, Fremont, California 94538 (US); KING, David, John, Belmont, California 94002 (US); PAN, Chin, Los Altos, California 94024 (US); CARDARELLI, Josephine, M., San Carlos, California 94070 (US)
(74) Representative: Woods, Geoffrey Corlett
(86) International application number: PCT/US2006/005852
(87) International publication number: WO 2006/089230

(56) References cited:
- WO-A-01/09192
- WO-A-03/064606
- US-B1- 6 290 956
- SUNDARAPANDIYAN K ET AL: "BISPECIFIC ANTIBODY-MEDIATED DESTRUCTION OF PROSTATE CANCER CELLS" PROCEEDINGS OF THE ANNUAL MEETING OF THE AMERICAN ASSOCIATION FOR CANCER RESEARCH, NEW YORK, NY, US, vol. 41, no. 41, March 2000 (2000-03), page 289, XP000941671 ISSN: 0197-016X
- HOLMES E H: "PSMA specific antibodies and their diagnostic and therapeutic use" EXPERT OPINION ON INVESTIGATIONAL DRUGS, ASHLEY PUBLICATIONS LTD., LONDON, GB, vol. 10, no. 3, 2001, pages 511-519, XP002988381 ISSN: 1354-3784

### SEQUENCE LISTING

<110> MEDAREX, INC.
<120> HUMAN MONOCLONAL ANTIBODIES TO PROSTATE SPECIFIC MEMBRANE ANTIGEN (PSMA)
<130> MXI-334PC
<140>
   <141>
<150> 60/748,417
   <151> 2005-12-08
<160> 48
<170> Patent In Ver. 3.3
<210> 1
   <211> 124
   <212> PRT
   <213> Homo sapiens
<400> 1
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Asn Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ala Val Pro Trp Gly Ser Arg Tyr Tyr Tyr Tyr Gly Met Asp
               100                 105                 110
   Val Trp Gly Gln Gly Thr Thr Val Thr Val Ser Ser
           115                 120
<210> 2
   <211> 119
   <212> PRT
   <213> Homo sapiens
<400> 2
   Glu Val Gln Leu Val Gln Ser Gly Ala Glu Val Lys Lys Pro Gly Glu
    1               5                  10                  15
   Ser Leu Lys Ile Ser Cys Lys Gly Ser Gly Tyr Ser Phe Thr Ser Asn
               20                  25                  30
   Trp Ile Gly Trp Val Arg Gln Met Pro Gly Lys Gly Leu Glu Trp Met
           35                  40                  45
   Gly Ile Ile Tyr Pro Gly Asp Ser Asp Thr Arg Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Gln Thr Gly Phe Leu Trp Ser Ser Asp Leu Trp Gly Arg Gly
               100                 105                 110
   Thr Leu Val Thr Val Ser Ser
           115
<210> 3
   <211> 119
   <212> PRT
   <213> Homo sapiens
<400> 3
   Glu Val Gln Leu Val Gln Ser Gly Ala Glu Val Lys Lys Pro Gly Glu
    1               5                  10                  15
   Ser Leu Lys Ile Ser Cys Lys Gly Ser Gly Tyr Ser Phe Thr Ser Asn
               20                  25                  30
   Trp Ile Gly Trp Val Arg Gln Met Pro Gly Lys Gly Leu Glu Trp Met
           35                  40                  45
   Gly Ile Ile Tyr Pro Gly Asp Ser Asp Thr Arg Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Asn Ser Leu Lys Ala Ser Asp Thr Ala Met Tyr Tyr Cys
                   85                  90                  95
   Ala Arg Gln Thr Gly Phe Leu Trp Ser Phe Asp Leu Trp Gly Arg Gly
               100                 105                 110
   Thr Leu Val Thr Val Ser Ser
           115
<210> 4
   <211> 121
   <212> PRT
   <213> Homo sapiens
<400> 4
   Glu Val Gln Leu Val Gln Ser Gly Ser Glu Val Lys Lys Pro Gly Glu
   1 5 10 15
   Ser Leu Lys Ile Ser Cys Lys Gly Ser Gly Tyr Ser Phe Thr Asn Tyr
   20 25 30
   Trp Ile Gly Trp Val Arg Gln Met Pro Gly Lys Gly Leu Glu Trp Met
   35 40 45
   Gly Ile Ile Tyr Pro Gly Asp Ser Asp Thr Arg Tyr Ser Pro Ser Phe
   50 55 60
   Gln Gly Gln Val Thr Ile Ser Ala Asp Lys Ser Ile Ser Thr Ala Tyr
   65 70 75 80
   Leu Gln Trp Ser Ser Leu Lys Ala Ser Asp Thr Ala Met Tyr Tyr Cys
   85 90 95
   Ala Ser Pro Gly Tyr Thr Ser Ser Trp Thr Ser Phe Asp Tyr Trp Gly
   100 105 110
   Gln Gly Thr Leu Val Thr Val Ser Ser
   115 120
<210> 5
   <211> 107
   <212> PRT
   <213> Homo sapiens
<400> 5
   Ala Ile Gln Leu Thr Gln Ser Pro Ser Ser Leu Ser Ala Ser Val Gly
   1 5 10 15
   Asp Arg Val Thr Ile Thr Cys Arg Ala Ser Gln Gly Ile Ser Ser Ala
   20 25 30
   Leu Ala Trp Tyr Gln Gln Lys Ser Gly Lys Ala Pro Lys Leu Leu Ile
   35 40 45
   Phe Asp Ala Ser Ser Leu Glu Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Phe Asn Ser Tyr Pro Leu
   85 90 95
   Thr Phe Gly Gly Gly Thr Lys Val Glu Ile Lys
   100 105
<210> 6
   <211> 107
   <212> PRT
   <213> Homo sapiens
<400> 6
   Ala Ile Gln Leu Thr Gln Ser Pro Ser Ser Leu Ser Ala Ser Val Gly
   1 5 10 15
   Asp Arg Val Thr Ile Thr Cys Arg Ala Ser Gln Asp Ile Ser Ser Ala
   20 25 30
   Leu Ala Trp Tyr Gln Gln Lys Pro Gly Lys Ala Pro Lys Leu Leu Ile
   35 40 45
   Tyr Asp Ala Ser Ser Leu Glu Ser Gly Val Pro Ser Arg Phe Ser Gly
   50 55 60
   Tyr Gly Ser Gly Thr Asp Phe Thr Leu Thr Ile Asn Ser Leu Gln Pro
   65 70 75 80
   Glu Asp Phe Ala Thr Tyr Tyr Cys Gln Gln Phe Asn Ser Tyr Pro Leu
   85 90 95
   Thr Phe Gly Gly Gly Thr Lys Val Glu Ile Lys
   100 105
<210> 7
   <211> 109
   <212> PRT
   <213> Homo sapiens
<400> 7
   Ala Ile Gln Leu Thr Gln Ser Pro Ser Ser Leu Ser Ala Ser Val Gly
   1 5 10 15
   Asp Arg Val Thr Ile Thr Cys Arg Ala Ser Gln Asp Ile Ser Ser Ala
   20 25 30
   Leu Ala Trp Tyr Gln Gln Lys Pro Gly Lys Ala Pro Lys Leu Leu Ile
   35 40 45
   Tyr Asp Ala Ser Ser Leu Glu Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Phe Asn Ser Tyr Pro Leu
   85 90 95
   Thr Phe Gly Gly Gly Thr Lys Val Glu Ile Lys Ile Lys
   100 105
<210> 8
   <211> 108
   <212> PRT
   <213> Homo sapiens
<400> 8
   Glu Ile Val Leu Thr Gln Ser Pro Ala Thr Leu Ser Leu Ser Pro Gly
   1 5 10 15
   Glu Arg Ala Thr Leu Ser Cys Arg Ala Ser Gln Ser Val Ser Ser Tyr
   20 25 30
   Leu Ala Trp Tyr Gln Gln Lys Pro Gly Gln Ala Pro Arg Leu Leu Ile
   35 40 45
   Tyr Asp Ala Ser Asn Arg Ala Thr Gly Ile Pro Ala Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Val Tyr Tyr Cys Gln Gln Arg Ser Asn Trp Pro Leu
   85 90 95
   Phe Thr Phe Gly Pro Gly Thr Lys Val Asp Ile Lys
   100 105
<210> 9
   <211> 5
   <212> PRT
   <213> Homo sapiens
<400> 9
   Ser Tyr Ala Met His
   1 5
<210> 10
   <211> 5
   <212> PRT
   <213> Homo sapiens
<400> 10
   Ser Asn Trp Ile Gly
   1 5
<210> 11
   <211> 5
   <212> PRT
   <213> Homo sapiens
<400> 11
   Ser Asn Trp Ile Gly
   1 5
<210> 12
   <211> 5
   <212> PRT
   <213> Homo sapiens
<400> 12
   Asn Tyr Trp Ile Gly
   1 5
<210> 13
   <211> 17
   <212> PRT
   <213> Homo sapiens
<400> 13
   Val Ile Ser Tyr Asp Gly Asn Asn Lys Tyr Tyr Ala Asp Ser Val Lys
   1 5 10 15
   Gly
<210> 14
   <211> 17
   <212> PRT
   <213> Homo sapiens
<400> 14
   Ile Ile Tyr Pro Gly Asp Ser Asp Thr Arg Tyr Ser Pro Ser Phe Gln
   1 5 10 15
   Gly
<210> 15
   <211> 17
   <212> PRT
   <213> Homo sapiens
<400> 15
   Ile Ile Tyr Pro Gly Asp Ser Asp Thr Arg Tyr Ser Pro Ser Phe Gln
   1 5 10 15
   Gly
<210> 16
   <211> 17
   <212> PRT
   <213> Homo sapiens
<400> 16
   Ile Ile Tyr Pro Gly Asp Ser Asp Thr Arg Tyr Ser Pro Ser Phe Gln
   1 5 10 15
   Gly
<210> 17
   <211> 15
   <212> PRT
   <213> Homo sapiens
<400> 17
   Ala Val Pro Trp Gly Ser Arg Tyr Tyr Tyr Tyr Gly Met Asp Val
   1 5 10 15
<210> 18
   <211> 10
   <212> PRT
   <213> Homo sapiens
<400> 18
   Gln Thr Gly Phe Leu Trp Ser Ser Asp Leu
   1 5 10
<210> 19
   <211> 10
   <212> PRT
   <213> Homo sapiens
<400> 19
   Gln Thr Gly Phe Leu Trp Ser Phe Asp Leu
   1 5 10
<210> 20
   <211> 12
   <212> PRT
   <213> Homo sapiens
<400> 20
   Pro Gly Tyr Thr Ser Ser Trp Thr Ser Phe Asp Tyr
   1 5 10
<210> 21
   <211> 11
   <212> PRT
   <213> Homo sapiens
<400> 21
   Arg Ala Ser Gln Gly Ile Ser Ser Ala Leu Ala
   1 5 10
<210> 22
   <211> 11
   <212> PRT
   <213> Homo sapiens
<400> 22
   Arg Ala Ser Gln Asp Ile Ser Ser Ala Leu Ala
   1 5 10
<210> 23
   <211> 11
   <212> PRT
   <213> Homo sapiens
<400> 23
   Arg Ala Ser Gln Asp Ile Ser Ser Ala Leu Ala
   1 5 10
<210> 24
   <211> 11
   <212> PRT
   <213> Homo sapiens
<400> 24
   Arg Ala Ser Gln Ser Val Ser Ser Tyr Leu Ala
   1 5 10
<210> 25
   <211> 7
   <212> PRT
   <213> Homo sapiens
<400> 25
   Asp Ala Ser Ser Leu Glu Ser
   1 5
<210> 26
   <211> 7
   <212> PRT
   <213> Homo sapiens
<400> 26
   Asp Ala Ser Ser Leu Glu Ser
   1 5
<210> 27
   <211> 7
   <212> PRT
   <213> Homo sapiens
<400> 27
   Asp Ala Ser Ser Leu Glu Ser
   1 5
<210> 28
   <211> 7
   <212> PRT
   <213> Homo sapiens
<400> 28
   Asp Ala Ser Asn Arg Ala Thr
   1 5
<210> 29
   <211> 9
   <212> PRT
   <213> Homo sapiens
<400> 29
   Gln Gln Phe Asn Ser Tyr Pro Leu Thr
   1               5
<210> 30
   <211> 9
   <212> PRT
   <213> Homo sapiens
<400> 30
   Gln Gln Phe Asn Ser Tyr Pro Leu Thr
   1               5
<210> 31
   <211> 9
   <212> PRT
   <213> Homo sapiens
<400> 31
   Gln Gln Phe Asn Ser Tyr Pro Leu Thr
   1               5
<210> 32
   <211> 10
   <212> PRT
   <213> Homo sapiens
<400> 32
   Gln Gln Arg Ser Asn Trp Pro Leu Phe Thr
   1               5                   10
<210> 33
   <211> 372
   <212> DNA
   <213> Homo sapiens
<220>
   <221> CDS
   <222> (1)..(372)
<400> 33
   cag gtg caa ctg gtg gag tct ggg gga ggc gtg gtc cag cct ggg agg      48
   Gln Val Gln Leu Val Glu Ser Gly Gly Gly Val Val Gln Pro Gly Arg
    1               5                  10                  15
   tcc ctg aga ctc tcc tgt gca gcc tct gga ttc acc ttc agt agc tat      96
   Ser Leu Arg Leu Ser Cys Ala Ala Ser Gly Phe Thr Phe Ser Ser Tyr
               20                  25                  30
   gct atg cac tgg gtc cgc cag gct cca ggc aag ggg ctg gag tgg gtg     144
   Ala Met His Trp Val Arg Gln Ala Pro Gly Lys Gly Leu Glu Trp Val
           35                  40                  45
   gca gtt ata tca tat gat gga aac aat aaa tac tac gca gac tcc gtg     192
   Ala Val Ile Ser Tyr Asp Gly Asn Asn Lys Tyr Tyr Ala Asp Ser Val
       50                  55                  60
   aag ggc cga ttc acc atc tcc aga gac aat tcc aag aac acg ctg tat     240
   Lys Gly Arg Phe Thr Ile Ser Arg Asp Asn Ser Lys Asn Thr Leu Tyr
   65                  70                  75                  80
   ctg caa atg aac agc ctg aga gct gag gac acg gct gtg tat tac tgt     288
   Leu Gln Met Asn Ser Leu Arg Ala Glu Asp Thr Ala Val Tyr Tyr Cys
                   85                  90                  95
   gcg aga gcc gtc ccc tgg gga tcg agg tac tac tac tac ggt atg gac     336
   Ala Arg Ala Val Pro Trp Gly Ser Arg Tyr Tyr Tyr Tyr Gly Met Asp
               100                 105                 110
   gtc tgg ggc caa ggg acc acg gtc acc gtc tcc tca                     372
   Val Trp Gly Gln Gly Thr Thr Val Thr Val Ser Ser
           115                 120
<210> 34
   <211> 357
   <212> DNA
   <213> Homo sapiens
<220>
   <221> CDS
   <222> (1)..(357)
<400> 34
   gag gtg cag ctg gtg cag tct gga gca gag gtg aaa aag ccc ggg gag    48
   Glu Val Gln Leu Val Gln Ser Gly Ala Glu Val Lys Lys Pro Gly Glu
     1               5                  10                  15
   tct ctg aag atc tcc tgt aag ggt tct gga tac agc ttt acc agt aac    96
   Ser Leu Lys Ile Ser Cys Lys Gly Ser Gly Tyr Ser Phe Thr Ser Asn
                20                  25                  30
   tgg atc ggc tgg gtg cgc cag atg ccc ggg aaa ggc ctg gag tgg atg   144
   Trp Ile Gly Trp Val Arg Gln Met Pro Gly Lys Gly Leu Glu Trp Met
            35                  40                  45
   ggg atc atc tat cct ggt gac tct gat acc aga tac agc ccg tcc ttc   192
   Gly Ile Ile Tyr Pro Gly Asp Ser Asp Thr Arg Tyr Ser Pro Ser Phe
        50                  55                  60
   caa ggc cag gtc acc atc tca gcc gac aag tcc atc agc acc gcc tac   240
   Gln Gly Gln Val Thr Ile Ser Ala Asp Lys Ser Ile Ser Thr Ala Tyr
    65                  70                  75                  80
   ctg cag tgg agc agc ctg aag gcc tcg gac acc gcc atg tat tac tgt   288
   Leu Gln Trp Ser Ser Leu Lys Ala Ser Asp Thr Ala Met Tyr Tyr Cys
                    85                  90                  95
   gcg agg caa act ggt ttc ctc tgg tcc tcc gat ctc tgg ggc cgt ggc   336
   Ala Arg Gln Thr Gly Phe Leu Trp Ser Ser Asp Leu Trp Gly Arg Gly
               100                 105                 110
   acc ctg gtc act gtc tcc tca                                       357
   Thr Leu Val Thr Val Ser Ser
           115
<210> 35
   <211> 357
   <212> DNA
   <213> Homo sapiens
<220>
   <221> CDS
   <222> (1)..(357)
<400> 35
   gag gtg cag ctg gtg cag tct gga gca gag gtg aaa aag ccc ggg gag    48
   Glu Val Gln Leu Val Gln Ser Gly Ala Glu Val Lys Lys Pro Gly Glu
     1               5                  10                  15
   tct ctg aag atc tcc tgt aag ggt tct gga tac agt ttt acc agc aac    96
   Ser Leu Lys Ile Ser Cys Lys Gly Ser Gly Tyr Ser Phe Thr Ser Asn
                20                  25                  30
   tgg atc ggc tgg gtg cgc cag atg ccc ggg aaa ggc ctg gag tgg atg   144
   Trp Ile Gly Trp Val Arg Gln Met Pro Gly Lys Gly Leu Glu Trp Met
            35                  40                  45
   ggg atc atc tat cct ggt gac tct gat acc aga tac agc ccg tcc ttc      192
   Gly Ile Ile Tyr Pro Gly Asp Ser Asp Thr Arg Tyr Ser Pro Ser Phe
       50                  55                  60
   caa ggc cag gtc acc atc tca gcc gac aag tcc atc agc acc gcc tac      240
   Gln Gly Gln Val Thr Ile Ser Ala Asp Lys Ser Ile Ser Thr Ala Tyr
   65                  70                  75                  80
   ctg cag tgg aac agc ctg aag gcc tcg gac acc gcc atg tat tac tgt      288
   Leu Gln Trp Asn Ser Leu Lys Ala Ser Asp Thr Ala Met Tyr Tyr Cys
                   85                  90                  95
   gcg aga caa act ggt ttc ctc tgg tcc ttc gat ctc tgg ggc cgt ggc      336
   Ala Arg Gln Thr Gly Phe Leu Trp Ser Phe Asp Leu Trp Gly Arg Gly
               100                 105                 110
   acc ctg gtc act gtc tcc tca                                          357
   Thr Leu Val Thr Val Ser Ser
           115
<210> 36
   <211> 363
   <212> DNA
   <213> Homo sapiens
<220>
   <221> CDS
   <222> (1)..(363)
<400> 36
   gag gtg cag ctg gtg cag tct gga tca gag gtg aaa aag ccc ggg gag      48
   Glu Val Gln Leu Val Gln Ser Gly Ser Glu Val Lys Lys Pro Gly Glu
   1               5                   10                  15
   tct ctg aag atc tcc tgt aag ggt tct gga tac agc ttt acc aac tac      96
   Ser Leu Lys Ile Ser Cys Lys Gly Ser Gly Tyr Ser Phe Thr Asn Tyr
               20                  25                  30
   tgg atc ggc tgg gtg cgc cag atg ccc ggg aaa ggc ctg gag tgg atg      144
   Trp Ile Gly Trp Val Arg Gln Met Pro Gly Lys Gly Leu Glu Trp Met
           35                  40                  45
   ggg atc atc tat cct ggt gac tct gat acc aga tac agc ccg tcc ttc      192
   Gly Ile Ile Tyr Pro Gly Asp Ser Asp Thr Arg Tyr Ser Pro Ser Phe
       50                  55                  60
   caa ggc cag gtc acc atc tca gcc gac aag tcc atc agc acc gcc tat      240
   Gln Gly Gln Val Thr Ile Ser Ala Asp Lys Ser Ile Ser Thr Ala Tyr
   65                  70                  75                  80
   ctg cag tgg agc agc ctg aag gcc tcg gac acc gcc atg tat tac tgt      288
   Leu Gln Trp Ser Ser Leu Lys Ala Ser Asp Thr Ala Met Tyr Tyr Cys
                   85                  90                  95
   gcg agt ccc ggg tat acc agc agt tgg act tct ttt gac tac tgg ggc   336
   Ala Ser Pro Gly Tyr Thr Ser Ser Trp Thr Ser Phe Asp Tyr Trp Gly
               100                 105                 110
   cag gga acc ctg gtc acc gtc tcc tca                               363
   Gln Gly Thr Leu Val Thr Val Ser Ser
           115                 120
<210> 37
   <211> 321
   <212> DNA
   <213> Homo sapiens
<220>
   <221> CDS
   <222> (1)..(321)
<400> 37
   gcc atc cag ttg acc cag tct cca tcc tcc ctg tct gca tct gta gga    48
   Ala Ile Gln Leu Thr Gln Ser Pro Ser Ser Leu Ser Ala Ser Val Gly
   1               5                   10                  15
   gac aga gtc acc atc act tgc cgg gca agt cag ggc att agc agt gct    96
   Asp Arg Val Thr Ile Thr Cys Arg Ala Ser Gln Gly Ile Ser Ser Ala
               20                  25                  30
   tta gcc tgg tat cag cag aaa tca ggg aaa gct cct aag ctc ctg atc   144
   Leu Ala Trp Tyr Gln Gln Lys Ser Gly Lys Ala Pro Lys Leu Leu Ile
           35                  40                  45
   ttt gat gcc tcc agt ttg gaa agt ggg gtc cca tca agg ttc agc ggc   192
   Phe Asp Ala Ser Ser Leu Glu Ser Gly Val Pro Ser Arg Phe Ser Gly
       50                  55                  60
   agt gga tct ggg aca gat ttc act ctc acc atc agc agc ctg cag cct   240
   Ser Gly Ser Gly Thr Asp Phe Thr Leu Thr Ile Ser Ser Leu Gln Pro
   65                  70                  75                  80
   gaa gat ttt gca act tat tac tgt caa cag ttt aac agt tat cct ctc   288
   Glu Asp Phe Ala Thr Tyr Tyr Cys Gln Gln Phe Asn Ser Tyr Pro Leu
                   85                  90                  95
   act ttc ggc gga ggg acc aag gtg gag atc aaa                       321
   Thr Phe Gly Gly Gly Thr Lys Val Glu Ile Lys
               100                 105
<210> 38
   <211> 321
   <212> DNA
   <213> Homo sapiens
<220>
   <221> CDS
   <222> (1)..(321)
<400> 38
   gcc atc cag ttg acc cag tct cca tcc tcc ctg tct gca tct gta gga    48
   Ala Ile Gln Leu Thr Gln Ser Pro Ser Ser Leu Ser Ala Ser Val Gly
    1               5                  10                  15
   gac aga gtc acc atc act tgc cgg gca agt cag gac att agc agt gct    96
   Asp Arg Val Thr Ile Thr Cys Arg Ala Ser Gln Asp Ile Ser Ser Ala
               20                  25                  30
   tta gcc tgg tat caa cag aaa cca ggg aaa gct cct aag ctc ctg atc   144
   Leu Ala Trp Tyr Gln Gln Lys Pro Gly Lys Ala Pro Lys Leu Leu Ile
           35                  40                  45
   tat gat gcc tcc agt ttg gaa agt ggg gtc cca tca agg ttc agc ggc   192
   Tyr Asp Ala Ser Ser Leu Glu Ser Gly Val Pro Ser Arg Phe Ser Gly
       50                  55                  60
   tat gga tct ggg aca gat ttc act ctc acc atc aac agc ctg cag cct   240
   Tyr Gly Ser Gly Thr Asp Phe Thr Leu Thr Ile Asn Ser Leu Gln Pro
   65                  70                  75                  80
   gaa gat ttt gca act tat tac tgt caa cag ttt aat agt tac ccg ctc   288
   Glu Asp Phe Ala Thr Tyr Tyr Cys Gln Gln Phe Asn Ser Tyr Pro Leu
                   85                  90                  95
   act ttc ggc gga ggg acc aag gtg gag atc aaa                       321
   Thr Phe Gly Gly Gly Thr Lys Val Glu Ile Lys
               100                 105
<210> 39
   <211> 327
   <212> DNA
   <213> Homo sapiens
<220>
   <221> CDS
   <222> (1) .. (327)
<400> 39
   gcc atc cag ttg acc cag tct cca tcc tcc ctg tct gca tct gta gga    48
   Ala Ile Gln Leu Thr Gln Ser Pro Ser Ser Leu Ser Ala Ser Val Gly
    1               5                  10                  15
   gac aga gtc acc atc act tgc cgg gca agt cag gac att agc agt gct    96
   Asp Arg Val Thr Ile Thr Cys Arg Ala Ser Gln Asp Ile Ser Ser Ala
               20                  25                  30
   tta gcc tgg tat cag cag aaa ccg ggg aaa gct cct aag ctc ctg atc   144
   Leu Ala Trp Tyr Gln Gln Lys Pro Gly Lys Ala Pro Lys Leu Leu Ile
           35                  40                  45
   tat gat gcc tcc agt ttg gaa agt ggg gtc cca tca agg ttc agc ggc   192
   Tyr Asp Ala Ser Ser Leu Glu Ser Gly Val Pro Ser Arg Phe Ser Gly
       50                  55                  60
   agt gga tct ggg aca gat ttc act ctc acc atc agc agc ctg cag cct     240
   Ser Gly Ser Gly Thr Asp Phe Thr Leu Thr Ile Ser Ser Leu Gln Pro
    65                  70                  75                  80
   gaa gat ttt gca act tat tac tgt caa cag ttt aat agt tac ccg ctc     288
   Glu Asp Phe Ala Thr Tyr Tyr Cys Gln Gln Phe Asn Ser Tyr Pro Leu
                    85                  90                  95
   act ttc ggc gga ggg acc aag gtg gag atc aaa atc aaa                 327
   Thr Phe Gly Gly Gly Thr Lys Val Glu Ile Lys Ile Lys
               100                 105
<210> 40
   <211> 324
   <212> DNA
   <213> Homo sapiens
<220>
   <221> CDS
   <222> (1) .. (324)
<400> 40
   gaa att gtg ttg aca cag tct cca gcc acc ctg tct ttg tct cca ggg     48
   Glu Ile Val Leu Thr Gln Ser Pro Ala Thr Leu Ser Leu Ser Pro Gly
     1               5                  10                  15
   gaa aga gcc acc ctc tcc tgc agg gcc agt cag agt gtt agc agc tac     96
   Glu Arg Ala Thr Leu Ser Cys Arg Ala Ser Gln Ser Val Ser Ser Tyr
                20                  25                  30
   tta gcc tgg tac caa cag aaa cct ggc cag gct ccc agg ctc ctc atc     144
   Leu Ala Trp Tyr Gln Gln Lys Pro Gly Gln Ala Pro Arg Leu Leu Ile
            35                  40                  45
   tat gat gca tcc aac agg gcc act ggc atc cca gcc agg ttc agt ggc     192
   Tyr Asp Ala Ser Asn Arg Ala Thr Gly Ile Pro Ala Arg Phe Ser Gly
        50                  55                  60
   agt ggg tct ggg aca gac ttc act ctc acc atc agc agc cta gag cct     240
   Ser Gly Ser Gly Thr Asp Phe Thr Leu Thr Ile Ser Ser Leu Glu Pro
    65                  70                  75                  80
   gaa gat ttt gca gtt tat tac tgt cag cag cgt agc aac tgg ccc cta     288
   Glu Asp Phe Ala Val Tyr Tyr Cys Gln Gln Arg Ser Asn Trp Pro Leu
                    85                  90                  95
   ttc act ttc ggc cct ggg acc aaa gtg gat atc aaa                     324
   Phe Thr Phe Gly Pro Gly Thr Lys Val Asp Ile Lys
               100                 105
<210> 41
   <211> 98
   <212> PRT
   <213> Homo sapiens
<400> 41
   Gln Val Gln Leu Val Glu Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Ala Met His Trp Val Arg Gln Ala Pro Gly Lys Gly Leu Glu Trp Val
   35 40 45
   Ala Val Ile Ser Tyr Asp Gly Ser Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg
<210> 42
   <211> 98
   <212> PRT
   <213> Homo sapiens
<400> 42
   Glu Val Gln Leu Val Gln Ser Gly Ala Glu Val Lys Lys Pro Gly Glu
   1 5 10 15
   Ser Leu Lys Ile Ser Cys Lys Gly Ser Gly Tyr Ser Phe Thr Ser Tyr
   20 25 30
   Trp Ile Gly Trp Val Arg Gln Met Pro Gly Lys Gly Leu Glu Trp Met
   35 40 45
   Gly Ile Ile Tyr Pro Gly Asp Ser Asp Thr Arg Tyr Ser Pro Ser Phe
   50 55 60
   Gln Gly Gln Val Thr Ile Ser Ala Asp Lys Ser Ile Ser Thr Ala Tyr
   65 70 75 80
   Leu Gln Trp Ser Ser Leu Lys Ala Ser Asp Thr Ala Met Tyr Tyr Cys
   85 90 95
   Ala Arg
<210> 43
   <211> 95
   <212> PRT
   <213> Homo sapiens
<400> 43
   Ala Ile Gln Leu Thr Gln Ser Pro Ser Ser Leu Ser Ala Ser Val Gly
   1 5 10 15
   Asp Arg Val Thr Ile Thr Cys Arg Ala Ser Gln Gly Ile Ser Ser Ala
   20 25 30
   Leu Ala Trp Tyr Gln Gln Lys Pro Gly Lys Ala Pro Lys Leu Leu Ile
   35 40 45
   Tyr Asp Ala Ser Ser Leu Glu Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Phe Asn Ser Tyr Pro
   85 90 95
<210> 44
   <211> 94
   <212> PRT
   <213> Homo sapiens
<400> 44
   Glu Ile Val Leu Thr Gln Ser Pro Ala Thr Leu Ser Leu Ser Pro Gly
   1 5 10 15
   Glu Arg Ala Thr Leu Ser Cys Arg Ala Ser Gln Ser Val Ser Ser Tyr
   20 25 30
   Leu Ala Trp Tyr Gln Gln Lys Pro Gly Gln Ala Pro Arg Leu Leu Ile
   35 40 45
   Tyr Asp Ala Ser Asn Arg Ala Thr Gly Ile Pro Ala Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Val Tyr Tyr Cys Gln Gln Arg Ser Asn Trp
   85 90
<210> 45
   <211> 20
   <212> PRT
   <213> Homo sapiens
<400> 45
   Tyr Tyr Tyr Tyr Tyr Gly Met Asp Val Trp Gly Gln Gly Thr Thr Val
   1 5 10 15
   Thr Val Ser Ser
   20
<210> 46
   <211> 12
   <212> PRT
   <213> Homo sapiens
<400> 46
   Leu Thr Phe Gly Gly Gly Thr Lys Val Glu Ile Lys
   1 5 10
<210> 47
   <211> 12
   <212> PRT
   <213> Homo sapiens
<400> 47
   Phe Thr Phe Gly Pro Gly Thr Lys Val Asp Ile Lys
   1 5 10
<210> 48
   <211> 15
   <212> PRT
   <213> Artificial Sequence
<220>
   <223> Description of Artificial Sequence: Synthetic Gly-Ser linker
<400> 48
   Gly Gly Gly Gly Ser Gly Gly Gly Gly Ser Gly Gly Gly Gly Ser
   1 5 10 15

## Description

### Background of the Invention

Prostate cancer is a leading cause of morbidity and mortality among men. Treatments for prostate cancer include surgery, hormones, radiation, and chemotherapy. There is little effective treatment for metastatic prostate disease. Therefore, the identification of genes and/or gene products that represent diagnostic and prognostic markers, as well as targets for therapy, is critical. Prostate specific antigen (PSA) is one such cancer marker which is useful in the clinical diagnosis and staging of prostate cancer. However, PSA cannot differentiate benign prostatic hyperplasia (BPH) from prostatitis or prostate cancer in the range of 4-10 ng/ml, thus, necessitating a cytologic and/or histologic assessment to confirm the proper diagnosis (Barren, R.J. et al. (1998) Prostate 36:181-188).

Prostate specific membrane antigen (PSMA) is a 750 amino acid, type II transmembrane glycoprotein of approximately 110 kD that has 54% homology to the transferrin receptor. PSMA has 3 structural domains, including a 19 amino acid intracellular domain, a 24 amino acid transmembrane domain, and a 707 amino acid extracellular domain. The PSMA protein displays neurocarboxypeptidase and folate hydrolase activity and is reported to be involved in the neuroendocrine regulation of prostate growth and differentiation (Heston, W.D. (1996) Urologe-Ausgabe A. 35:400-407). PSM' is an alternatively spliced form of PSMA which is localized in the cytoplasm.

PSMA is predominantly expressed by prostatic epithelial cells. The expression of PSMA is increased in prostate cancer, especially in poorly differentiated, metastatic, and hormone refractory carcinomas (Gregorakis, A.K. et al. (1998) Seminars in Urologic Oncology 16:2-12; Silver, D.A. (1997) Clinical Cancer Research 3:81-85). Low level expression of PSMA is observed in extraprostatic tissues such as the small bowel, salivary gland, duodenal mucosa, proximal renal tubules, and brain (Silver, D.A. (1997) Clinical Cancer Research 3:81-85). PSMA is also expressed in endothelial cells of capillary vessels in peritumoral and endotumoral areas of certain malignancies, including renal cell carcinomas, and colon carcinomas, but not in blood vessels from normal tissues. In addition, PSMA is reported to be related to tumor angiogenesis (Silver, D.A. (1997) Clinical Cancer Research 3:81-85). Recently, PSMA has been demonstrated to be expressed in endothelial cells of tumor-associated neovasculature in carcinomas of the colon, breast, bladder, pancreas, kidney, and melanoma (Chang, S.S. (2004) Curr Opin Investig Drugs 5:611-5).

Antibodies against the extracellular domain of PSMA have been described (see *e.g*., Liu, H. et al. (1997) Cancer Res. 57:3629-3634; Murphy, G.P. et al (1998) J. Urol. 160:2396-2401; Wang, S. et al. (2001) Int. J. Cancer 92:871-876; Kato, K. et al. (2003) Int. J. Urol. 10:439-444; US Patent No. 6,150,508 and US Patent No. 6,107,090). More recently, human and humanized antibodies that bind PSMA have been described (see *e.g*., Bander, N.H. et al. (2003) Semin. Oncol. 30:667-676; PCT Publication WO 02/098897; PCT Publication WO 01/09192; PCT Publication WO 03/064606; PCT Publication WO 03/034903; and US Application No. 2004/0033229). Such antibodies have been used for imaging of prostate cancer cells (see *e.g*., Yao, D. et al. (2002) Semin. Urol. Oncol. 20:211-218; Bander, N.H. et al (2003) J. Urol. 170:1717-1721). Anti-PSMA antibodies also have been used for therapeutic intervention in treatment of prostate cancer, typically as a conjugate with a chemotherapeutic agent or radioactive isotope (see e.g., Nanus, D.M. et al. (2003) J. Urol. 170:S84-89; Milowsky, M.I. et al. (2004) J. Clin. Oncol. 22:2522-2531; Henry, M.D. et al. (2004) Cancer Res. 64:7995-8001).

Accordingly, PSMA represents a valuable target for the treatment of prostate cancer and a variety of other diseases characterized by PSMA expression and additional therapeutic agents that recognize PSMA are desired.

### Summary of the Invention

The invention provides an isolated monoclonal antibody, or an antigen-binding portion thereof, comprising:
(a) a heavy chain variable region CDR1 comprising SEQ ID NO: 10;
(b) a heavy chain variable region CDR2 comprising SEQ ID NO: 14;
(c) a heavy chain variable region CDR3 comprising SEQ ID NO: 18;
(d) a light chain variable region CDR1 comprising SEQ DD NO: 22;
(e) a light chain variable region CDR2 comprising SEQ ID NO: 26; and
(f) a light chain variable region CDR3 comprising SEQ ID NO: 30.

Other preferred antibodies of the invention, or antigen binding portions thereof, comprise:
(a) a heavy chain variable region comprising the amino acid sequence of SEQ ID NO: 2; and
(b) a light chain variable region comprising the amino acid sequence of SEQ ID NO: 6.

The antibodies of the invention can be, for example, full-length antibodies, for example of an IgG 1 or IgG4 isotype. Alternatively, the antibodies can be antibody fragments, such as Fab or Fab'2 fragments, or single chain antibodies.

The invention also provides an immunoconjugate comprising an antibody of the invention, or antigen-binding portion thereof, linked to a therapeutic agent, such as a cytotoxin or a radioactive isotope. The invention also provides a bispecific molecule comprising an antibody, or antigen-binding portion thereof, of the invention, linked to a second functional moiety having a different binding specificity than said antibody, or antigen binding portion thereof.

Compositions comprising an antibody, or antigen-binding portion thereof, or immunoconjugate or bispecific molecule of the invention and a pharmaceutically acceptable carrier are also provided.

Nucleic acid molecules encoding the antibodies, or antigen-binding portions thereof, of the invention are also encompassed by the invention, as well as expression vectors comprising such nucleic acids and host cells comprising such expression vectors. Moreover, the invention provides a transgenic mouse comprising human immunoglobulin heavy and light chain transgenes, wherein the mouse expresses an antibody of the invention, as well as hybridomas prepared from such a mouse, wherein the hybridoma produces the antibody of the invention.

In yet another aspect, the invention provides a method of inhibiting growth of tumor cells in a subject, wherein the tumor cells or vascular endothelial cells proximate to the tumor cells express PSMA, comprising administering to a subject an anti-PSMA human antibody of the present invention in an amount effective to growth of the tumor cells. In a preferred embodiment, growth of prostate tumor cells is inhibited.

In another aspect, the invention provides a method of inhibiting or preventing growth of a tumor (e.g., prostate, colon, renal, rectal, urothelial, breast, bladder, liver, pancreas or melanoma) in a subject, wherein cells of the tumor or vascular endothelial cells proximate to the tumor express PSMA. The method includes administering to a subject an anti-PSMA antibody, or antigen-binding portion thereof, in combination with an anti-tumor agent both in an amount effective to inhibit or prevent growth of the tumor.

In another aspect, the invention provides a method of stimulating antibody dependent cell-mediated cytotoxicity (ADCC) of a tumor in a subject, wherein cells of the tumor or vascular endothelial cells proximate to the tumor express PSMA. The method includes administering to a subject an anti-PSMA antibody, or antigen-binding portion thereof, in combination with an anti-tumor agent both in an amount effective to stimulate antibody dependent cell-mediated cytotoxicity (ADCC) of the tumor.

In a further aspect, the invention provides a method of of inhibiting tumor-related cachexia in a subject, wherein cells of the tumor or vascular endothelial cells proximate to the tumor express PSMA. The method includes administering to a subject an anti-PSMA antibody, or antigen-binding portion thereof, in combination with an anti-tumor agent both in an amount effective to inhibit tumor-related cachexia in the subject.

In one embodiment of the invention, administration of the anti-PSMA antibody, or antigen-binding portion thereof, to a subject, in combination with the anti-tumor agent, leads to a synergistic effect on the inhibition of the growth of the tumor. In another embodiment, the anti-tumor agent causes damage in the tumor mass, thereby leading to a more effective antibody dependent cell-mediated cytotoxicity (ADCC) of the tumor.

In another embodiment, the anti-PSMA antibody may be the 2A10 antibody.

In one embodiment of the invention, the anti-tumor agent is a chemotherapeutic agent, such as Taxotere® (docetaxel). In another embodiment, the anti-tumor agent is an anti-angiogenic agent, such as angiostatin K1-3, Arresten, aaAT, Canstatin, DL-α-Difluoromethyl-ornithine, Endostatin, Fumagillin, Genistein, Minocycline, Staurosporine, Thalidomide, and Tumstatin. In another embodiment, the anti-tumor agent is an immunomodulatory agent, such as anti-PD1 antibodies, anti-CTLA-4 antibodies, phosphorothiolate oligodeoxyribonucleotide (1018 ISS), GM-CSF gene vaccines, interleukin-2, interleukin-7 (CYT 99 07), interleukin-12 and interleukin-21.

In another aspect, the invention provides method of identifying an anti-rumor agent capable of acting synergistically with an anti-PSMA antibody in inhibiting or preventing growth of a tumor, wherein cells of the tumor or vascular endothelial cells proximate to the tumor express PSMA. The method includes contacting an indicator composition with (a) a test anti-tumor agent alone, (b) an anti-PSMA antibody alone, and (c) both a test anti-tumor agent and an anti-PSMA antibody; and comparing the ability of (a) the test anti-tumor agent alone and (b) the anti-PSMA antibody alone to inhibit or prevent growth of a tumor to the ability of (c) both the test anti-rumor agent and the anti-PSMA antibody to inhibit or prevent growth of a tumor, wherein inhibition or prevention of tumor growth by (c) in an amount that is greater than the additive effect of (a) and (b) would lead to the identification of an anti-tumor agent capable of acting synergistically with an anti-PSMA antibody in inhibiting or preventing growth of a tumor.

In another aspect, the invention pertains to a composition comprising an anti-PSMA antibody (e.g., 2A10) and an anti-tumor agent (e.g., Taxotere® (docetaxel), angiostatin K1-3, Arresten, aaAT, Canstatin, DL-α- Difluoromethyl-ornithine, Endostatin, Fumagillin, Genistein, Minocycline, Staurosporine, Thalidomide, Tumstatin, an anti-PD1 antibody, an anti-CTLA-4 antibody, phosphorothiolate oligodeoxyribonucleotide (1018 ISS), a GM-CSF gene vaccine, interleukin-2, interleukin-7 (CYT 99 07), interleukin-12 or interleukin-21) in an amount effective to inhibit or prevent growth of a tumor and a pharmaceutically acceptable carrier or in an amount effective to stimulate antibody dependent cell- mediated cytotoxicity (ADCC) of a tumor and a pharmaceutically acceptable carrier.

Other features and advantages of the instant invention will be apparent from the following detailed description and examples which should not be construed as limiting.

### Brief Description of the Drawings

Figure 1A shows the nucleotide sequence (SEQ ID NO: 33) and amino acid sequence (SEQ ID NO: 1) of the heavy chain variable region of the 1C3 human monoclonal antibody. The CDR1 (SEQ ID NO: 9), CDR2 (SEQ ID NO: 13) and CDR3 (SEQ ID NO: 17) regions are delineated and the V, D and J germline derivations are indicated.
Figure 1B shows the nucleotide sequence (SEQ ID NO: 37) and amino acid sequence (SEQ ID NO: 5) of the light chain variable region of the 1C3 human monoclonal antibody. The CDR1 (SEQ ID NO: 21), CDR2 (SEQ ID NO: 25) and CDR3 (SEQ ID NO: 29) regions are delineated and the V and J germline derivations are indicated.
Figure 2A shows the nucleotide sequence (SEQ ID NO: 34) and amino acid sequence (SEQ ID NO: 2) of the heavy chain variable region of the 2A10 human monoclonal antibody. The CDR1 (SEQ ID NO: 10), CDR2 (SEQ ID NO: 14) and CDR3 (SEQ ID NO: 18) regions are delineated and the V and J germline derivations are indicated.
Figure 2B shows the nucleotide sequence (SEQ ID NO: 38) and amino acid sequence (SEQ ID NO: 6) of the light chain variable region of the 2A10 human monoclonal antibody. The CDR1 (SEQ ID NO: 22), CDR2 (SEQ ID NO: 26) and CDR3 (SEQ ID NO: 30) regions are delineated and the V and J germline derivations are indicated.
Figure 3A shows the nucleotide sequence (SEQ ID NO: 35) and amino acid sequence (SEQ ID NO: 3) of the heavy chain variable region of the 2F5 human monoclonal antibody. The CDR1 (SEQ ID NO: 11), CDR2 (SEQ ID NO: 15) and CDR3 (SEQ ID NO: 19) regions are delineated and the V and J germline derivations are indicated.
Figure 3B shows the nucleotide sequence (SEQ ID NO: 39) and amino acid sequence (SEQ ID NO: 7) of the light chain variable region of the 2F5 human monoclonal antibody. The CDR1 (SEQ ID NO: 23), CDR2 (SEQ ID NO: 27) and CDR3 (SEQ ID NO: 31) regions are delineated and the V and J germline derivations are indicated.
Figure 4A shows the nucleotide sequence (SEQ ID NO: 36) and amino acid sequence (SEQ ID NO: 4) of the heavy chain variable region of the 2C6 human monoclonal antibody. The CDR1 (SEQ ID NO: 12), CDR2 (SEQ ID NO: 16) and CDR3 (SEQ ID NO: 20) regions are delineated and the V and J germline derivations are indicated.
Figure 4B shows the nucleotide sequence (SEQ ID NO: 40) and amino acid sequence (SEQ ID NO: 8) of the light chain variable region of the 2C6 human monoclonal antibody. The CDR1 (SEQ ID NO: 24), CDR2 (SEQ ID NO: 28) and CDR3 (SEQ ID NO: 32) regions are delineated and the V and J germline derivations are indicated.
Figure 5 shows the alignment of the amino acid sequence of the heavy chain variable region of 1C3 (SEQ ID NO: 1) with the human germline V_{H} 3-30.3 amino acid sequence (SEQ ID NO: 41) and the JH6b germline (SEQ ID NO: 45).
Figure 6 shows the alignment of the amino acid sequence of the heavy chain variable region of 2A10 (SEQ ID NO: 2), 2F5 (SEQ ID NO: 3), and 2C6 (SEQ ID NO: 4) with the human germline V_{H} 5-51 amino acid sequence (SEQ ID NO: 42).
Figure 7 shows the alignment of the amino acid sequence of the light chain variable region of 1C3 (SEQ ID NO: 5), 2A10 (SEQ ID NO: 6), and 2F5 (residues 1-107 of SEQ ID NO: 7) with the human germline Vₖ L18 amino acid sequence (SEQ ID NO:43) and the JK4 germline (SEQ ID NO: 46).
Figure 8 shows the alignment of the amino acid sequence of the light chain variable region of 2C6 (SEQ ID NO: 8) with the human germline Vₖ L6 amino acid sequence (SEQ ID NO:44) and the JK3 germline (SEQ ID NO: 47).
Figure 9 shows the results of flow cytometry experiments demonstrating that the human monoclonal antibodies 2F5, 2A10, and 2C6, directed against human PSMA, binds the cell surface of PSMA-expressing LNCaP cells.
Figures 10 shows the results of ELISA experiments demonstrating that human monoclonal antibodies against human PSMA specifically bind to PSMA purified from LNCaP cells.
Figures 11A-11B are results of antibody binding competition studies that demonstrate that the human monoclonal antibodies 2A10 and 7F12, directed against human PSMA, compete for binding to PSMA-expressing LNCaP prostate cancer cells. Figure 11 shows competition of ^{12S}I-2A10 with cold 7F12 antibody. Figure 11B shows competition of ¹²⁵I-7F12 with cold 2A10 antibody.
Figures 12A-12B shows the results of internalization experiments demonstrating that the human monoclonal antibody 2A10, directed against human PSMA, enters PSMA-expressing LNCaP prostate cancer cells by a ³H-thymidine release assay. Figure 12A shows results for LNCaP cells seeded for 2 hours prior to introduction of antibody. Figure 12B shows results for LNCaP cells seeded overnight prior to introduction of antibody.

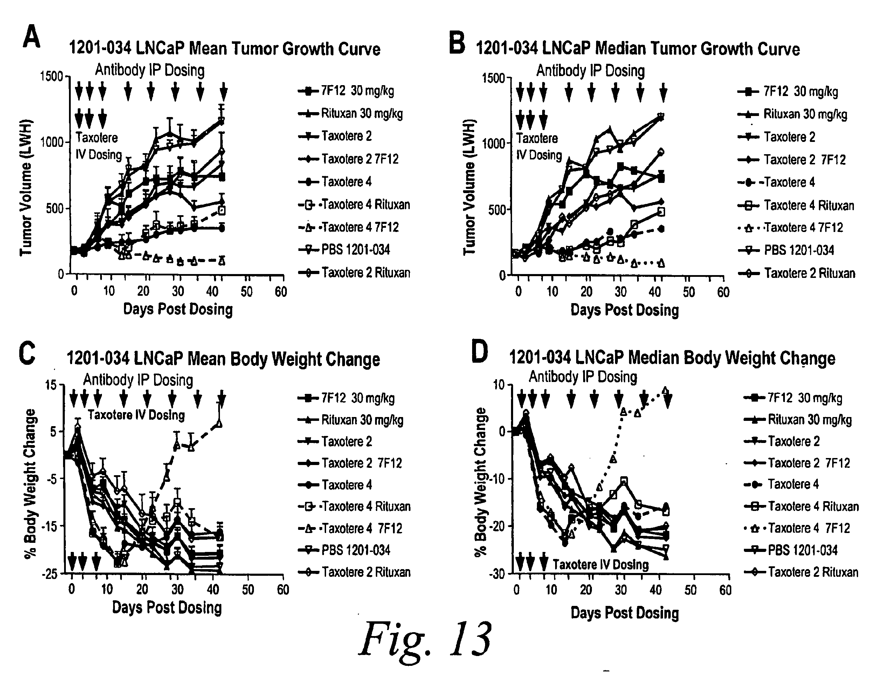

Figures 13A-13B are graphs depicting the mean and median LNCaP tumor xenografts growth curves for mice treated with either: the anti-PSMA 7F12 antibody, the isotype control antibody Rituxan, Taxotere (2 mg/kg), Taxotere (4 mg/kg), the anti-PSMA 7F12 antibody in combination with Taxotere (2 mg/kg), the and-PSMA 7F12 antibody in combination with Taxotere (4 mg/kg), the isotype control antibody Rituxan in combination with Taxotere (2 mg/kg), the isotype control antibody Rituxan in combination with Taxotere (4 mg/kg), or PBS. Figures 13C-13D are graphs depicting the mean and median body weight change of LNCaP tumor-bearing mice treated with either: the anti-PSMA 7F12 antibody, the isotype control antibody Rituxan, Taxotere (2 mg/kg), Taxotere (4 mg/kg), the anti-PSMA 7F12 antibody in combination with Taxotere (2 mg/kg), the anti-PSMA 7F12 antibody in combination with Taxotere (4 mg/kg), the isotype control antibody Rituxan in combination with Taxotere (2 mg/kg), the isotype control antibody Rituxan in combination with Taxotere (4 mg/kg), or PBS.
Figures 14A-14B are graphs depicting the mean and median LNCaP tumor xenografts growth curves for mice treated with either: the anti-PSMA 7F12 antibody, the isotype control antibody Rituxan, or PBS. Figures 14C-14D are graphs depicting the mean and median body weight change of LNCaP tumor-bearing mice treated with either: the anti-PSMA 7F12 antibody, the isotype control antibody Rituxan, or PBS.
Figures 15A-15B are graphs depicting the mean and median LNCaP tumor xenografts growth curves for mice treated with either: Taxotere (4 mg/kg), the isotype control antibody Rituxan in combination with Taxotere (4 mg/kg), the anti-PSMA 7F12 antibody in combination with Taxotere (4 mg/kg), or PBS. Figures 15C-15D are graphs depicting the mean and median body weight change of LNCaP tumor-bearing mice treated with either: Taxotere (4 mg/kg), the isotype control antibody Rituxan in combination with Taxotere (4 mg/kg), the anti-PSMA 7F12 antibody in combination with Taxotere (4 mg/kg), or PBS.
Figures 16A-16B are graphs depicting the mean and median LNCaP tumor xenografts growth curves for mice treated with either: Taxotere (2 mg/kg), the anti-PSMA 7F12 antibody in combination with Taxotere (2 mg/kg), the isotype control antibody Rituxan in combination with Taxotere (2 mg/kg), or PBS. Figures 16C-16D are graphs depicting the mean and median body weight change of LNCaP tumor-bearing mice treated with either: Taxotere (2 mg/kg), the anti-PSMA 7F12 antibody in combination with Taxotere (2 mg/kg), the isotype control antibody Rituxan in combination with Taxotere (2 mg/kg), or PBS.
Figure 17A-B shows the results of a cell proliferation assay demonstrating that toxin-conjugated human monoclonal anti-PSMA antibodies show cytotoxicity to prostate cancer cells (A) with a three-hour wash and (B) with a continuous wash.
FIG. 18 is a graph of changes in tumor volume over time for mice dosed with an isotype control antibody-drug conjugate, a αPSMA antibody-drug conjugate, or a conjugation buffer alone (vehicle).
FIG. 19 is a graph of changes in tumor volume over time for mice dosed with various amounts of a αPSMA antibody-drug conjugate or a conjugation buffer alone (vehicle).
FIG. 20 is a graph of changes in tumor volume over time for mice dosed with various amounts of an isotype control antibody-drug conjugate or a conjugation buffer alone (vehicle).
FIG. 21 is a graph of body weight change over time for mice dosed with various amounts of an isotype control antibody-drug conjugate or a conjugation buffer alone (vehicle).
FIG. 22 is a graph of body weight change over time for mice dosed with various amounts of a αPSMA antibody-drug conjugate or a conjugation buffer alone (vehicle).
FIG. 23 is a graph of changes in tumor volume over time, for tumors having an initial average tumor volume of 240 mm³, for mice dosed with an isotype control antibody-drug conjugate, a αPSMA antibody-drug conjugate, or a conjugation buffer alone (vehicle).
FIG. 24 is a graph of changes in tumor volume over time, for tumors having an initial average tumor volume of 430 mm³, for mice dosed with a αPSMA antibody-drug conjugate or a conjugation buffer alone (vehicle).

### Detailed Description of the Invention

The present invention relates to isolated monoclonal antibodies, particularly human monoclonal antibodies, that bind specifically to PSMA with high affinity. In certain embodiments, the antibodies of the invention are derived from particular heavy and light chain germline sequences and/or comprise particular structural features such as CDR regions comprising particular amino acid sequences. The invention provides isolated antibodies, methods of making such antibodies, immunoconjugates and bispecific molecules comprising such antibodies and pharmaceutical compositions containing the antibodies, immunconjugates or bispecific molecules of the invention. The invention also relates to methods of using the antibodies, such as to treat diseases such as cancer.

In order that the present invention may be more readily understood, certain terms are first defined. Additional definitions are set forth throughout the detailed description.

The terms "prostate specific membrane antigen" and "PSMA" are used interchangeably herein, and include any variants, isoforms and species homologs of human PSMA that are naturally expressed by cells and that retain binding to the antibodies 1C3, 2A10, 2F5 or 2C6 described herein. The complete amino acid sequence of human PSMA protein has the Genbank accession number NP_004467. The complete cDNA sequence encoding the human PSMA protein has the Genbank accession number NM_004476.

A "signal transduction pathway" refers to the biochemical relationship between a variety of signal transduction molecules that play a role in the transmission of a signal from one portion of a cell to another portion of a cell. As used herein, the phrase "cell surface receptor" includes, for example, molecules and complexes of molecules capable of receiving a signal and the transmission of such a signal across the plasma membrane of a cell. An example of a "cell surface receptor" of the present invention is the PSMA receptor.

The term "antibody" as referred to herein includes whole antibodies and any antigen binding fragment (*i.e*., "antigen-binding portion") or single chains thereof. An "antibody" refers to a glycoprotein comprising at least two heavy (H) chains and two light (L) chains inter-connected by disulfide bonds, or an antigen binding portion thereof. Each heavy chain is comprised of a heavy chain variable region (abbreviated herein as V_{H}) and a heavy chain constant region. The heavy chain constant region is comprised of three domains, C_{H1}, C_{H2} and C_{H3}. Each light chain is comprised of a light chain variable region (abbreviated herein as V_{L}) and a light chain constant region. The light chain constant region is comprised of one domain, C_{L}. The V_{H} and V_{L} regions can be further subdivided into regions ofhypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (*e.g*., effector cells) and the first component (Clq) of the classical complement system.

The term "antigen-binding portions" of an antibody (or simply "antibody portion"), as used herein, refers to one or more fragments of an antibody that retain the ability to specifically bind to an antigen (*e.g*., PSMA). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the V_{L}, V_{H}, C_{L} and C_{H1} domains; (ii) a F(ab')₂ fragment, a bivalent fragment comprising two Fab fragments linked by a disulfide bridge at the hinge region; (iii) a Fd fragment consisting of the V_{H} and C_{H1} domains; (iv) a Fv fragment consisting of the V_{L} and V_{H} domains of a single arm of an antibody, (v) a dAb fragment (Ward et al., (1989) Nature 341:544-546), which consists of a V_{H} domain; and (vi) an isolated complementarity determining region (CDR). Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); *see e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies.

An "isolated antibody", as used herein, is intended tp refer to an antibody that is substantially free of other antibodies having different antigenic specificities (*e.g*., an isolated antibody that specifically binds PSMA is substantially free of antibodies that specifically bind antigens other than PSMA). An isolated antibody that specifically binds PSMA may, however, have cross-reactivity to other antigens, such as PSMA molecules from other species. Moreover, an isolated antibody may be substantially free of other cellular material and/or chemicals.

The terms "monoclonal antibody" or "monoclonal antibody composition" as used herein refer to a preparation of antibody molecules of single molecular composition. A monoclonal antibody composition displays a single binding specificity and affinity for a particular epitope.

The term "human antibody", as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies of the invention may include amino acid residues not encoded by human germline immunoglobulin sequences (*e.g*., mutations introduced by random or site-specific mutagenesis *in vitro* or by somatic mutation *in vivo).* However, the term "human antibody", as used herein, is not intended to include antibodies in which CDR sequences derived from the germline of another mammalian species, such as a mouse, have been grafted onto human framework sequences.

The term "human monoclonal antibody" refers to antibodies displaying a single binding specificity which have variable regions in which both the framework and CDR regions are derived from human germline immunoglobulin sequences. In one embodiment, the human monoclonal antibodies are produced by a hybridoma which includes a B cell obtained from a transgenic nonhuman animal, *e.g*., a transgenic mouse, having a genome comprising a human heavy chain transgene and a light chain transgene fused to an immortalized cell.

The term "recombinant human antibody", as used herein, includes all human antibodies that are prepared, expressed, created or isolated by recombinant means, such as (a) antibodies isolated from an animal (*e.g*., a mouse) that is transgenic or transchromosomal for human immunoglobulin genes or a hybridoma prepared therefrom (described further below), (b) antibodies isolated from a host cell transformed to express the human antibody, *e.g*., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

As used herein, "isotype" refers to the antibody class (*e.g*., IgM or IgG1) that is encoded by the heavy chain constant region genes.

The phrases "an antibody recognizing an antigen" and "an antibody specific for an antigen" are used interchangeably herein with the term "an antibody which binds specifically to an antigen."

The term "human antibody derivatives" refers to any modified form of the human antibody, *e.g*., a conjugate of the antibody and another agent or antibody.

The term "humanized antibody" is intended to refer to antibodies in which CDR sequences derived from the germline of another mammalian species, such as a mouse, have been grafted onto human framework sequences. Additional framework region modifications may be made within the human framework sequences.

The term "chimeric antibody" is intended to refer to antibodies in which the variable region sequences are derived from one species and the constant region sequences are derived from another species, such as an antibody in which the variable region sequences are derived from a mouse antibody and the constant region sequences are derived from a human antibody.

As used herein, an antibody that "specifically binds to human PSMA" is intended to refer to an antibody that binds to human PSMA with a K_{D} of 5 x 10⁻⁸ M or less, more preferably 1 x 10⁻⁸ M or less, more preferably 5 x 10⁻⁹ M or less, more preferably 1.2 x 10⁻⁹ M or less, even more preferably between 1.2 x 10⁻⁹ M and 1.2x10⁻¹⁰ M or less.

The term "K_{assoc}" or "Kₐ", as used herein, is intended to refer to the association rate of a particular antibody-antigen interaction, whereas the term "K_{dis}" or "K_{d}," as used herein, is intended to refer to the dissociation rate of a particular antibody-antigen interaction. The term "K_{D}", as used herein, is intended to refer to the dissociation constant, which is obtained from the ratio of K_{d} to Kₐ *(i.e,.* K_{d}/Kₐ) and is expressed as a molar concentration (M). K_{D} values for antibodies can be determined using methods well established in the art. A preferred method for determining the K_{D} of an antibody is by using surface plasmon resonance, preferably using a biosensor system such as a Biacore® system.

As used herein, the term "high affinity" for an IgG antibody refers to an antibody having a K_{D} of 10⁻⁷ M or less, more preferably 10⁻⁸ M or less, more preferably 10⁻⁹ M or less, and even more preferably 10⁻¹⁰ M or less for a target antigen. However, "high affinity" binding can vary for other antibody isotypes. For example, "high affinity" binding for an IgM isotype refers to an antibody having a K_{D} of 10⁻⁷ M or less, more preferably 10⁻⁸ M or less, even more preferably 10⁻⁹ M or less. The term "vector," as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.*, bacterial vectors having a bacterial origin of replication and episomal mammalian vectors). Other vectors (*e.g*., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (*e.g*., replication defective retroviruses, adenoviruses and adeno-associated viruses), which serve equivalent functions.

The term "recombinant host cell" (or simply "host cell"), as used herein, is intended to refer to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. Recombinant host cells include, for example, CHO cells, transfectomas, and lymphocytic cells.

As used herein, the term "subject" includes any human or nonhuman animal. The term "nonhuman animal" includes all vertebrates, e.g., mammals and non-mammals, such as nonhuman primates, sheep, dogs, cats, horses, cows, chickens, amphibians, reptiles, etc.

The terms "transgenic, nonhuman animal" refers to a nonhuman animal having a genome comprising one or more human heavy and/or light chain transgenes or transchromosomes (either integrated or non-integrated into the animal's natural genomic DNA) and which is capable of expressing fully human antibodies. For example, a transgenic mouse can have a human light chain transgene and either a human heavy chain trans gene or human heavy chain transchromosome, such that the mouse produces human anti-PSMA antibodies when immunized with PSMA antigen and/or cells expressing PSMA. The human heavy chain transgene can be integrated into the chromosomal DNA of the mouse, as is the case for transgenic, *e.g*., HuMAb mice, or the human heavy chain transgene can be maintained extrachromosomally, as is the case for transchromosomal (*e.g*., KM) mice as described in WO 02/43478. Such transgenic and transchromosomal mice are capable of producing multiple isotypes of human monoclonal antibodies to PSMA (*e.g*., IgG, IgA and/or IgE) by undergoing V-D-J recombination and isotype switching.

As used herein, the term "subject" includes any human or nonhuman animal. The term "nonhuman animal" includes all vertebrates, *e.g*., mammals and non-mammals, such as nonhuman primates, sheep, dogs, cats, horses, cows chickens, amphibians, reptiles, *etc*.

Various aspects of the invention are described in further detail in the following subsections.

### Anti-PSMA Antibodies

The antibodies of the invention are characterized by particular functional features or properties of the antibodies. For example, the antibodies bind specifically to human PSMA. Preferably, an antibody of the invention binds to PSMA with high affinity, for example with a K_{D} of 5 x 10⁻⁷ M or less. As another example, the antibodies bind specifically to a PSMA-expressing LNCaP (ATCC CRL-1740) cell line. Standard assays to evaluate the binding ability of the antibodies toward PSMA are known in the art, including for example, ELISAs, Western blots and RIAs. Suitable assays are described in detail in the Examples. The binding kinetics (e.g., binding affinity) of the antibodies also can be assessed by standard assays known in the art, such as by ELISA, Scatchard, and Biacore analysis. Other preferred properties of the antibodies of the invention include the ability to be internalized by PSMA expressing cells and high thermostability. Internalization of antibodies can be assessed as described in Example 6. Thermostability can be assessed as described in Example 7. Preferred antibodies of the invention have a melting point of at least 65°C, more preferably, at least 66°C, even more preferably at least 67°C, even more preferably at least 68°C, even more preferably at least 69°C, even more preferably at least 70°C and even more preferably at least 71°C. Preferably an antibody of the invention has melting point in a range of 67°C to 72°C, more preferably 68°C to 72°C, or 69°C to 72°C, or 70°C to 72°C or 69°C to 71.43°C, or the antibody has a melting point of approximately 71.43°C.

### Monoclonal Antibody 2AIO

A preferred antibody of the invention is the human monoclonal antibody 2A10 isolated and structurally characterized as described in Examples 1 and 2. The V_{H} amino acid sequence of 2A10 is shown in SEQ ID NO: 2. The V_{L} amino acid sequence of 2A10 is shown in SEQ ID NO: 6.

In another aspect, the invention provides antibodies that comprise the heavy chain and light chain CDR1s, CDR2s and CDR3s of 2A10. The amino acid sequence of the V_{H} CDR1 of 2A10 is shown in SEQ ID NO: 10. The amino acid sequence of the V_{H} CDR2 of 2A10 is shown in SEQ ID NO: 14. The amino acid sequence of the V_{H} CDR3 of 2A10 is shown in SEQ ID NO: 18. The amino acid sequence of the Vₖ CDR1 of 2A10 is shown in SEQ ID NO: 22. The amino acid sequence of the Vₖ CDR2 of 2A10 is shown in SEQ ID NO: 26. The amino acid sequence of the Vₖ CDR3 of 2A10 is shown in SEQ ID NO: 30. The CDR regions are delineated using the Kabat system (Kabat, E. A., *et al.* (1991) Sequences of Proteins of Immunological Interest, Fifth Edition, U.S. Department of Health and Human Services, NIH Publication No. 91-3242).

In a preferred embodiment, the antibody comprises:
(a) a heavy chain variable region CDR1 comprising SEQ ID NO: 10;
(b) a heavy chain variable region CDR2 comprising SEQ ID NO: 14;
(c) a heavy chain variable region CDR3 comprising SEQ ID NO: 18;
(d) a light chain variable region CDR1 comprising SEQ ID NO: 22;
(e) a light chain variable region CDR2 comprising SEQ ID NO: 26; and
(f) a light chain variable region CDR3 comprising SEQ ID NO: 30.

### Nucleic Acid Molecules Encoding Antibodies of the Invention

Another aspect of the invention pertains to nucleic acid molecules that encode the antibodies of the invention. The nucleic acids may be present in whole cells, in a cell lysate, or in a partially purified or substantially pure form. A nucleic acid is "isolated" or "rendered substantially pure" when purified away from other cellular components or other contaminants, e.g., other cellular nucleic acids or proteins, by standard techniques, including alkaline/SDS treatment, CsCl banding, column chromatography, agarose gel electrophoresis and others well known in the art. See, F. Ausubel, et al, ed. (1987) Current Protocols in Molecular Biology, Greene Publishing and Wiley Interscience, New York. A nucleic acid of the invention can be, for example, DNA or RNA and may or may not contain intronic sequences. In a preferred embodiment, the nucleic acid is a cDNA molecule.

Nucleic acids of the invention can be obtained using standard molecular biology techniques. For antibodies expressed by hybridomas (e.g., hybridomas prepared from transgenic mice carrying human immunoglobulin genes as described further below), cDNAs encoding the light and heavy chains of the antibody made by the hybridoma can be obtained by standard PCR amplification or cDNA cloning techniques. For antibodies obtained from an immunoglobulin gene library (e.g., using phage display techniques), nucleic acid encoding the antibody can be recovered from the library.

Preferred nucleic acids molecules of the invention are those encoding the VH and VL sequences of the 2A10 monoclonal antibody. DNA sequence encoding the VH sequence of 2A10 is shown in SEQ ID NO: 34. DNA sequence encoding the VL sequence of 2A10 is shown in SEQ ID NO: 38.

Once DNA fragments encoding VH and VL segments are obtained, these DNA fragments can be further manipulated by standard recombinant DNA techniques, for example to convert the variable region genes to full-length antibody chain genes, to Fab fragment genes or to a scFv gene. In these manipulations, a VL-or VH-encoding DNA fragment is operatively linked to another DNA fragment encoding another protein, such as an antibody constant region or a flexible linker. The term "operatively linked", as used in this context, is intended to mean that the two DNA fragments are joined such that the amino acid sequences encoded by the two DNA fragments remain in-frame.

The isolated DNA encoding the VH region can be converted to a full-length heavy chain gene by operatively linking the VH-encoding DNA to another DNA molecule encoding heavy chain constant regions (CH1, CH2 and CH3). The sequences of human heavy chain constant region genes are known in the art (see e.g., Kabat, E. A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region, but most preferably is an IgG1 or IgG4 constant region. For a Fab fragment heavy chain gene, the VH-encoding DNA can be operatively linked to another DNA molecule encoding only the heavy chain CH1 constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see *e.g.,* Kabat, E. A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or lambda constant region, but most preferably is a kappa constant region.

To create a scFv gene, the VH- and VL-encoding DNA fragments are operatively linked to another fragment encoding a flexible linker, *e.g*., encoding the amino acid sequence (Gly₄ -Ser)₃ (SEQ ID NO: 48), such that the VH and VL sequences can be expressed as a contiguous single-chain protein, with the VL and VH regions joined by the flexible linker (see *e.g.,* Bird et al. (1988) Science 242:423-426; Huston et al. (1988) Proc. Natl. Acad. Sci. USA 85:5879-5883; McCafferty et al., (1990) Nature 348:552-554).

### Production of Monoclonal Antibodies of the Invention

Monoclonal antibodies (mAbs) of the present invention can be produced by a variety of techniques, including conventional monoclonal antibody methodology *e.g*., the standard somatic cell hybridization technique of Kohler and Milstein (1975) Nature 256: 495. Although somatic cell hybridization procedures are preferred, in principle, other techniques for producing monoclonal antibody can be employed *e.g.,* viral or oncogenic transformation of B lymphocytes.

The preferred animal system for preparing hybridomas is the murine system. Hybridoma production in the mouse is a very well-established procedure. Immunization protocols and techniques for isolation of immunized splenocytes for fusion are known in the art. Fusion partners (*e.g*., murine myeloma cells) and fusion procedures are also known.

Chimeric or humanized antibodies of the present invention can be prepared based on the sequence of a murine monoclonal antibody prepared as described above. DNA encoding the heavy and light chain immunoglobulins can be obtained from the murine hybridoma of interest and engineered to contain non-murine (*e.g*., human) immunoglobulin sequences using standard molecular biology techniques. For example, to create a chimeric antibody, the murine variable regions can be linked to human constant regions using methods known in the art (see *e.g*., U.S. Patent No. 4,816,567 to Cabilly et al.*).* To create a humanized antibody, the murine CDR regions can be inserted into a human framework using methods known in the art (see *e.g.,* U.S. Patent No. 5,225,539 to Winter, and U.S. Patent Nos. 5,530,101; 5,585,089; 5,693,762 and 6,180,370 to Queen et al.*).*

In a preferred embodiment, the antibodies of the invention are human monoclonal antibodies. Such human monoclonal antibodies directed against PSMA can be generated using transgenic or transchromosomic mice carrying parts of the human immune system rather than the mouse system. These transgenic and transchromosomic mice include mice referred to herein as HuMAb mice and KM mice^{™}, respectively, and are collectively referred to herein as "human Ig mice."

The HuMAb mouse@ (Medarex, Inc.) contains human immunoglobulin gene miniloci that encode unrearranged human heavy (µ and γ) and κ light chain immunoglobulin sequences, together with targeted mutations that inactivate the endogenous µ, and κ chain loci (see e.g., Lonberg, et al. (1994) Nature 368(6474): 856-859). Accordingly, the mice exhibit reduced expression of mouse IgM or κ, and in response to immunization, the introduced human heavy and light chain transgenes undergo class switching and somatic mutation to generate high affinity human IgGκ monoclonal (Lonberg, N. *et al.* (1994), *supra;* reviewed in Lonberg, N. (1994) Handbook of Experimental Pharmacology 113:49-101; Lonberg, N. and Huszar, D. (1995) Intern. Rev. Immunol. 13: 65-93, and Harding, F. and Lonberg, N. (1995) Ann. N.Y. Acad. Sci. 764:536-546). The preparation and use ofHuMab mice, and the genomic modifications carried by such mice, is further described in Taylor, L. et al. (1992) Nucleic Acids Research 20:6287-6295; Chen, J. et al. (1993) International Immunology 5: 647-656; Tuaillon et al. (1993) Proc. Natl. Acad. Sci. USA 90:3720-3724; Choi et al. (1993) Nature Genetics 4:117-123; Chen, J. et al. (1993) EMBO J. 12: 821-830; Tuaillon et al. (1994) J. Immunol. 152:2912-2920; Taylor, L. et al. (1994) International Immunology 6: 579-591; and Fishwild, D. et al. (1996) Nature Biotechnology 14: 845-851, the contents of all of which are hereby specifically incorporated by reference in their entirety. See further, U.S. Patent Nos. 5,545,806; 5,569,825; 5,625,126; 5,633,425; 5,789,650; 5,877,397; 5,661,016; 5,814,318; 5,874,299; and 5,770,429; all to Lonberg and Kay; U.S. Patent No. 5,545,807 to Surani et al.*;* PCT Publication Nos. WO 92/03918, WO 93/12227, WO 94/25585, WO 97/13852, WO 98/24884 and WO 99/45962, all to Lonberg and Kay; and PCT Publication No. WO 01/14424 to Korman et al.

In another embodiment, human antibodies of the invention can be raised using a mouse that carries human immunoglobulin sequences on transgenes and transchomosomes, such as a mouse that carries a human heavy chain transgene and a human light chain transchromosome. Such mice, referred to herein as "KM mice^{™}", are described in detail in PCT Publication WO 02/43478 to Ishida et al.

Still further, alternative transgenic animal systems expressing human immunoglobulin genes are available in the art and can be used to raise anti-PSMA antibodies of the invention. For example, an alternative transgenic system referred to as the Xenomouse (Abgenix, Inc.) can be used; such mice are described in, for example, U.S. Patent Nos. 5,939,598; 6,075,181; 6,114,598; 6, 150,584 and 6,162,963 to Kucherlapati et al.

Moreover, alternative transchromosomic animal systems expressing human immunoglobulin genes are available in the art and can be used to raise anti-PSMA antibodies of the invention. For example, mice carrying both a human heavy chain transchromosome and a human light chain tranchromosome, referred to as "TC mice" can be used; such mice are described in Tomizuka et al. (2000) Proc. Natl. Acad Sci. USA 97:722-727. Furthermore, cows carrying human heavy and light chain transchromosomes have been described in the art (Kuroiwa et al. (2002) Nature Biotechnology 20:889-894) and can be used to raise anti-PSMA antibodies of the invention.

Human monoclonal antibodies of the invention can also be prepared using phage display methods for screening libraries of human immunoglobulin genes. Such phage display methods for isolating human antibodies are established in the art. See for example: U.S. Patent Nos. 5,223,409; 5,403,484; and 5,571,698 to Ladner et al.*;* U.S. Patent Nos. 5,427,908 and 5,580,717 to Dower et al.*;* U.S. Patent Nos. 5,969,108 and 6,172,197 to McCafferty et al.*;* and U.S. Patent Nos. 5,885,793; 6,521,404; 6,544,731; 6,555,313; 6,582,915 and 6,593,081 to Griffiths et al.

Human monoclonal antibodies of the invention can also be prepared using SCID mice into which human immune cells have been reconstituted such that a human antibody response can be generated upon immunization. Such mice are described in, for example, U.S. Patent Nos. 5,476,996 and 5,698,767 to Wilson et al.

### Immunization of Human Ig Mice

When human Ig mice are used to raise human antibodies of the invention, such mice can be immunized with a PSMA-expressing cell line, such as the LNCaP cell line (ATCC CRL-1740), a purified or enriched preparation of PSMA antigen and/or recombinant PSMA, or an PSMA fusion protein, as described by Lonberg, N. et al. (1994) Nature 368(6474): 856-859; Fishwild, D. et al. (1996) Nature Biotechnology 14: 845-851; and PCT Publication WO 98/24884 and WO 01/14424. Preferably, the mice will be 6-16 weeks of age upon the first infusion. For example, a purified or recombinant preparation (5-50 µg) of PSMA antigen can be used to immunize the human Ig mice intraperitoneally.

Detailed procedures to generate fully human monoclonal antibodies to PSMA are described in Example 1 below. Cumulative experience with various antigens has shown that the transgenic mice respond when initially immunized intraperitoneally (IP) with antigen in complete Freund's adjuvant, followed by every other week IP immunizations up to a total of 6) with antigen in incomplete Freund's adjuvant. However, adjuvants other than Freund's are also found to be effective. In addition, whole cells in the absence of adjuvant are found to be highly immunogenic. The immune response can be monitored over the course of the immunization protocol with plasma samples being obtained by retroorbital bleeds. The plasma can be screened by ELISA (as described below), and mice with sufficient titers of anti-PSMA human immunoglobulin can be used for fusions. Mice can be boosted intravenously with antigen 3 days before sacrifice and removal of the spleen. It is expected that 2-3 fusions for each immunization may need to be performed. Between 6 and 24 mice are typically immunized for each antigen. Usually both HCo7 and HCo12 strains are used. In addition, both HCo7 and HCo12 transgene can be bred together into a single mouse having two different human heavy chain transgenes (HCo7/HCo12). Alternatively or additionally, the KM mouse^{™} strain can be used.

### Generation of Hybridomas Producing Human Monoclonal Antibodies of the Invention

To generate hybridomas producing human monoclonal antibodies of the invention, splenocytes and/or lymph node cells from immunized mice can be isolated and fused to an appropriate immortalized cell line, such as a mouse myeloma cell line. The resulting hybridomas can be screened for the production of antigen-specific antibodies. For example, single cell suspensions of splenic lymphocytes from immunized mice can be fused to one-sixth the number of P3X63-Ag8.653 nonsecreting mouse myeloma cells (ATCC, CRL 1580) with 50% PEG. Cells are plated at approximately 2 x 10⁵ in flat bottom microtiter plate, followed by a two week incubation in selective medium containing 20% fetal Clone Serum, 18% "653" conditioned media, 5% origen (IGEN), 4 mM L-glutamine, 1 mM sodium pyruvate, 5mM HEPES, 0.055 mM 2-mercaptoethanol, 50 units/ml penicillin, 50 mg/ml streptomycin, 50 mg/ml gentamycin and 1X HAT (Sigma; the HAT is added 24 hours after the fusion). After approximately two weeks, cells can be cultured in medium in which the HAT is replaced with HT. Individual wells can then be screened by ELISA for human monoclonal IgM and IgG antibodies. Once extensive hybridoma growth occurs, medium can be observed usually after 10-14 days. The antibody secreting hybridomas can be replated, screened again, and if still positive for human IgG, the monoclonal antibodies can be subcloned at least twice by limiting dilution. The stable subclones can then be cultured *in vitro* to generate small amounts of antibody in tissue culture medium for characterization.

To purify human monoclonal antibodies, selected hybridomas can be grown in two-liter spinner-flasks for monoclonal antibody purification. Supernatants can be filtered and concentrated before affinity chromatography with protein A-sepharose (Pharmacia, Piscataway, N.J.). Eluted IgG can be checked by gel electrophoresis and high performance liquid chromatography to ensure purity. The buffer solution can be exchanged into PBS, and the concentration can be determined by OD280 using 1.43 extinction coefficient. The monoclonal antibodies can be aliquoted and stored at -80° C.

### Generation of Transfectomas Producing Monoclonal Antibodies of the Invention

Antibodies of the invention also can be produced in a host cell transfectoma using, for example, a combination of recombinant DNA techniques and gene transfection methods as is well known in the art (e.g., Morrison, S. (1985) Science 229:1202).

For example, to express the antibodies, or antibody fragments thereof, DNAs encoding partial or full-length light and heavy chains, can be obtained by standard molecular biology techniques (e.g., PCR amplification or cDNA cloning using a hybridoma that expresses the antibody of interest) and the DNAs can be inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (*e.g*., ligation of complementary restriction sites on the antibody gene fragment and vector, or blunt end ligation if no restriction sites are present). The light and heavy chain variable regions of the antibodies described herein can be used to create full-length antibody genes of any antibody isotype by inserting them into expression vectors already encoding heavy chain constant and light chain constant regions of the desired isotype such that the V_{H} segment is operatively linked to the C_{H} segment(s) within the vector and the V_{K} segment is operatively linked to the C_{L} segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (*i.e*., a signal peptide from a non-immunoglobulin protein).

In addition to the antibody chain genes, the recombinant expression vectors of the invention carry regulatory sequences that control the expression of the antibody chain genes in a host cell. The term "regulatory sequence" is intended to include promoters, enhancers and other expression control elements (*e.g*., polyadenylation , signals) that control the transcription or translation of the antibody chain genes. Such regulatory sequences are described, for example, in Goeddel (Gene Expression Technology. Methods in Enzymology 185, Academic Press, San Diego, CA (1990)). It will be appreciated by those skilled in the art that the design of the expression vector, including the selection of regulatory sequences, may depend on such factors as the choice of the host cell to be transformed, the level of expression of protein desired, *etc.* Preferred regulatory sequences for mammalian host cell expression include viral elements that direct high levels of protein expression in mammalian cells, such as promoters and/or enhancers derived from cytomegalovirus (CMV), Simian Virus 40 (SV40), adenovirus, (*e.g*., the adenovirus major late promoter (AdMLP) and polyoma. Alternatively, nonviral regulatory sequences may be used, such as the ubiquitin promoter or β-globin promoter. Still further, regulatory elements composed of sequences from different sources, such as the SRα promoter system, which contains sequences from the SV40 early promoter and the long terminal repeat of human T cell leukemia virus type 1 (Takebe, Y. et al. (1988) Mol. Cell. Biol. 8:466-472).

In addition to the antibody chain genes and regulatory sequences, the recombinant expression vectors of the invention may carry additional sequences, such as sequences that regulate replication of the vector in host cells (*e.g*., origins of replication) and selectable marker genes. The selectable marker gene facilitates selection of host cells into which the vector has been introduced (see, *e.g*., U.S. Pat. Nos. 4,399,216, 4,634,665 and 5,179,017, all by Axel *et al.).* For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr- host cells with methotrexate selection/amplification) and the neo gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss, M. A. and Wood, C. R. (1985) Immunology Today 6:12-13).

Preferred mammalian host cells for expressing the recombinant antibodies of the invention include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, *e.g*., as described in R. J. Kaufman and P. A. Sharp (1982) Mol. Biol. 159:601-621), NSO myeloma cells, COS cells and SP2 cells. In particular, for use with NSO myeloma cells, another preferred expression system is the GS gene expression system disclosed in WO 87/04462, WO 89/01036 and EP 338,841. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

### Characterization of Antibody Binding to Antigen

Antibodies of the invention can be tested for binding to PSMA by, for example, flow cytometry. Briefly, LNCaP cells are freshly harvested from tissue culture flasks and a single cell suspension prepared. LNCaP cell suspensions are either stained with primary antibody directly or after fixation with 1% paraformaldehyde in PBS. Approximately one million cells are resuspended in PBS containing 0.5% BSA and 50-200 µg/ml of primary antibody and incubated on ice for 30 minutes. The cells are washed twice with PBS containing 0.1% BSA, 0.01% NaN₃, resuspended in 100 µl of 1:100 diluted FITC-conjugated goat-anti-human IgG (Jackson ImmunoResearch, West Grove, PA), and incubated on ice for an additional 30 minutes. The cells are again washed twice, resuspended in 0.5 ml of wash buffer and analyzed for fluorescent staining on a FACSCalibur cytometer (Becton-Dickinson, San Jose, CA).

Alternatively, antibodies of the invention can be tested for binding to PSMA by standard ELISA. Briefly, microtiter plates are coated with purified PSMA at 0.25 µg/ml in PBS, and then blocked with 5% bovine serum albumin in PBS. Dilutions of antibody (*e.g*., dilutions of plasma from PSMA-immunized mice) are added to each well and incubated for 1-2 hours at 37°C. The plates are washed with PBS/Tween and then incubated with secondary reagent (*e.g.*, for human antibodies, a goat-anti-human IgG Fc-specific polyclonal reagent) conjugated to alkaline phosphatase for 1 hour at 37°C. After washing, the plates are developed with pNPP substrate (1 mg/ml), and analyzed at OD of 405-650. Preferably, mice which develop the highest titers will be used for fusions.

An ELISA assay as described above can also be used to screen for hybridomas that show positive reactivity with PSMA immunogen. Hybridomas that bind with high avidity to PSMA are subcloned and further characterized. One clone from each hybridoma, which retains the reactivity of the parent cells (by ELISA), can be chosen for making a 5-10 vial cell bank stored at -140 °C, and for antibody purification.

To purify anti-PSMA antibodies, selected hybridomas can be grown in two-liter spinner-flasks for monoclonal antibody purification. Supernatants can be filtered and concentrated before affinity chromatography with protein A-sepharose (Pharmacia, Piscataway, NJ). Eluted IgG can be checked by gel electrophoresis and high performance liquid chromatography to ensure purity. The buffer solution can be exchanged into PBS, and the concentration can be determined by OD₂₈₀ using 1.43 extinction coefficient. The monoclonal antibodies can be aliquoted and stored at -80 °C.

To determine if the selected anti-PSMA monoclonal antibodies bind to unique epitopes, each antibody can be biotinylated using commercially available reagents (Pierce, Rockford, IL). Competition studies using unlabeled monoclonal antibodies and biotinylated monoclonal antibodies can be performed using PSMA coated-ELISA plates as described above. Biotinylated mAb binding can be detected with a strep-avidin-alkaline phosphatase probe. Alternatively, competition studies can be performed using radiolabelled antibody and unlabelled competing antibodies can be detected in a Scatchard analysis, as further described in the Examples below.

To determine the isotype of purified antibodies, isotype ELISAs can be performed using reagents specific for antibodies of a particular isotype. For example, to determine the isotype of a human monoclonal antibody, wells of microtiter plates can be coated with 1 µg/ml of anti-human immunoglobulin overnight at 4° C. After blocking with 1% BSA, the plates are reacted with 1 µg /ml or less of test monoclonal antibodies or purified isotype controls, at ambient temperature for one to two hours. The wells can then be reacted with either human IgGl or human IgM-specific alkaline phosphatase-conjugated probes. Plates are developed and analyzed as described above.

Anti-PSMA human IgGs can be further tested for reactivity with PSMA antigen by Western blotting. Briefly, PSMA can be prepared and subjected to sodium dodecyl sulfate polyacrylamide gel electrophoresis. After electrophoresis, the separated antigens are transferred to nitrocellulose membranes, blocked with 10% fetal calf serum, and probed with the monoclonal antibodies to be tested. Human IgG binding can be detected using anti-human IgG alkaline phosphatase and developed with BCIP/NBT substrate tablets (Sigma Chem. Co., St. Louis, Mo.).

### Immunoconjugates

In another aspect, the present invention features an anti-PSMA antibody, or a fragment thereof, conjugated to a therapeutic moiety, such as a cytotoxin, a drug (*e.g*., an immunosuppressant) or a radiotoxin. Such conjugates are referred to herein as "immunoconjugates". Immunoconjugates that include one or more cytotoxins are referred to as "immunotoxins." A cytotoxin or cytotoxic agent includes any agent that is detrimental to (*e.g*., kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents also include, for example, antimetabolites (*e.g*., methotrexate, 6-mercaptopurine, 6-thioguanine, cytarabine, 5-fluorouracil decarbazine), alkylating agents (*e.g*., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g.*, daunorubicin (formerly daunomycin) and doxorubicin), antibiotics (*e.g*., dactinomycin (formerly actinomycin), bleomycin, mithramycin, and anthramycin (AMC)), and anti-mitotic agents (*e.g.*, vincristine and vinblastine).

Other preferred examples of therapeutic cytotoxins that can be conjugated to an antibody of the invention include duocarmycins, calicheamicins, maytansines and auristatins, and derivatives thereof. An example of a calicheamicin antibody conjugate is commercially available (Mylotarg™; Wyeth-Ayerst).

Cytoxins can be conjugated to antibodies of the invention using linker technology available in the art. Examples of linker types that have been used to conjugate a cytotoxin to an antibody include, but are not limited to, hydrazones, thioethers, esters, disulfides and peptide-containing linkers. A linker can be chosen that is, for example, susceptible to cleavage by low pH within the lysosomal compartment or susceptible to cleavage by proteases, such as proteases preferentially expressed in tumor tissue such as cathepsins (*e.g*., cathepsins B, C, D).

For further discussion of types of cytotoxins, linkers and methods for conjugating therapeutic agents to antibodies, see also Saito, G. et al. (2003) Adv. Drug Deliv. Rev. 55:199-215; Trail, P.A. et al. (2003) Cancer Immunol. Immunother. 52:328-337; Payne, G. (2003) Cancer Cell 3:207-212; Allen, T.M. (2002) Nat. Rev. Cancer 2:750-763; Pastan, I. and Kreitman, R. J. (2002) Curr. Opin. Investig. Drugs 3:1089-1091; Senter, P.D. and Springer, C.J. (2001) Adv. Drug Deliv. Rev. 53:247-264.

Antibodies of the present invention also can be conjugated to a radioactive isotope to generate cytotoxic radiopharmaceuticals, also referred to as radioimmunoconjugates. Examples of radioactive isotopes that can be conjugated to antibodies for use diagnostically or therapeutically include, but are not limited to, iodine¹³¹, iodine¹²⁵, indium¹¹¹, yttrium⁹⁰ and lutetium¹⁷⁷. Method for preparing radioimmunconjugates are established in the art. Examples of radioimmunoconjugates are commercially available, including Zevalin™ (IDEC Pharmaceuticals) and Bexxar™ (Corixa Pharmaceuticals), and similar methods can be used to prepare radioimmunoconjugates using the antibodies of the invention.

The antibody conjugates of the invention can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-γ; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known, see, *e.g*., Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982).

### Bispecific Molecules

In another aspect, the present invention features bispecific molecules comprising an anti-PSMA antibody, or a fragment thereof, of the invention. An antibody of the invention, or antigen-binding portions thereof, can be derivatized or linked to another functional molecule, *e.g*., another peptide or protein (*e.g*., another antibody or ligand for a receptor) to generate a bispecific molecule that binds to at least two different binding sites or target molecules. The antibody of the invention may in fact be derivatized or linkd to more than one other functional molecule to generate multispecific molecules that bind to more than two different binding sites and/or target molecules; such multispecific molecules are also intended to be encompassed by the term "bispecific molecule" as used herein. To create a bispecific molecule of the invention, an antibody of the invention can be functionally linked (*e.g*., by chemical coupling, genetic fusion, noncovalent association or otherwise) to one or more other binding molecules, such as another antibody, antibody fragment, peptide or binding mimetic, such that a bispecific molecule results.

Accordingly, the present invention includes bispecific molecules comprising at least one first binding specificity for PSMA and a second binding specificity for a second target epitope. In a particular embodiment of the invention, the second target epitope is an Fc receptor, *e.g*., human FcγRI (CD64) or a human Fcα receptor (CD89). Therefore, the invention includes bispecific molecules capable of binding both to FcγR or FcαR expressing effector cells (*e.g*., monocytes, macrophages or polymorphonuclear cells (PMNs)), and to target cells expressing PSMA. These bispecific molecules target PSMA expressing cells to effector cell and trigger Fc receptor-mediated effector cell activities, such as phagocytosis of an PSMA expressing cells, antibody dependent cell-mediated cytotoxicity (ADCC), cytokine release, or generation of superoxide anion.

In an embodiment of the invention in which the bispecific molecule is multispecific, the molecule can further include a third binding specificity, in addition to an anti-Fc binding specificity and an anti-PSMA binding specificity. In one embodiment, the third binding specificity is an anti-enhancement factor (EF) portion, *e.g*., a molecule which binds to a surface protein involved in cytotoxic activity and thereby increases the immune response against the target cell. The "anti-enhancement factor portion" can be an antibody, functional antibody fragment or a ligand that binds to a given molecule, *e.g*., an antigen or a receptor, and thereby results in an enhancement of the effect of the binding determinants for the F_{c} receptor or target cell antigen. The "anti-enhancement factor portion" can bind an F_{c} receptor or a target cell antigen. Alternatively, the anti-enhancement factor portion can bind to an entity that is different from the entity to which the first and second binding specificities bind. For example, the anti-enhancement factor portion can bind a cytotoxic T-cell (*e.g*. via CD2, CD3, CD8, CD28, CD4, CD40, ICAM-1 or other immune cell that results in an increased immune response against the target cell).

In one embodiment, the bispecific molecules of the invention comprise as a binding specificity at least one antibody, or an antibody fragment thereof, including, *e.g*., an Fab, Fab', F(ab')₂, Fv, or a single chain Fv. The antibody may also be a light chain or heavy chain dimer, or any minimal fragment thereof such as a Fv or a single chain construct as described in Ladner et al. U.S. Patent No. 4,946,778, the contents of which is expressly incorporated by reference.

In one embodiment, the binding specificity for an Fcγ receptor is provided by a monoclonal antibody, the binding of which is not blocked by human immunoglobulin G (IgG). As used herein, the term "IgG receptor" refers to any of the eight γ-chain genes located on chromosome 1. These genes encode a total of twelve transmembrane or soluble receptor isoforms which are grouped into three Fcγ receptor classes: FcγRI (CD64), FcγRII(CD32), and FcγRIII (CD16). In one preferred embodiment, the Fcγ receptor a human high affinity FcγRI. The human FcγRI is a 72 kDa molecule, which shows high affinity for monomeric IgG (10⁸ - 10⁹ M⁻¹).

The production and characterization of certain preferred anti-Fcγ monoclonal antibodies are described by Fanger et al. in PCT Publication WO 88/00052 and in U.S. Patent No. 4,954,617, the teachings of which are fully incorporated by reference herein. These antibodies bind to an epitope of FcγRI, FcγRII or FcγRIII at a site which is distinct from the Fcγ binding site of the receptor and, thus, their binding is not blocked substantially by physiological levels of IgG. Specific anti-FcγRI antibodies useful in this invention are mAb 22, mAb 32, mAb 44, mAb 62 and mAb 197. The hybridoma producing mAb 32 is available from the American Type Culture Collection, ATCC Accession No. HB9469. In other embodiments, the anti-Fcγ receptor antibody is a humanized form of monoclonal antibody 22 (H22). The production and characterization of the H22 antibody is described in Graziano, R.F. et al. (1995) J. Immunol 155 (10): 4996-5002 and PCT Publication WO 94/10332. The H22 antibody producing cell line was deposited at the American Type Culture Collection under the designation HA022CL1 and has the accession no. CRL 11177.

In still other preferred embodiments, the binding specificity for an Fc receptor is provided by an antibody that binds to a human IgA receptor, *e.g*., an Fc-alpha receptor (FcαRI (CD89)), the binding of which is preferably not blocked by human immunoglobulin A (IgA). The term "IgA receptor" is intended to include the gene product of one α-gene (FcαRI) located on chromosome 19. This gene is known to encode several alternatively spliced transmembrane isoforms of 55 to 110 kDa. FcαRI (CD89) is constitutively expressed on monocytes/macrophages, eosinophilic and neutrophilic granulocytes, but not on non-effector cell populations. FcαRI has medium affinity (≈ 5 × 10⁷ M⁻¹) for both IgA1 and IgA2, which is increased upon exposure to cytokines such as G-CSF or GM-CSF (Morton, H.C. et al. (1996) Critical Reviews in Immunology 16:423-440). Four FcαRI-specific monoclonal antibodies, identified as A3, A59, A62 and A77, which bind FcαRI outside the IgA ligand binding domain, have been described (Monteiro, R.C. et al. (1992) J. Immunol. 148:1764).

FcαRI and FcγRI are preferred trigger receptors for use in the bispecific molecules of the invention because they are (1) expressed primarily on immune effector cells, e.g., monocytes, PMNs, macrophages and dendritic cells; (2) expressed at high levels (*e.g.,* 5,000-100,000 per cell); (3) mediators of cytotoxic activities (*e.g.,* ADCC, phagocytosis); (4) mediate enhanced antigen presentation of antigens, including self-antigens, targeted to them.

While human monoclonal antibodies are preferred, other antibodies which can be employed in the bispecific molecules of the invention are murine, chimeric and humanized monoclonal antibodies.

The bispecific molecules of the present invention can be prepared by conjugating the constituent binding specificities, *e.g*., the anti-FcR and anti-PSMA binding specificities, using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-S-acetylthioacetate (SATA), 5,5'-dithiobis(2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl) cyclohaxane-1-carboxylate (sulfo-SMCC) (see *e.g.,* Karpovsky et al. (1984) J. Exp. Med. 160:1686; Liu, MA et al. (1985) Proc. Natl. Acad. Sci. USA 82:8648). Other methods include those described in Paulus (1985) Behring Ins. Mitt. No. 78, 118-132; Brennan et al. (1985) Science 229:81-83), and Glennie et al. (1987) J. Immunol. 139: 2367-2375). Preferred conjugating agents are SATA and sulfo-SMCC, both available from Pierce Chemical Co. (Rockford, IL). '

When the binding specificities are antibodies, they can be conjugated via sulfhydryl bonding of the C-terminus hinge regions of the two heavy chains. In a particularly preferred embodiment, the hinge region is modified to contain an odd number of sulfhydryl residues, preferably one, prior to conjugation.

Alternatively, both binding specificities can be encoded in the same vector and expressed and assembled in the same host cell. This method is particularly useful where the bispecific molecule is a mAb x mAb, mAb x Fab, Fab x F(ab')₂ or ligand x Fab fusion protein. A bispecific molecule of the invention can be a single chain molecule comprising one single chain antibody and a binding determinant, or a single chain bispecific molecule comprising two binding determinants. Bispecific molecules may comprise at least two single chain molecules. Methods for preparing bispecific molecules are described for example in U.S. Patent Number 5,260,203; U.S. Patent Number 5,455,030; U.S. Patent Number 4,881,175; U.S. Patent Number 5,132,405; U.S. Patent Number 5,091,513; U.S. Patent Number 5,476,786; U.S. Patent Number 5,013,653; U.S. Patent Number 5,258,498; and U.S. Patent Number 5,482,858.

Binding of the bispecific molecules to their specific targets can be confirmed by, for example, enzyme-linked immunosorbent assay (ELISA), radioimmunoassay (RIA), FACS analysis, bioassay (*e.g*., growth inhibition), or Western Blot assay. Each of these assays generally detects the presence of protein-antibody complexes of particular interest by employing a labeled reagent (*e.g*., an antibody) specific for the complex of interest. For example, the FcR-antibody complexes can be detected using *e.g.,* an enzyme-linked antibody or antibody fragment which recognizes and specifically binds to the antibody-FcR complexes. Alternatively, the complexes can be detected using any of a variety of other immunoassays. For example, the antibody can be radioactively labeled and used in a radioimmunoassay (RIA) (see, for example, Weintraub, B., Principles of Radioimmunoassays, Seventh Training Course on Radioligand Assay Techniques, The Endocrine Society, March, 1986, which is incorporated by reference herein). The radioactive isotope can be detected by such means as the use of a γ counter or a scintillation counter or by autoradiography.

### Pharmaceutical Compositions

In another aspect, the present invention provides a composition, *e.g*., a pharmaceutical composition, containing one or a combination of monoclonal antibodies, or antigen-binding portion(s) thereof, of the present invention, formulated together with a pharmaceutically acceptable carrier. Such compositions may include one or a combination of (*e.g*., two or more different) antibodies, or immunoconjugates or bispecific molecules of the invention. For example, a pharmaceutical composition of the invention can comprise a combination of antibodies (or immunoconjugates or bispecifics) that bind to different epitopes on the target antigen or that have complementary activities.

Pharmaceutical compositions of the invention also can be administered in combination therapy, *i.e*., combined with other agents. For example, the combination therapy can include an anti-PSMA antibody of the present invention combined with at least one other anti-inflammatory or immunosuppressant agent. Examples of therapeutic agents that can be used in combination therapy are described in greater detail below in the section on uses of the antibodies of the invention.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (*e.g*., by injection or infusion). Depending on the route of administration, the active compound, *i.e.,* antibody, immunoconjuage, or bispecific molecule, may be coated in a material to protect the compound from the action of acids and other natural conditions that may inactivate the compound.

The pharmaceutical compounds of the invention may include one or more pharmaceutically acceptable salts. A "pharmaceutically acceptable salt" refers to a salt that retains the desired biological activity of the parent compound and does not impart any undesired toxicological effects (see *e.g*., Berge, S.M., et al. (1977) J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A pharmaceutical composition of the invention also may include a pharmaceutically acceptable anti-oxidant. Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Examples of suitable aqueous and nonaqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, *supra,* and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred per cent, this amount will range from about 0.01 per cent to about ninety-nine percent of active ingredient, preferably from about 0.1 per cent to about 70 per cent, most preferably from about 1 per cent to about 30 per cent of active ingredient in combination with a pharmaceutically acceptable carrier.

Dosage regimens are adjusted to provide the optimum desired response (*e.g*., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

For administration of the antibody, the dosage ranges from about 0.0001 to 100 mg/kg, and more usually 0.01 to 5 mg/kg, of the host body weight. For example dosages can be 0.3 mg/kg body weight, 1 mg/kg body weight, 3 mg/kg body weight, 5 mg/kg body weight or 10 mg/kg body weight or within the range of 1-10 mg/kg. An exemplary treatment regime entails administration once per week, once every two weeks, once every three weeks, once every four weeks, once a month, once every 3 months or once every three to 6 months. Preferred dosage regimens for an anti-PSMA antibody of the invention include 1 mg/kg body weight or 3 mg/kg body weight via intravenous administration, with the antibody being given using one of the following dosing schedules: (i) every four weeks for six dosages, then every three months; (ii) every three weeks; (iii) 3 mg/kg body weight once followed by 1 mg/kg body weight every three weeks.

In some methods, two or more monoclonal antibodies with different binding specificities are administered simultaneously, in which case the dosage of each antibody administered falls within the ranges indicated. Antibody is usually administered on multiple occasions. Intervals between single dosages can be, for example, weekly, monthly, every three monthgs or yearly. Intervals can also be irregular as indicated by measuring blood levels of antibody to the target antigen in the patient. In some methods, dosage is adjusted to achieve a plasma antibody concentration of about 1-1000 µg /ml and in some methods about 25-300 µg /ml.

Alternatively, antibody can be administered as a sustained release formulation, in which case less frequent administration is required. Dosage and frequency vary depending on the half-life of the antibody in the patient. In general, human antibodies show the longest half life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A "therapeutically effective dosage" of an anti-PSMA antibody of the invention preferably results in a decrease in severity of disease symptoms, an increase in frequency and duration of disease symptom-free periods, or a prevention of impairment or disability due to the disease affliction. For example, for the treatment of PSMA+ tumors, a "therapeutically effective dosage" preferably inhibits cell growth or tumor growth by at least about 20%, more preferably by at least about 40%, even more preferably by at least about 60%, and still more preferably by at least about 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subject. One of ordinary skill in the art would be able to determine such amounts based on such factors as the subject's size, the severity of the subject's symptoms, and the particular composition or route of administration selected.

A composition of the present invention can be administered via one or more routes of administration using one or more of a variety of methods known in the art. As will be appreciated by the skilled artisan, the route and/or mode of administration will vary depending upon the desired results. Preferred routes of administration for antibodies of the invention include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Alternatively, an antibody of the invention can be administered via a non-parenteral route, such as a topical, epidermal or mucosal route of administration, for example, intranasally, orally, vaginally, rectally, sublingually or topically.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. *See, e.g.,* Sustained and Controlled Release Drug Delivery Systems, J.R. Robinson, ed., Marcel Dekker, Inc., New York, 1978.

Therapeutic compositions can be administered with medical devices known in the art. For example, in a preferred embodiment, a therapeutic composition of the invention can be administered with a needleless hypodermic injection device, such as the devices disclosed in U.S. Patent Nos. 5,399,163; 5,383,851; 5,312,335; 5,064,413; 4,941,880; 4,790,824; or 4,596,556. Examples of well-known implants and modules useful in the present invention include: U.S. Patent No. 4,487,603, which discloses an implantable micro-infusion pump for dispensing medication at a controlled rate; U.S. Patent No. 4,486,194, which discloses a therapeutic device for administering medicants through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. These patents are incorporated herein by reference. Many other such implants, delivery systems, and modules are known to those skilled in the art.

In certain embodiments, the human monoclonal antibodies of the invention can be formulated to ensure proper distribution *in vivo.* For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, *e.g.,* U.S. Patents 4,522,811; 5,374,548; and 5,399,331. The liposomes may comprise one or more moieties which are selectively transported into specific cells or organs, thus enhance targeted drug delivery (*see, e.g.,* V.V. Ranade (1989) J. Clin. Pharmacol. 29:685). Exemplary targeting moieties include folate or biotin (see, *e.g.,* U.S. Patent 5,416,016 to Low et al.); mannosides (Umezawa et al., (1988) Biochem. Biophys. Res. Commun. 153:1038); antibodies (P.G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233:134); p120 (Schreier et al. (1994) J. Biol. Chem. 269:9090); see also K. Keinanen; M.L. Laukkanen (1994) FEBS Lett. 346:123; J.J. Killion; I.J. Fidler (1994) Immunomethods 4:273.

### Uses and Methods of the Invention

The antibodies, antibody compositions and methods of the present invention have numerous *in vitro* and *in vivo* diagnostic and therapeutic utilities involving the diagnosis and treatment of disorders involving expression of PSMA. For example, these molecules can be administered to cells in culture, *e.g. in vitro* or *ex vivo,* or to human subjects, *e.g., in vivo,* to treat, prevent and to diagnose a variety of disorders. As used herein, the term "subject" is intended to include human and non-human animals. Non-human animals includes all vertebrates, *e.g*., mammals and non-mammals, such as non-human primates, sheep, dogs, cats, cows, horses, pigs, chickens, avians, amphibians, and reptiles. Preferred subjects include human patients having disorders associated with PSMA expression. When antibodies to PSMA are administered together with another agent, the two can be administered in either order or simultaneously.

Suitable routes of administering the antibody compositions (*e.g.*, antibody or immunoconjugate) of the invention *in vivo* and *in vitro* are well known in the art and can be selected by those of ordinary skill. For example, the antibody compositions can be administered by injection (*e.g*., intravenous or subcutaneous). Suitable dosages of the molecules used will depend on the age and weight of the subject and the concentration and/or formulation of the antibody composition.

In one embodiment, the antibodies of the invention can be initially tested for binding activity associated with therapeutic or diagnostic use *in vitro.* For example, compositions of the invention can be tested using ELISA and flow cytometric assays. Moreover, the activity of these molecules in triggering at least one effector-mediated effector cell activity, including inhibiting the growth of and/or killing of cells expressing PSMA can be assayed. Protocols for assaying for effector cell-mediated ADCC are described in the Examples below.

### A. Detection Methods

In one embodiment, the antibodies of the invention can be used to detect levels of PSMA, or levels of cells which contain PSMA on their membrane surface, which levels can then be linked to certain disease symptoms.

In a particular embodiment, the invention provides methods for detecting the presence of PSMA in a sample, or measuring the amount of PSMA on the surface of cells, comprising contacting the sample, and a control sample, with an antibody, or an antigen binding portion thereof, which specifically binds to PSMA, under conditions that allow for formation of a complex between the antibody or portion thereof and PSMA. The formation of a complex is then detected, wherein a difference complex formation between the sample compared to the control sample is indicative the presence of PSMA in the sample. For example, standard detection methods, well-known in the art, such as ELISA and flow cytometic assays, can be performed using the compositions of the invention.

Accordingly, in one aspect, the invention further provides methods for detecting the presence of PSMA (*e.g.*, human PSMA) in a sample, or measuring the amount of PSMA, comprising contacting the sample, and a control sample, with an antibody of the invention, or an antigen binding portion thereof, which specifically binds to PSMA, under conditions that allow for formation of a complex between the antibody or portion thereof and PSMA. The formation of a complex is then detected, wherein a difference in complex formation between the sample compared to the control sample is indicative of the presence of PSMA in the sample.

The compositions of the invention can also be used to target cells expressing PSMA, for example for labeling such cells. For such use, the binding agent can be linked to a molecule that can be detected. Thus, the invention provides methods for localizing *ex vivo* or *in vitro* cells expressing PSMA. The detectable label can be, *e.g*., a radioisotope, a fluorescent compound, an enzyme, or an enzyme co-factor.

### B. Inhibition of Growth of PSMA+ Cells

The antibodies can be used to inhibit growth of cells expressing PSMA which, in turn, can be linked to the prevention or amelioration of certain disease symptoms associated with PSMA expression. Differences in PSMA expression during a disease state as compared to a non-disease state can be determined by contacting a test sample from a subject suffering from the disease and a control sample with the anti-PSMA antibody under conditions that allow for the formation of a complex between the antibody and PSMA. Any complexes formed between the antibody and PSMA are detected and compared in the sample and the control.

For example, the antibodies can be used to elicit *in vivo* or *in vitro* one or more of the following biological activities: to inhibit the growth of and/or kill a cell expressing PSMA; to mediate phagocytosis or ADCC of a cell expressing PSMA in the presence of human effector cells; to inhibit shedding of soluble PSMA. As discussed herein, the antibodies of the invention exhibit enhanced ADCC activity as compared to the fucosylated form of the antibody.

Accordingly, in another aspect, the invention provides a method of inhibiting growth of PSMA⁺ cells comprising contacting said cells with an anti-PSMA antibody under conditions sufficient to induce antibody-dependent cellular cytoxicity (ADCC) of said cells. The cells can be, for example, tumor cells. In a preferred embodiment, the anti-PSMA antibody is a human antibody.

In one embodiment, the antibodies, or binding portions thereof, of the present invention can be used to modulate PSMA levels on target cells, such as by capping and eliminating receptors on the cell surface. Mixtures of anti-Fc receptor antibodies can also be used for this purpose.

Target-specific effector cells, *e.g*., effector cells linked to compositions of the invention can also be used as therapeutic agents. Effector cells for targeting can be human leukocytes such as macrophages, neutrophils or monocytes. Other cells include eosinophils, natural killer cells and other IgG- or IgA-receptor bearing cells. If desired, effector cells can be obtained from the subject to be treated. The target-specific effector cells, can be administered as a suspension of cells in a physiologically acceptable solution. The number of cells administered can be in the order of 10⁸-10⁹ but will vary depending on the therapeutic purpose. In general, the amount will be sufficient to obtain localization at the target cell, *e.g*., a tumor cell expressing PSMA, and to effect cell killing by, *e.g*., phagocytosis. Routes of administration can also vary.

Therapy with target-specific effector cells can be performed in conjunction with other techniques for removal of targeted cells. For example, anti-tumor therapy using the compositions of the invention and/or effector cells armed with these compositions can be used in conjunction with chemotherapy. Additionally, combination immunotherapy may be used to direct two distinct cytotoxic effector populations toward tumor cell rejection.

### C. Use of Immunoconjugates and Combination Therapy

In one embodiment, immunoconjugates of the invention can be used to target compounds (*e.g*., therapeutic agents, labels, cytotoxins, radiotoxins immunosuppressants, etc.) to cells which have PSMA cell surface molecules by linking such compounds to the antibody. Thus, the invention also provides methods for localizing *ex vivo* or *in vitro* cells expressing PSMA (*e.g.,* with a detectable label, such as a radioisotope, a fluorescent compound, an enzyme, or an enzyme co-factor). Alternatively, the immunoconjugates can be used to kill cells which have PSMA cell surface receptors by targeting cytotoxins or radiotoxins to PSMA, such as to PSMA-expressing tumor cells to thereby eliminate the tumor cell.

In other embodiments, the subject can be additionally treated with an agent that modulates, *e.g*., enhances or inhibits, the expression or activity of Fcγ or Fcγ receptors by, for example, treating the subject with a cytokine. Preferred cytokines for administration during treatment include of granulocyte colony-stimulating factor (G-CSF), granulocyte- macrophage colony-stimulating factor (GM-CSF), interferon-γ (IFN-γ), and tumor necrosis factor (TNF).

In another embodiment, patients treated with antibody compositions of the invention can be additionally administered (prior to, simultaneously with, or following administration of an antibody of the invention) with another therapeutic agent, such as a cytotoxic or radiotoxic agent, which enhances or augments the therapeutic effect of the human antibodies. The antibody can be linked to the agent (as an immunocomplex) or can be administered separate from the agent. In the latter case (separate administration), the antibody can be administered before, after or concurrently with the agent or can be co-administered with other known therapies, *e.g.,* an anti-cancer therapy, *e.g.,* radiation. Such therapeutic agents include, among others, anti-neoplastic agents such as doxorubicin (adriamycin), cisplatin bleomycin sulfate, carmustine, chlorambucil, and cyclophosphamide hydroxyurea which, by themselves, are only effective at levels which are toxic or subtoxic to a patient. Cisplatin is intravenously administered as a 100 mg/ml dose once every four weeks and adriamycin is intravenously administered as a 60-75 mg/ml dose once every 21 days. Co-administration of the anti-PSMA antibodies, or antigen binding fragments thereof, of the present invention with chemotherapeutic agents provides two anti-cancer agents which operate via different mechanisms which yield a cytotoxic effect to human tumor cells. Such co-administration can solve problems due to development of resistance to drugs or a change in the antigenicity of the tumor cells which would render them unreactive with the antibody.

### D. Treatment of Cancer

PSMA has been shown to be expressed on tumor cells, such as prostate carcinoma tumor cells, and also has been shown to be expressed on vascular endothelial cells proximate to cancerous cells, such as urothelial cancerous cells, colon cancerous cells, rectal cancerous cells, lung cancerous cells, breast cancerous cells and metastatic adenocarcinoma cancerous cells of the liver (see US Patent No. 6,136,311). Accordingly, the antibodies of the invention can be used to treat cancer by inhibiting growth of PSMA-expressing tumor cells or by inhibiting growth of vascular endothelial cells proximate to tumor cells. Thus, in another embodiment, the present invention provides a method of inhibiting growth of a tumor in a subject, wherein cells of the tumor or vascular endothelial cells proximate to the tumor are PSMA⁺, in which an anti-PSMA antibody of the invention is administered to the subject such that growth of the tumor is inhibited. For human subjects, the antibody preferably is a humanized or human antibody. In a preferred embodiment, the tumor cells are prostate tumor cells. In other embodiments, the tumor cells are from cancers such as colon, renal, rectal, urothelial, breast, bladder, liver, pancreas or melanoma.

The treatment methods of the invention involve administering to a subject an antibody composition of the present invention in an amount effective to treat or prevent the disorder. The antibody composition can be administered alone or another therapeutic agent, such as a cytotoxic or a radiotoxic agent (conjugated to or administered with the antibody), which acts in conjunction with or synergistically with the antibody composition to treat or prevent the disease associated with PSMA expression.

The treatment methods of the invention also involve administering to a subject an anti-PSMA antibody in combination with another agent, such as an anti-tumor agent, which acts in conjunction with or synergistically with the antibody composition to treat or prevent the disease associated with PSMA expression. As shown herein, use of the anti-PSMA antibody 7F12 in combination with Taxotere® (Docetaxel) resulted in inhibition of tumor growth in an animal model and cured the animals from tumor related cachexia (see Example 8). Without intending to be limited by mechanism, it is believed that the use of an anti-tumor agent causes damage in the tumor mass, thus, allowing improved penetration of antibody, effector cells or an alternative effector component, leading to more effective cytotoxicity. In one embodiment, use of an anti-tumor agent causes damage in the tumor mass, thus; allowing effector cells to access the tumor, leading to a more effective antibody dependent cell-mediated cytotoxicity (ADCC) of the tumor cells. In another embodiment, use of an anti-tumor agent, *e.g*., a microtubule inhibitor, causes tumor cells to be intrinsically more susceptible to anti-PSMA mediated killing. In one embodiment of the invention, the synergistic or additive effect obtained by the administration of an anti-PSMA antibody and an anti-tumor agent is independent of, i.e., not caused by, the reversal of the apical polarity of the PSMA antigen to the basolateral plasma membrane.

As used herein, the term "and-PSMA antibody" includes any antibody that specifically binds to the human prostate specific membrane antigen. Examples of such antibodies include the antibodies described herein, the antibodies described in U.S. Patent Application No. 10/059989 and PCT Publication No. WO 03064606A3, or the antibodies described in U.S. Provisional Application No. 60/660431. The entire contents of each of the foregoing applications are incorporated herein by reference.

As used herein, the term "anti-tumor agent" includes any agent that may be used to destroy or assist in destroying (*e.g*., partially or totally) a tumor. In one embodiment, the anti-tumor agent is capable of causing damage in the tumor mass, thereby allowing effector cells to access the tumor more readily, leading to a more effective antibody dependent cell-mediated cytotoxicity (ADCC) of the tumor cells. The term "anti-tumor agent" includes chemotherapeutic agents, angiogenesis inhibitors, microtubule blocking agents, immunomodulatory agents, DNA intercalators/cross linkers, DNA synthesis inhibitors, DNA-RNA transcription regulators, enzyme inhibitors, and gene regulators.

The term "chemotherapeutic agent" includes any agent that may be used to treat cancer, *e.g*., prostate cancer. Chemotherapeutic agents include alkylating agents, antimetabolites, plant alkaloids, antitumor antibiotics and steroid hormones. Specific examples of chemotherapeutic agents include, but are not limited to, all-trans retinoic acid, Aminoglutethimide, Azacitidine, Azathioprine, Bleomycin (Blenoxane), Busulfan (Myeleran), Carboplatin, Carboplatinum (Paraplatin), Carmustine (BCNU), Capecitabine, CCNU (Lomustine), Chlorambucil (Leukeran), 2-Cholrodeoxyadenosine (2-CDA; Cladribine, Leustatin), Cis-platinum (Platinol), Cisplatin (cis-DDP), Cisplatin bleomycin sulfate, Chlorambucil, Cyclophosphamide (Cytoxanl CTX), Cyclophosphamide hydroxyurea, Cytarabine (Ara-C; cytosine arabinoside), Daunorubicin (Cerubidine), Dacarbazine (DTIC; dimethyltriazenoimidazolecarboxamide), Dactinomycin (actinomycin D), Daunorubicin (daunomycin; rubidomycin), Diethylstilbestrol, Docetaxel (Taxotere), Doxifluridine, Doxorubicin (Adriamycin), Epirubicin, Ethinyl estradoil, Etopaside (VP-16, VePesid), Fluorouracil (5-Fu; Floxuridine, fluorodeoxyuridine; FUdR), Fludarabine (Fludara), Flutamide, Fluoxymesterone, Gemcitabine (Gemzar), Herceptin (Trastuzumab; anti-HER 2 monoclonal antibody), Hydroxyurea (Hydrea), Hydroxyprogesterone caproate, Idarubicin, Ifosfamide (Ifex), Interferon alpha, Irinotecan (CPT-11), L-Asparaginase, Leuprolide, Mechlorethamine, Medroxyprogesterone acetate, Megestrol acetate, Melphelan (Alkeran), Mercaptopurine (6-mercaptopurine; 6-MP), Methotrexate (MTX; amethopterin), Mitomycin (mitomycin C), Mitotane (o,p'-DDD), Mitoxantrone (Novantrone), Oxaliplatin, Paclitaxel (Taxol), Pemetrexed , Pentostatin (2-deoxycoformycin), Plicamycin (mithramycin), Prednisone, Procarbazine (Matulane; N-methylhydrazine, MIH), Rituxin (Rituximap), Semustine (Methyl-CCNU), Streptozocin, Taxol, Tamoxifen, Teniposide, Tertiposide, Testosterone propionate, Thioguanine (6-thioguanine; TG), Thiotepa, Tomudex (Raltitrexed), Topotecan (Hycamtin; (S)-10-[(dimethylamino) methyl]-4-ethyl-4,9-dihydroxy-1H-pyrano[3',4'), Treosulfan (Ovastat), Valrubicin, Vinblastine (VLB; Velban), Vincristine (Oncovin), Vindesine, and Vinorelbine (Navelbine).

The term "microtubule blocking agent" includes any agent that is capable of disrupting the normal organization and dynamics of microtubules. Examples of microtubule blocking agents include, but are not limited to, taxanes (Taxol® (Paclitaxel), Taxotere® (docetaxel)), vinca alkaloids (vinblastine, vincristine (Oncovin), Vindesine (Eldisine, Fildesin), Vinorelbine (Navelbine)), 2-methoxyestradiol (2ME2), estramustine, epothilones, Colchicine, Dolastatin 15, Nocodazole, Podophyllotoxin, Rhizoxin.

The terms "angiogenesis inhibitor" and "anti-angiogenic agent" are used interchangeably herein and include any agent that is capable of preventing or inhibiting the formation of blood vessels. Specific examples of angiogenesis inhibitors include, but are not limited to, Angiostatin K1-3, Arresten, aaAT, Canstatin, DL-α-Difluoromethyl-ornithine, Endostatin, Fumagillin, Genistein, Minocycline, Staurosporine, (±)-Thalidomide, and Tumstatin.

The term "immunomodulating agent" includes any agent that modulates; *e.g*., stimulates, an immune response. Examples of immunomodulating agents include antibodies, such as anti-PD1 antibodies and anti-CTLA-4 antibodies, alone or in combination, described, *inter alia,* in U.S. Provisional Application No. 60/679,466, filed 05/09/2005; in PCT Publication WO 01/14424; in U.S. Provisional Application 60/738,434, filed 11/21/2005; and in U.S. Provisional Application , filed 12/08/2005 (Attorney Docket No. MEDX-0124US2 or 04280/1203401-US1), the entire contents of each of which are expressly incorporated herein by reference.

Additional immunomodulating agents that may be used in the methods of the present invention include antibodies that block a costimulatory signal, (*e.g*., CD28 or ICOS), antibodies that activate an inhibitory signal via CTLA4, and/or antibodies against other immune cell markers (*e.g.,* CD40, CD40 ligand, or cytokines), fusion proteins (*e.g.*, CTLA4-Fc, PD-1-Fc), and immunosuppressive drugs, (*e.g*., rapamycin, cyclosporine A or FK506). Other examples of immunomodulating agents include phosphorothiolate oligodeoxyribonucleotide (1018 ISS), GVAX (a GM-CSF gene vaccine), interleukins (*e.g*., interleukin-1, -2, -3, -4, -5, -6, -7 (CYT 99 07), -8, -9, -10, -11, -13, -14, -15, -16, -17, -18, -19, -20, -21, -22, -23, -24, -25, -26, -27, -28, -29 and -30), *e.g.*, recombinant interleukin antibodies, *e.g.,* IL-2, *e.g.,* Aldesleukin, *e.g.,* recombinant interleukins (*e.g.,* recombinant interleukin-21 (rIL-21)), *e.g.,* IL-11, *e.g.,* oprelvekin, *e.g.,* interleukin receptor antagonists, *e.g.,* IL-1 receptor antagonist, *e.g*., anakinra, glucocerebrosidase, *e.g.,* Imiglucerase, macrophage activating factor, macrophage peptide, B cell factor, and T cell factor.

Examples of DNA intercalators/cross linkers include, but are not limited to, Bleomycin, Carboplatin, Carmustine, Chlorambucil, Cyclophosphamide, cis-Diammineplatinum(II) dichloride (Cisplatin), Melphalan, Mitoxantrone, and Oxaliplatin.

Examples of DNA synthesis inhibitors include, but are not limited to, (±)-Amethopterin (Methotrexate), 3-Amino-1,2,4-benzotriazine 1,4-dioxide, Aminopterin, Cytosine β-D-arabinofuranoside, 5-Fluoro-5'-deoxyuridine, 5-Fluorouracil, Ganciclovir, Hydroxyurea, and Mitomycin C.

Examples of DNA-RNA transcription regulators include, but are not limited to, Actinomycin D, Daunorubicin, Doxorubicin, Homoharringtonine, and Idarubicin.

Examples of enzyme inhibitors include, but are not limited to, S(+)-Camptothecin, Curcumin, (-)-Deguelin, 5,6-Dichlorobenz-imidazole 1-β-D-ribofuranoside, Etoposide, Formestane, Fostriecin, Hispidin, 2-Imino-1-imidazolidineacetic acid (Cyclocreatine), Mevinolin, Trichostatin A, Tyrphostin AG 34, and Tyrphostin AG 879.

Examples of gene regulators include, but are not limited to, 5-Aza-2'-deoxycytidine, 5-Azacytidine, Cholecalciferol (Vitamin D3), 4-Hydroxytamoxifen, Melatonin, Mifepristone, Raloxifene, all trans-Retinal (Vitamin A aldehyde), Retinoic acid, all trans (Vitamin A acid), 9-cis-Retinoic Acid, 13-cis-Retinoic acid, Retinol (Vitamin A), Tamoxifen, and Troglitazone.

The methods of the invention also involve administering to a subject an immunoconjugate (comprising an anti-PSMA antibody, or antigen-binding portion thereof, linked to a therapeutic agent, such as a cytotoxin or a radioactive isotope) in combination with an anti-tumor agent, which acts in conjunction with or synergistically with the antibody composition to treat or prevent the disease associated with PSMA expression.

The methods of the invention further involve administering to a subject a bispecific molecule (comprising an anti-PSMA antibody, or antigen-binding portion thereof, linked to a second functional moiety having a different binding specificity than the antibody) in combination with an anti-tumor agent, which acts in conjunction with or synergistically with the antibody composition to treat or prevent the disease associated with PSMA expression. The anti-tumor agent may be administered in any therapeutically effective dosage known in the art or as described herein (see pharmaceutical compositions).

The anti-PSMA antibody may be administered in combination with a single anti-tumor agent. The anti-PSMA antibody may also be administered in combination with two or more anti-tumor agents.

The anti-PSMA antibody and the anti-tumor agent may be administered simultaneously. For example, the anti-PSMA antibody and the anti-tumor agent may be administered together in a single pharmacutical formulation. In another embodiment, the anti-PSMA antibody and the anti-tumor agent may be administered at the same time as two or more separate pharmaceutical formulations.

The anti-PSMA antibody and the anti-tumor agent may also be administered at different times. For example, the anti-tumor agent(s) may be administered prior to administration of the anti-PSMA antibody (*e.g*., about 1, 2, 3, 4, 5, 6, 7, 8, 9,10,15, 20,24 or 48 hours prior to administration of the anti-PSMA antibody). Alternatively, the anti-PSMA antibody may be administered prior to administration of the anti-tumor agent(s) (*e.g*., about 1, 2, 3, 4, 5, 6, 7, 8, 9, 10, 15, 20, 24 or 48 hours prior to administration of the anti-tumor agent(s)). In one embodiment, the anti-PSMA antibody may be administered in combination with an anti-tumor agent according to the the dosing schedule delineated herein in Table 1.

### Kits

Also within the scope of the invention are kits comprising an antibody of the invention and instructions for use. The kit can further contain one or more additional reagents, such as an immunostimulatory reagent, a cytotoxic agent or a radiotoxic agent, or one or more additional antibodies of the invention (*e.g*., an antibody having a complementary activity which binds to an epitope in the PSMA antigen distinct from the first antibody). Kits typically include a label indicating the intended use of the contents of the kit The term label includes any writing, or recorded material supplied on or with the kit, or which otherwise accompanies the kit.

The present invention is further illustrated by the following examples which should not be construed as further limiting.

### Example 1: Generation of Human Monoclonal Antibodies Against PSMA

### Antigen

Immunization protocols utilized as antigen cells of the PSMA expressing prostate cancer cell line LNCaP (ATCC CRL-1740).

### Transgenic HuMab mice

Fully human monoclonal antibodies to PSMA were prepared using the HCo12 strain of HuMab transgenic mice, which expresses human antibody genes. In this mouse strains, the endogenous mouse kappa light chain gene has been homozygously disrupted as described in Chen et al. (1993) EMBO J. 12:811-820 and the endogenous mouse heavy chain gene has been homozygously disrupted as described in Example 1 of PCT Publication WO 01/09187. Futhermore, this mouse strain carries a human kappa light chain transgene, KCo5, as described in Fishwild et al. (1996) Nature Biotechnology 14:845-851, and a human heavy chain transgene, HCo12, as described in Example 2 of PCT Publication WO 01/09187.

### HuMab Immunizations:

To generate fully human monoclonal antibodies to PSMA, HuMab mice were immunized with LNCaP cells expressing PSMA as antigen. General immunization schemes for HuMab mice are described in Lonberg, N. et al (1994) Nature 368(6474): 856-859; Fishwild, D. et al. (1996) Nature Biotechnology 14: 845-851 and PCT Publication WO 98/24884. The mice were 6-16 weeks of age upon the first infusion of antigen. 5-10x10⁶ cells were used to immunize the HuMab mice intraperitonealy (IP), subcutaneously (Sc) or via footpad injection.

Transgenic mice were immunized twice with antigen in complete Freund's adjuvant or Ribi adjuvant IP, followed by 3-21 days IP (up to a total of 11 immunizations) with the antigen in incomplete Freund's or Ribi adjuvant. The immune response was monitored by retroorbital bleeds. The plasma was screened by ELISA (as described below), and mice with sufficient titers of anti-PSMA human immunogolobulin were used for fusions. Mice were boosted intravenously with antigen 3 days before sacrifice and removal of the spleen. Typically, 10-35 fusions for each antigen were performed. Several dozen mice were immunized for each antigen.

### Selection of HuMab Mice Producing Anti-PSMA Antibodies:

To select HuMab mice producing antibodies that bound PSMA, sera from immunized mice were screened for binding to the PSMA expressing prostate cancer cell line LNCaP, but not to a negative prostate cancer cell line by flow cytometry. Briefly, the binding of anti-PSMA antibodies was assessed by incubating LNCaP cells with the anti-PSMA antibody at a concentration of 20 µg/ml. The cells were washed and binding was detected with a FITC-labeled anti-human IgG Ab. Flow cytometric analyses were performed using a FACScan flow cytometry (Becton Dickinsqn, San Jose, CA). Antibodies that bound to the PSMA expressing LNCaP cells but not the non-PSMA expressing prostate cancer cells were further tested for binding to PSMA by ELISA, as described by Fishwild, D*. et al.* (1996). Briefly, microtiter plates were coated with purified PSMA at 1-2 µg /ml in PBS, 100 µl/wells incubated 4 °C overnight then blocked with 200 µl/well of 5% fetal bovine serum in PBS/Tween (0.05%). Dilutions of sera from PSMA-immunized mice were added to each well and incubated for 1-2 hours at ambient temperature. The plates were washed with PBS/Tween and then incubated with a goat-anti-human IgG polyclonal antibody conjugated with horseradish peroxidase (HRP) for 1 hour at room temperature. After washing, the plates were developed with ABTS substrate (Sigma, A-1888, 0.22 mg/ml) and analyzed by spectrophotometer at OD 415-495. Mice that developed the highest titers of anti-PSMA antibodies were used for fusions. Fusions were performed as described below and hybridoma supernatants were tested for anti-PSMA activity by ELISA.

### Generation of Hybridomas Producing Human Monoclonal Antibodies to PSMA:

The mouse splenocytes, isolated from the HuMab mice, were fused with PEG to a mouse myeloma cell line based upon standard protocols. The resulting hybridomas were then screened for the production of antigen-specific antibodies. Single cell suspensions of splenocytes from immunized mice were fused to one-fourth the number of SP2/0 nonsecreting mouse myeloma cells (ATCC, CRL 1581) with 50% PEG (Sigma). Cells were plated at approximately 1x10⁵/well in flat bottom microtiter plate, followed by about two week incubation in selective medium containing 10% fetal bovine serum, 10% P388D1 (ATCC, CRL TIB-63) conditioned medium, 3-5% origen (IGEN) in DMEM (Mediatech, CRL 10013, with high glucose, L-glutamine and sodium pyruvate) plus 5 mM HEPES, 0.055 mM 2-mercaptoethanol, 50 mg/ml gentamycin and 1x HAT (Sigma, CRL P-7185). After 1-2 weeks, cells were cultured in medium in which the HAT was replaced with HT. Individual wells were then screened by ELISA (described above) for human anti-PSMA monoclonal IgG antibodies. Once extensive hybridoma growth occurred, medium was monitored usually after 10-14 days. The antibody-secreting hybridomas were replated, screened again and, if still positive for human IgG, anti-PSMA monoclonal antibodies were subcloned at least twice by limiting dilution. The stable subclones were then cultured *in vitro* to generate small amounts of antibody in tissue culture medium for further characterization.

Hybridoma clones 1C3, 2A10, 2F5, 2C6, were selected for further analysis.

### Example 2: Structural Characterization of Human Monoclonal Antibodies 1C3, 2A10, 2F5, and 2C6

The cDNA sequences encoding the heavy and light chain variable regions of the 1C3, 2A10, 2F5, and 2C6 monoclonal antibodies were obtained from the 1 C3, 2A10, 2F5, and 2C6 hybridomas, respectively, using standard PCR techniques and were sequenced using standard DNA sequencing techniques.

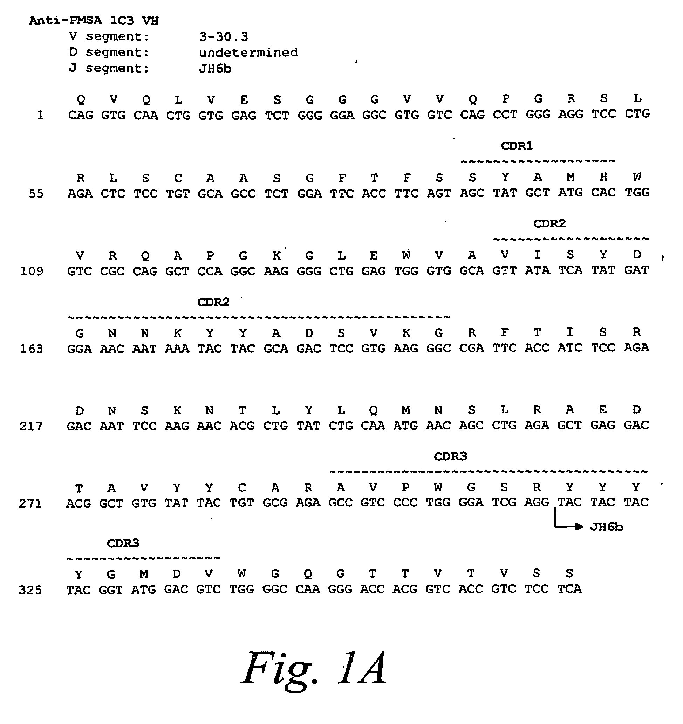

The nucleotide and amino acid sequences of the heavy chain variable region of 1C3 are shown in Figure 1A and in SEQ ID NO: 33 and 1, respectively.

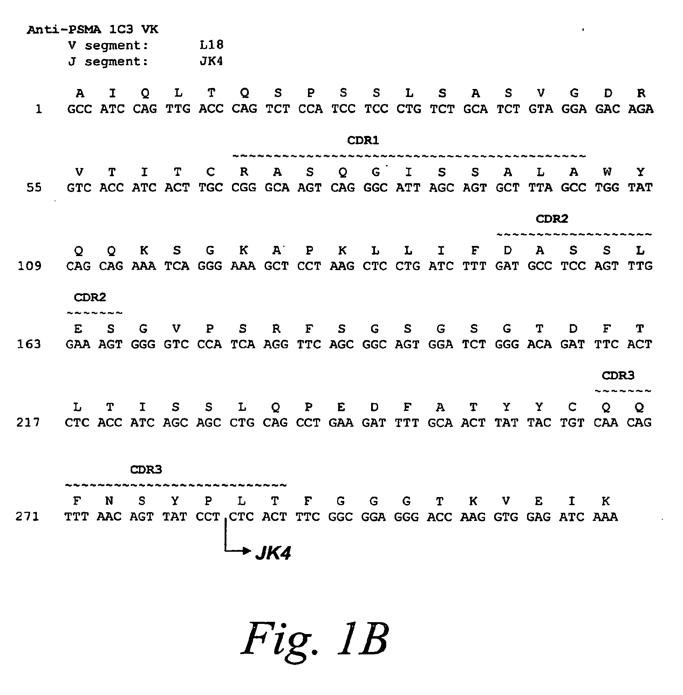

The nucleotide and amino acid sequences of the light chain variable region of 1C3 are shown in Figure 1B and in SEQ ID NO: 37 and 5, respectively.

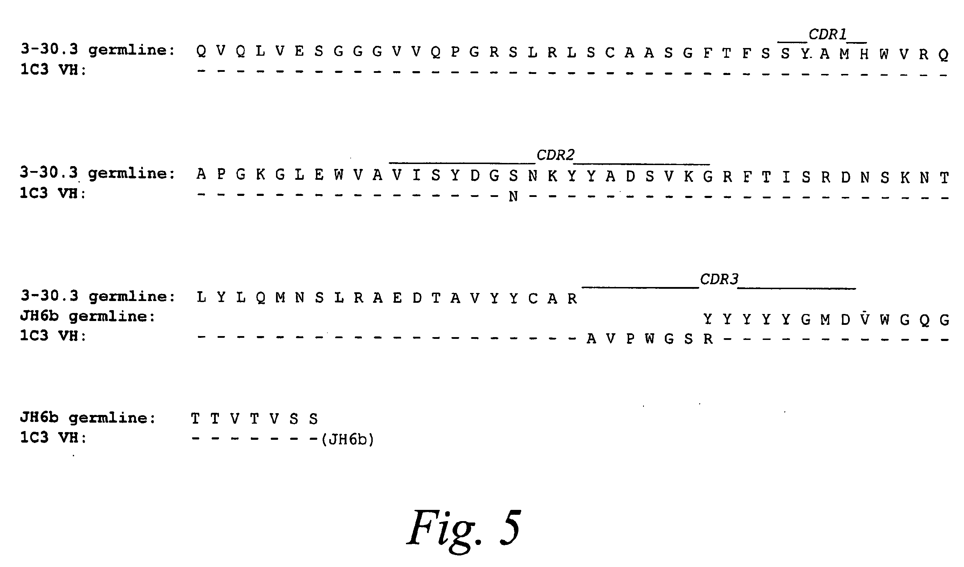

Comparison of the 1C3 heavy chain immunoglobulin sequence to the known human germline immunoglobulin heavy chain sequences demonstrated that the 1C3 heavy chain utilizes a VH segment from human germline VH 3-30.3, an undetermined D segment, and a JH segment from human germline JH 6b. The alignment of the 1 C3 VH sequence to the germline VH 3-30.3 sequence is shown in Figure 5. Further analysis of the 1C3 VH sequence using the Kabat system of CDR region determination led to the delineation of the heavy chain CDR1, CDR2 and CD3 regions as shown in Figures 1A and 5, and in SEQ ID NOs:9, 13 and 17, respectively.

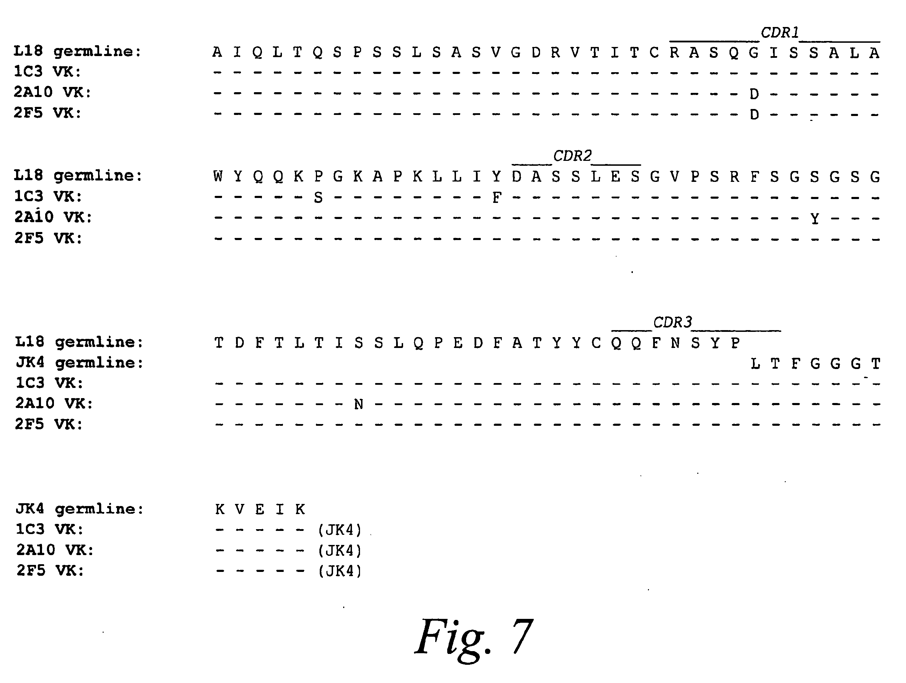

Comparison of the 1C3 light chain immunoglobulin sequence to the known human germline immunoglobulin light chain sequences demonstrated that the 1C3 light chain utilizes a VL segment from human germline VK L18 and a JK segment from human germline JK 4. The alignment of the 1C3 VL sequence to the germline VK L18 sequence is shown in Figure 7. Further analysis of the 1C3 VL sequence using the Kabat system of CDR region determination led to the delineation of the light chain CDR1, CDR2 and CD3 regions as shown in Figures 1B and 7, and in SEQ ID NOs:21, 25 and 29, respectively.

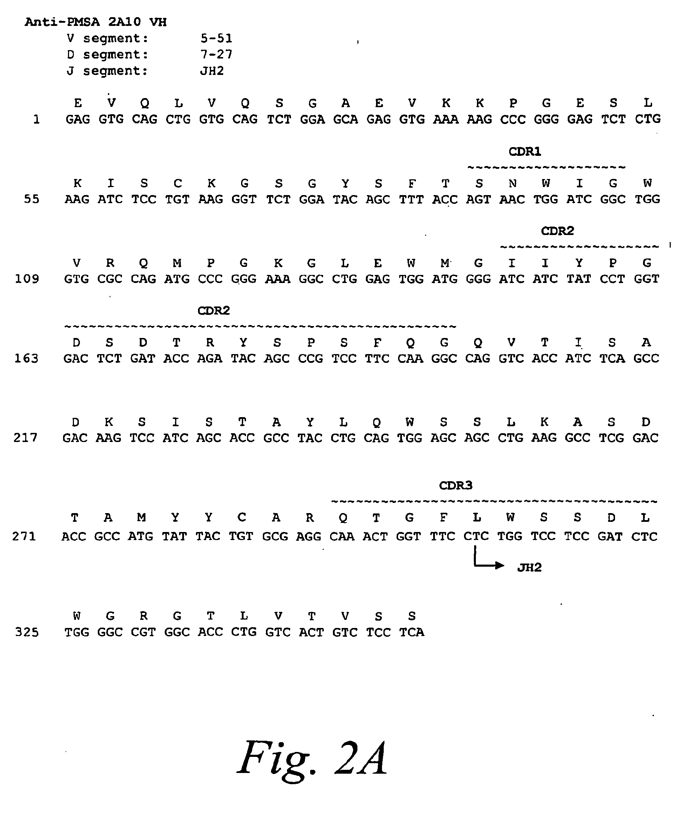

The nucleotide and amino acid sequences of the heavy chain variable region of 2A10 are shown in Figure 2A and in SEQ ID NO: 34 and 2, respectively.

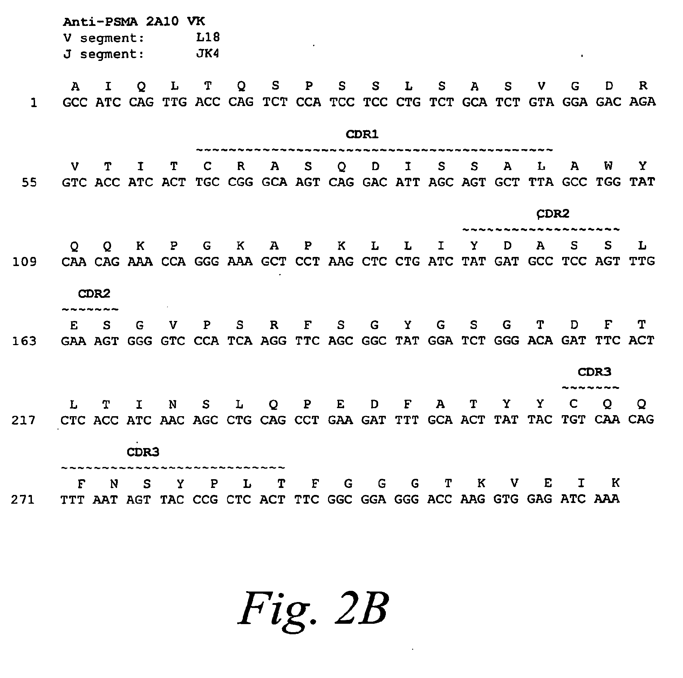

The nucleotide and amino acid sequences of the light chain variable region of 2A10 are shown in Figure 2B and in SEQ ID NO: 38 and 6, respectively.

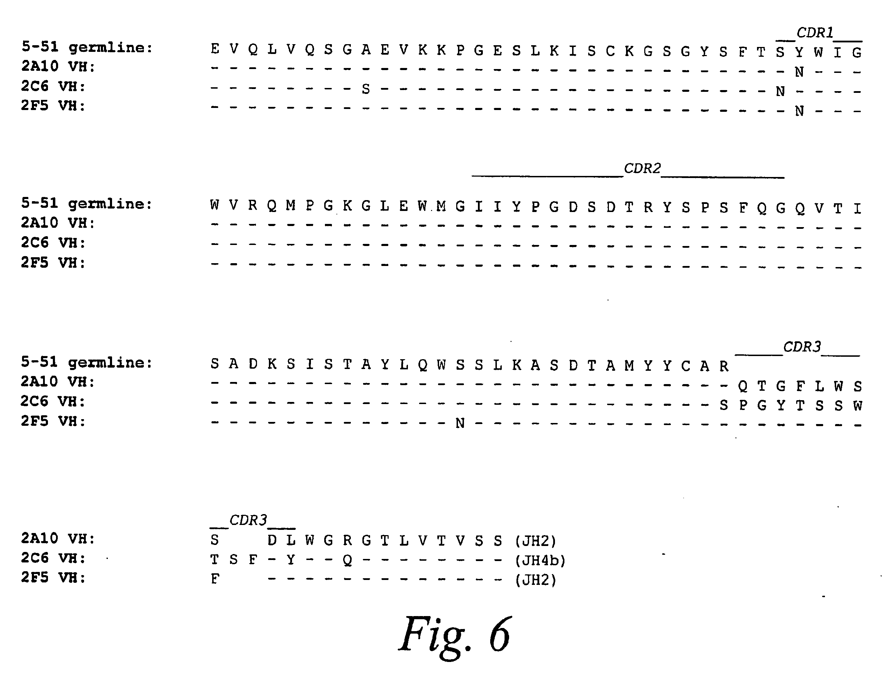

Comparison of the 2A10 heavy chain immunoglobulin sequence to the known human germline immunoglobulin heavy chain sequences demonstrated that the 2A10 heavy chain utilizes a VH segment from human germline VH 5-51, a D segment from human germline 7-27, and a JH segment from human germline JH 2. The alignment of the 2A10 VH sequence to the germline VH 5-51 sequence is shown in Figure 6. Further analysis of the 2A10 VH sequence using the Kabat system of CDR region determination led to the delineation of the heavy chain CDR1, CDR2 and CD3 regions as shown in Figures 2A and 6, and in SEQ ID NOs: 10, 14 and 18, respectively.

Comparison of the 2A10 light chain immunoglobulin sequence to the known human germline immunoglobulin light chain sequences demonstrated that the 2A10 light chain utilizes a VL segment from human germline VK L18 and a JK segment from human germline JK 4. The alignment of the 2A10 VL sequence to the germline VK L18 sequence is shown in Figure 7. Further analysis of the 2A10 VL sequence using the Kabat system of CDR region determination led to the delineation of the light chain CDR1, CDR2 and CD3 regions as shown in Figures 2B and 7, and in SEQ ID NOs: 22, 26 and 30, respectively.

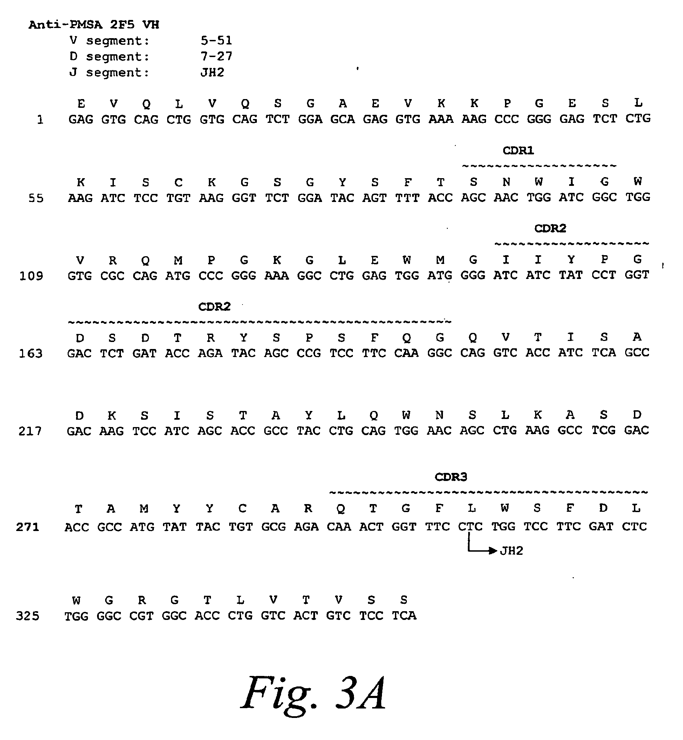

The nucleotide and amino acid sequences of the heavy chain variable region of 2F5 are shown in Figure 3A and in SEQ ID NO: 35 and 3, respectively.

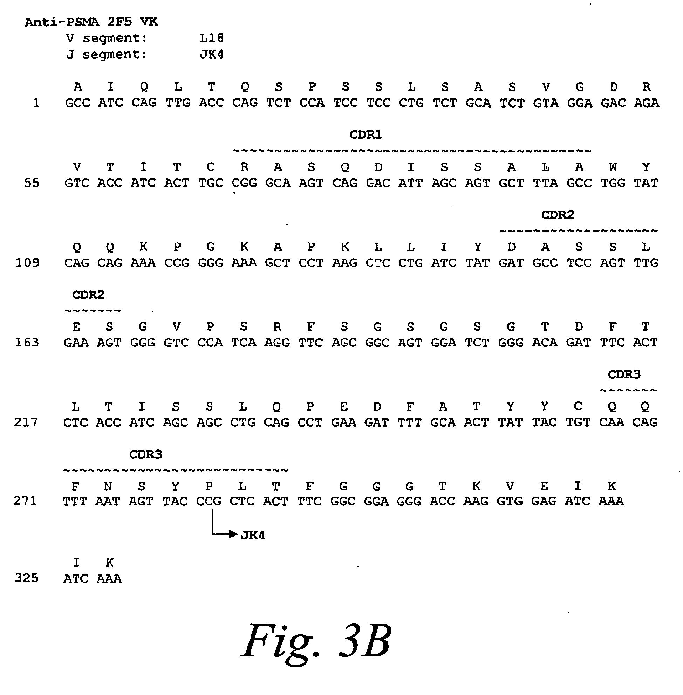

The nucleotide and amino acid sequences of the light chain variable region of 2F5 are shown in Figure 3B and in SEQ ID NO: 39 and 7, respectively.

Comparison of the 2F5 heavy chain immunoglobulin sequence to the known human germline immunoglobulin heavy chain sequences demonstrated that the 2F5 heavy chain utilizes a VH segment from human germline VH 5-51, a D segment from human germline 7-27, and a JH segment from human germline JH 2. The alignment of the 2F5 VH sequence to the germline VH 5-51 sequence is shown in Figure 6. Further analysis of the 2F5 VH sequence using the Kabat system of CDR region determination led to the delineation of the heavy chain CDR1, CDR2 and CD3 regions as shown in Figures 3A and 6, and in SEQ ID NOs:11, 15 and 19, respectively.

Comparison of the 2F5 light chain immunoglobulin sequence to the known human germline immunoglobulin light chain sequences demonstrated that the 2F5 light chain utilizes a VL segment from human germline VK L18 and a JK segment from human germline JK 4. The alignment of the 2F5 VL sequence to the germline VK L18 sequence is shown in Figure 7. Further analysis of the 2F5 VL sequence using the Kabat system of CDR region determination led to the delineation of the light chain CDR1, CDR2 and CD3 regions as shown in Figures 3B and 7, and in SEQ ID NOs:23, 27 and 31, respectively.

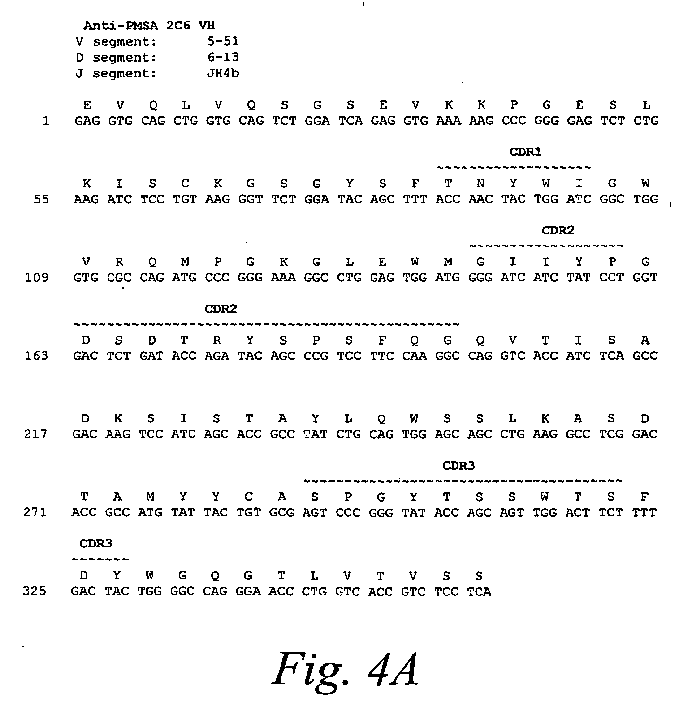

The nucleotide and amino acid sequences of the heavy chain variable region of 2C6 are shown in Figure 4A and in SEQ ID NO: 36 and 4, respectively.

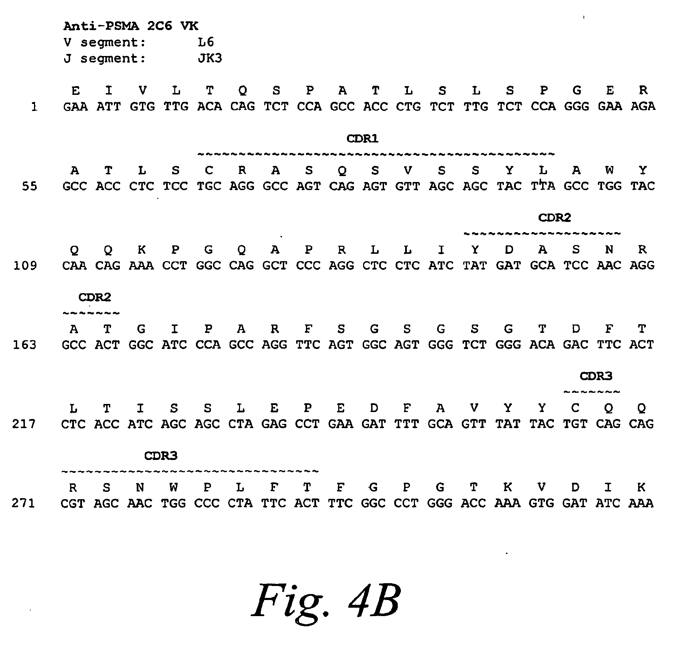

The nucleotide and amino acid sequences of the light chain variable region of 2C6 are shown in Figure 4B and in SEQ ID NO: 40 and 8, respectively.

Comparison of the 2C6 heavy chain immunoglobulin sequence to the known human germline immunoglobulin heavy chain sequences demonstrated that the 2C6 heavy chain utilizes a VH segment from human germline VH 5-51, a D segment from human germline 6-13, and a JH segment from human germline JH 4b. The alignment of the 2C6 VH sequence to the germline VH 5-51 sequence is shown in Figure 6. Further analysis of the 2C6 VH sequence using the Kabat system of CDR region determination led to the delineation of the heavy chain CDR1, CDR2 and CD3 regions as shown in Figures 4A and 6, and in SEQ ID NOs: 12, 16 and 20, respectively.

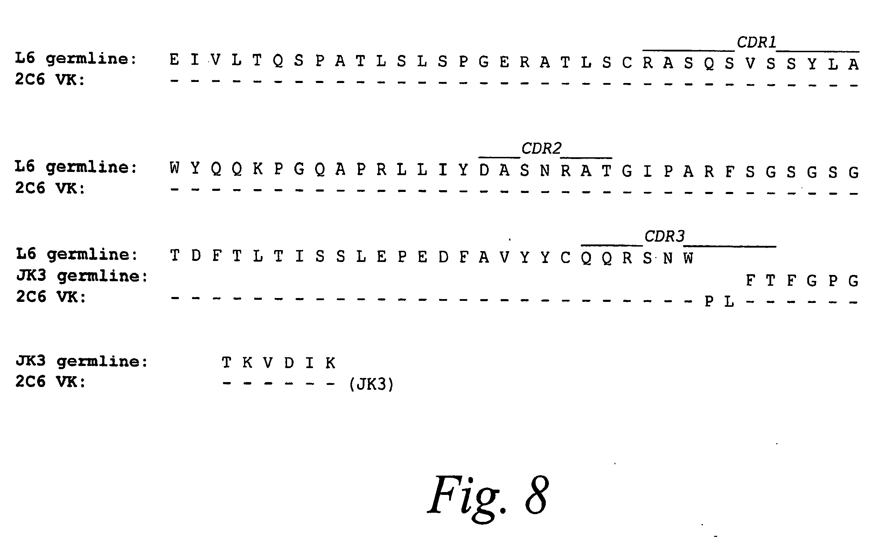

Comparison of the 2C6 light chain immunoglobulin sequence to the known human germline immunoglobulin light chain sequences demonstrated that the 2C6 light chain utilizes a VL segment from human germline VK L6 and a JK segment from human germline JK 3. The alignment of the 2C6 VL sequence to the germline VK L6 sequence is shown in Figure 8. Further analysis of the 2C6 VL sequence using the Kabat system of CDR region determination led to the delineation of the light chain CDR1, CDR2 and CD3 regions as shown in Figures 4B and 8, and in SEQ ID NOs: 24, 28 and 32, respectively.

### Example 3: Characterization of Binding Specificity of Anti-PSMA Human Monoclonal Antibodies

In this example, binding specificity was examined by flow cytometry on a prostate cancer cell line expressing PSMA and by ELISA using purified PSMA.

### Binding specificity by flow cytometry

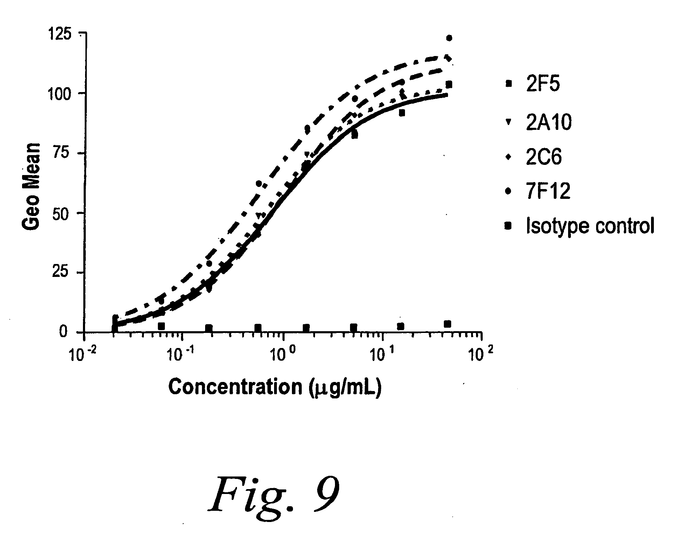

The human PSMA-expressing prostate cancer cell line LNCaP was used to determine the specificity of anti-PSMA human monoclonal antibodies by flow cytometry. Binding of the 2F5, 2A10, and 2C6 anti-PSMA human monoclonal antibodies was assessed by incubating the LNCaP cells with the anti-PSMA human monoclonal antibodies at different concentrations. The cells were washed and binding was detected with a FITC-labeled anti-human IgG Ab. Flow cytometric analyses were performed using a FACScan flow cytometry (Becton Dickinson, San Jose, CA). The human anti-PSMA monoclonal antibody 7F12 (as described in PCT Publication WO 03/064606) was used as a positive control and a non-PSMA specific isotype control antibody was used as a negative control. The results are depicted in Figure 9. The anti-PSMA human monoclonal antibodies 2F5, 2A10, and 2C6 bound specifically to the PSMA-expressing LNCaP cells.

### Binding specificity by ELISA

A comparison of anti-PSMA antibodies on binding to immunopurified PSMA was performed by standard ELISA to examine the specificity of binding for PSMA.

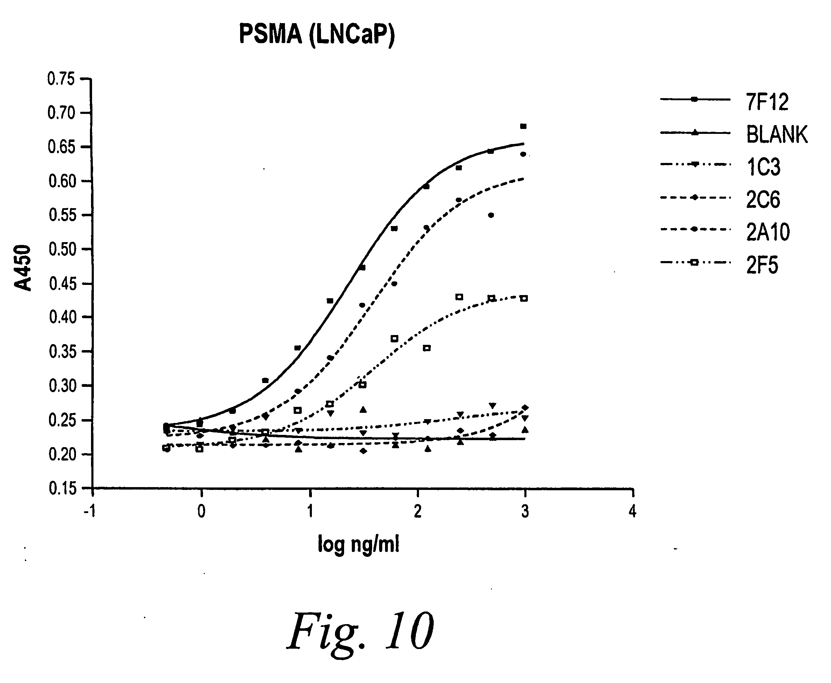

PSMA was immunopurified from LNCaP cells and tested for binding against the anti-PSMA human monoclonal antibodies 1C3, 2A10, 2F5, and 2C6. Standard ELISA procedures were performed. The anti-PSMA human monoclonal antibodies were added at a concentration of 5 µg/ml and titrated down at 1:2 serial dilutions. Goat-anti-human IgG (kappa chain-specific) polyclonal antibody conjugated with horseradish peroxidase (HRP) was used as secondary antibody. The human anti-PSMA monoclonal antibody 7F12 was used as a positive control and a blank was used as a negative control. The results are shown in Figure 10. The anti-PSMA human monoclonal antibodies 2A10 and 2F5 bound with high specificity to PSMA. The anti-PSMA human monoclonal antibodies 1C3 and 2C6 exhibited detectable but only low level binding to PSMA in an ELISA assay, suggesting that these antibodies bind to a hindered epitope in an ELISA assay.

### Example 4: Scatchard analysis of binding affinity of anti-PSMA monoclonal antibodies

The binding affinity of the 2A10 antibody for the PSMA-expressing prostate cancer LNCaP cell line was tested using a Scatchard analysis.

LNCaP cells were obtained from ATCC (CRL-1740) and grown in RPMI media containing 10% fetal bovine serum (FBS). The cells were trypsinized and washed once in Tris based binding buffer (24mM Tris pH 7.2, 137mM NaCl, 2.7mM KCI, 2mM Glucose, 1mM CaCl₂, 1mM MgCl₂, 0.1% BSA) and the cells were adjusted to 2x10⁶ cells/ml in binding buffer. Millipore plates (MAFB NOB) were coated with 1% nonfat dry milk in water and stored a 4 °C overnight. The plates were washed three times with 0.2ml of binding buffer. Fifty microliters of buffer alone was added to the maximum binding wells (total binding). Twenty-five microliters of buffer alone was added to the control wells (non-specific binding). Varying concentration of ¹²⁵I-anti-PSMA antibody was added to all wells in a volume of 25µl. Varying concentrations of unlabeled antibody at 100 fold excess was added in a volume of 25µl to control wells and 25µl of LNCaP cells (2 X 10⁶ cells/ml) in binding buffer were added to all wells. The plates were incubated for 2 hours at 200 RPM on a shaker at 4°C. At the completion of the incubation the Millipore plates were washed three times with 0.2 ml of cold wash buffer (24mM Tris pH 7.2, 500mM NaCl, 2.7mM KCI, 2mM Glucose, 1mM CaCl₂, 1mM MgCl₂, 0.1% BSA.). The filters were removed and counted in a gamma counter. Evaluation of equilibrium binding was performed using single site binding parameters with the Prism software (San Diego, CA).

Using the above scatchard binding assay, the K_{D} of the 2A10 antibody for LNCaP cells was approximately 0.8 nM.

### Example 5: Epitope competition binding assay

The anti-PSMA monoclonal antibody 2A10 was compared to a known anti-PSMA antibody, 7F12 (described in PCT Publication WO 03/064606) to examine whether the two antibodies bound to the same epitope region using a competition assay.

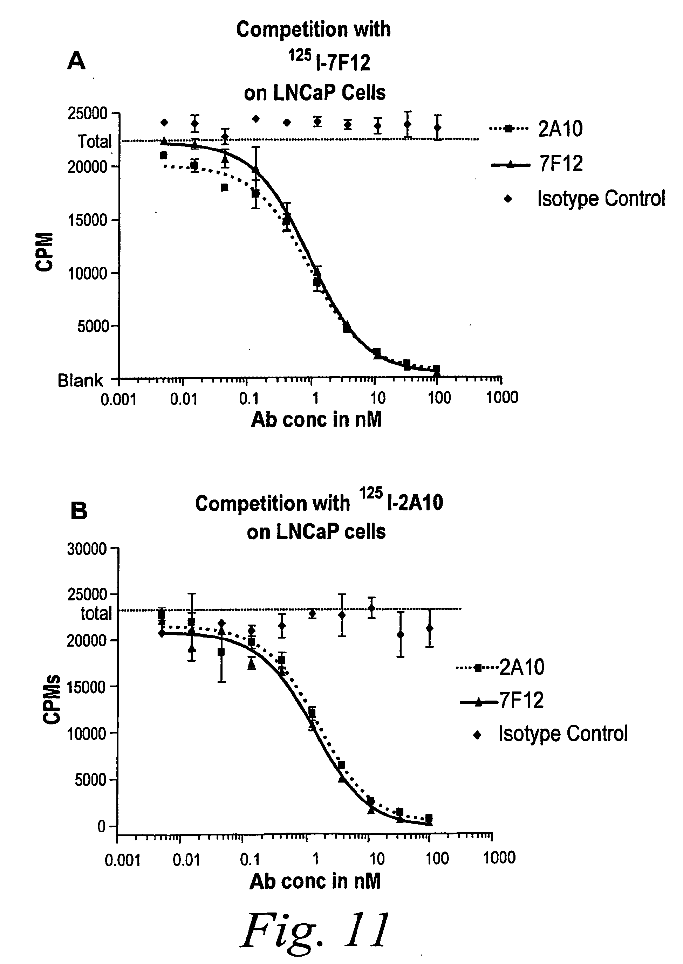

LNCaP cells were obtained from ATCC (CRL-1740) and grown in RPMI media containing 10% fetal bovine serum (FBS). The cells were trypsinized and washed once in Tris based binding buffer (24mM Tris pH 7.2, 137mM NaCl, 2.7mM KCI, 2mM Glucose, 1mM CaCl₂, 1mM MgCl₂, 0.1% BSA) and the cells were adjusted to 2x10⁶ cells/ml in binding buffer. Millipore plates (MAFB NOB) were coated with 1% nonfat dry milk in water and stored a 4 °C overnight. The plates were washed three times with 0.2ml of binding buffer. Twenty-five microliters of buffer alone was added to the wells (non-specific binding). A fixed concentration of ¹²⁵I-anti-PSMA antibody was added to all wells in a volume of 25µl. Varying concentrations of unlabeled antibody was added to wells in a volume of 25µl and 25µl of LNCaP cells (2 X 10⁶ cells/ml) in binding buffer were added to all wells. The plates were incubated for 2 hours at 200 RPM on a shaker at 4°C. At the completion of the incubation the Millipore plates were washed three times with 0.2 ml of cold wash buffer (24mM Tris pH 7.2, 500mM NaCl, 2.7mM KCI, 2mM Glucose, 1mM CaCl₂, 1mM MgCl₂, 0.1% BSA.). The filters were removed and counted in a gamma counter. Evaluation of equilibrium binding was performed using single site binding parameters with the Prism software (San Diego, CA). An isotype control antibody was used as a negative control. The results of competition binding against ¹²⁵I-2A10 are shown in Figure 11A and competition binding against ¹²⁵I-7F12 are shown in Figure 11B. The results show that addition of unlabeled 7F12 antibody inhibits the binding of labeled 2A10 to LNCaP cells and the addition of unlabeled 2A10 antibody inhibits the binding of labeled 7F12 to LNCaP cells. This demonstrates that the 2A10 and 7F12 anti-PSMA antibodies bind to the same or a very similar epitope on PSMA.

### Example 6: Internalization of anti-PSMA monoclonal antibody

Anti-PSMA HuMAbs were tested for the ability to internalize into PSMA-expressing prostate cancer cells using a Hum-Zap internalization assay. Hum-Zap tests for internalization of a primary human antibody through binding of a secondary antibody with affinity for human IgG conjugated to the toxin saporin.

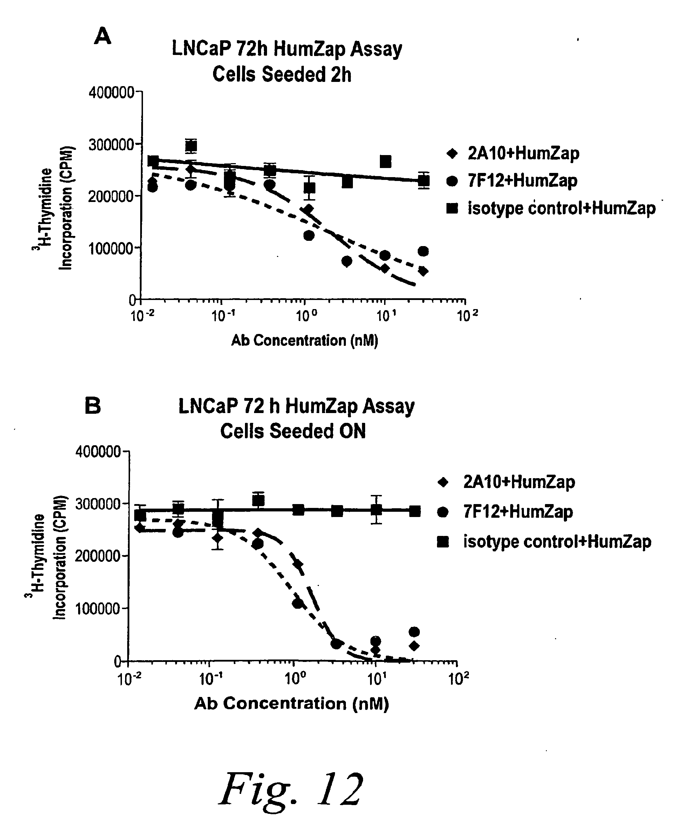

The PSMA-expressing prostate cancer cell line LNCaP was seeded at 2.5x10⁴ cells/well in 100 µl wells either overnight or the following day for a two hour period. Either the anti-PSMA antibody 2A10 or 7F12 were added to the wells at a starting concentration of 30 nM and titrated down at 1:3 serial dilutions. An isotype control antibody that is non-specific for PSMA was used as a negative control. The Hum-Zap (Advanced Targeting Systems, IT-22-25) was added at a concentration of 11 nM and plates were allowed to incubate for 48 hours. The plates were then pulsed with 1.0 µCi of ³H-thymidine for 24 hours, harvested and read in a Top Count Scintillation Counter (Packard Instruments). The results are shown in Figure 12. The anti-PSMA antibody 2A10 showed an antibody concentration dependent decrease in ³H-thymidine incorporation in PSMA-expressing LNCap prostate cancer cells. This data demonstrates that the anti-PSMA antibody 2A10 internalizes into a prostate cancer cell line.

### Example 7: Thermostability of anti-PSMA monoclonal antibodies by differential scanning calorimetry

The thermal stability of the anti-PSMA monoclonal antibody 2A10 was compared to the 7F12 antibody using calorimetric analysis of the melting temperature of the antibody.

Calorimetric measurements of melting temperatures ™ were carried out on a VP-Capillary DSC differential scanning microcalorimeter platform that is combined with an autosampler (MicroCal LLC, Northampton, MA, USA). Sample cell volume is 0.144 mL. Denaturation data on the glycosylated and deglycosylated forms of the antibodies was obtained by heating the samples, at a concentration of 2.3µM, from 30 to 95 °C at a rate of 1 °C/min. The protein samples were present in phosphate-buffered saline (PBS) at pH 7.4. The same buffer was used in the reference cell to obtain the molar heat capacity by comparison. The observed thermograms were baseline corrected and normalized data analyzed based on a 2-step model, using the software Origin v7.0. The clone 2A10 has higher thermostability, with a Tₘ of 71.43 °C, compared to 63.05 °C obtained for the clone 7F12.

### Example 8: Treatment of in vivo tumor xenografts with a combination of Taxotere® and the 7F12 antibody

The anti-tumor efficacy of the anti-PSMA 7F12 antibody in combination with Taxotere® (docetaxel) was tested on LNCaP human prostate carcinoma xenografts grown in male CB17.SCID mice.

LNCaP prostate cancer cells expressing high levels of PSMA were obtained from ATCC (Cat# CRL-1740) and expanded *in vitro* following ATCC instruction. 8 week-old male CB17.SCID mice from Taconic were implanted subcutaneously in the right flank with 2.5 x10⁶ LNCaP cells in 0.2 ml of PBS/Matrigel (1:1) per mouse. Mice were weighed and measured for tumor volume using an electronic caliper twice weekly starting three weeks post implantation. Tumor volumes were calculated as height x width x length. Mice with vascularized tumors (determined by the appearance of the tumors) of 180 mm³ were randomized into treatment group and were dosed per individual body weight on Day 0. Mice were monitored for tumor growth around 60 days post dosing and terminated at the end of the study. Mice were euthanized when the tumors reached tumor end point (1500 mm³). The dosing information is summarized in Table 1. Taxotere® was dosed at Q3Dx3 intravenously (iv) through the tail vein. Isotype control antibody Rituxan® and the anti-PSMA 7F12 antibody were dosed intraperitoneally (*ip*) Q3Dx5, followed with Q7Dx6.

**Table 1: Dosing Information**

| **Treatment** | **N per group** | **Taxotere ®Dose iv (mg/kg)** | **Antibody Dose ip (mg/kg)** | **Dosing Schedule** |
|---|---|---|---|---|
| **PBS** | 10 | | | Day 0, 3, 7, 10, 14, 21, 28, 35, 42, 49, 56 |
| **Taxotere® 2** | 10 | 2 | 0 | Day 0, 3; 7 |
| **Taxotere® 4** | 10 | 4 | 0 | Day 0, 3, 7 |
| **Isotype Ab Rituxan®** | 10 | 0 | 30 | Day 0, 3, 7, 10, 14, 21, 28, 35, 42, 49, 56 |
| **7F12 Ab** | 10 | 0 | 30 | Day 0, 3, 7, 10, 14, 21, 28, 35, 42, 49, 56 |
| **Taxotere® 2 + 7F12 Ab** | 10 | 2 | 30 | Day 0, 3, 7 for Taxotere Day 0, 3, 7, 10, 14, 21, 28, 35, 42, 49, 56 |
| **Taxotere 2 + Isotype Rituxan®** | 10 | 2 | 30 | Day 0, 3, 7 for Taxotere Day 0, 3, 7, 10, 14, 21, 28, 35, 42, 49, 56 |
| **Taxotere 4 + 7F12 Ab** | 10 | 4 | 30 | Day 0, 3, 7 for Taxotere Day 0, 3, 7, 10, 14, 21, 28, 35, 42, 49, 56 |
| **Taxotere 4 + Isotype Rituxan®** | 10 | 4 | 30 | Day 0, 3, 7 for Taxotere Day 0, 3, 7, 10, 14, 21, 28, 35, 42, 49, 56 |

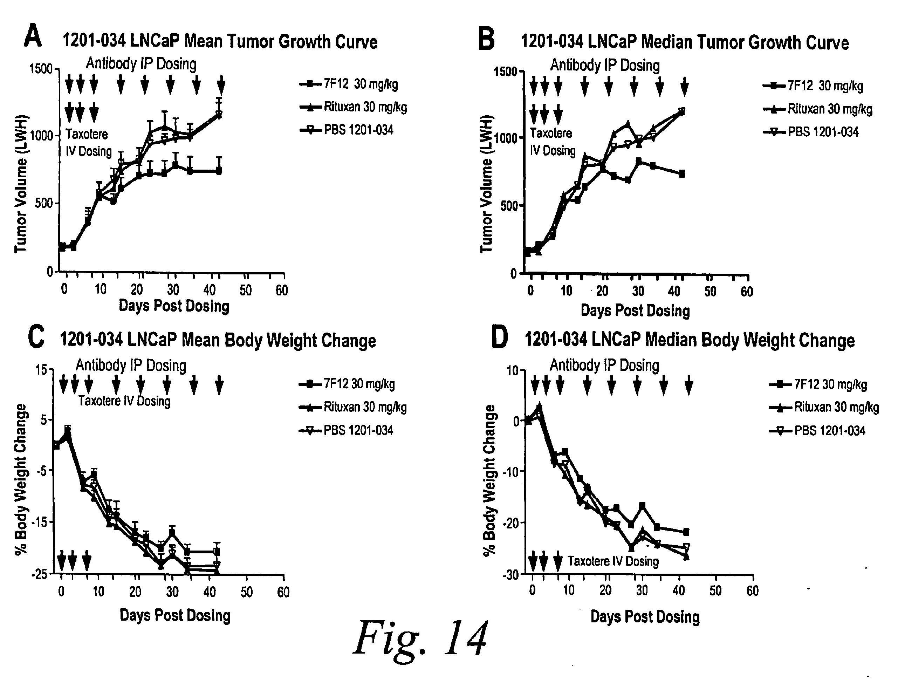

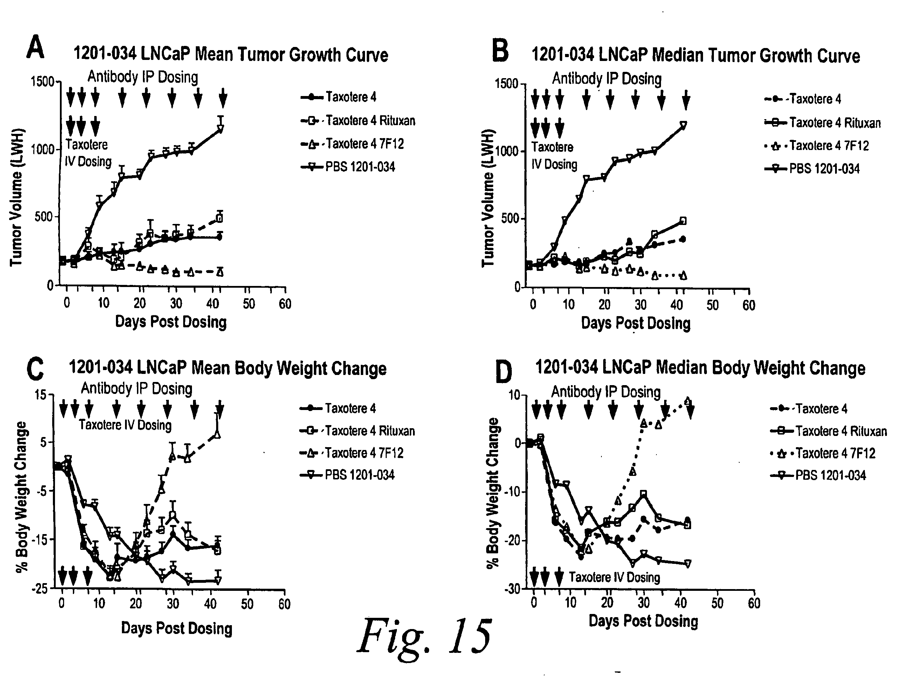

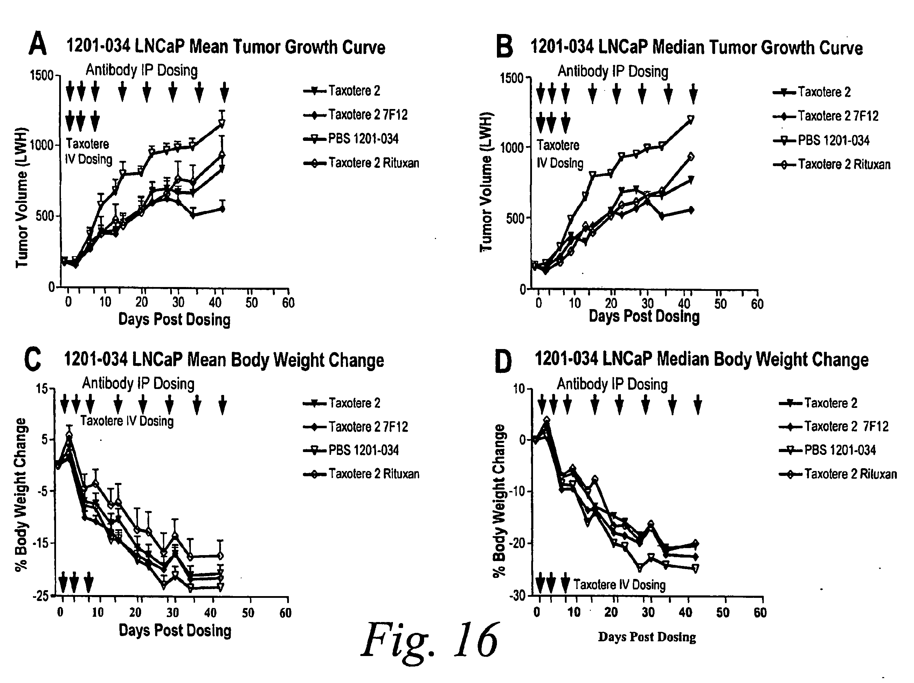

The results of the foregoing experiments are depicted in Figures 13-16. As shown in Figures 13A-13B and Figures 14A-14B, 30 mg/kg of the anti-PSMA 7F12 antibody alone modestly decreased the growth of LNCaP tumors. Taxotere® showed a dose-dependent anti-tumor growth efficacy at the two doses tested (*i.e.,* 2 and 4 mg/kg). Combination therapy of 30 mg/kg of the anti-PSMA 7F12 antibody and 4 mg/kg of Taxotere® showed superior efficacy than each treatment alone and resulted in near complete inhibition of LNCaP tumor growth (see Figures 13A-13B and Figures 15A-15B). As shown in Figures 13C-13D and Figures 15C-15D, this combination regimen also cured the mice of LNCaP-tumor-related cachexia. Combination therapy of 30 mg/kg of the anti-PSMA 7F12 antibody and 2 mg/kg of Taxotere® also showed superior efficacy than each treatment alone (see Figures 13A-13D and Figures 16A-16D). The foregoing data demonstrate an additive and possibly synergistic effect of the anti-PSMA 7F12 antibody in combination with Taxotere® chemotherapy in treating tumors, such as prostate tumors.

### Example 8: Assessment of cell killing of a toxin-conjugated anti-PSMA antibody on prostate cancer cell lines

In this example, anti-PSMA monoclonal antibodies conjugated to a toxin were tested for the ability to kill PSMA+ prostate cancer cell lines in a cell proliferation assay.

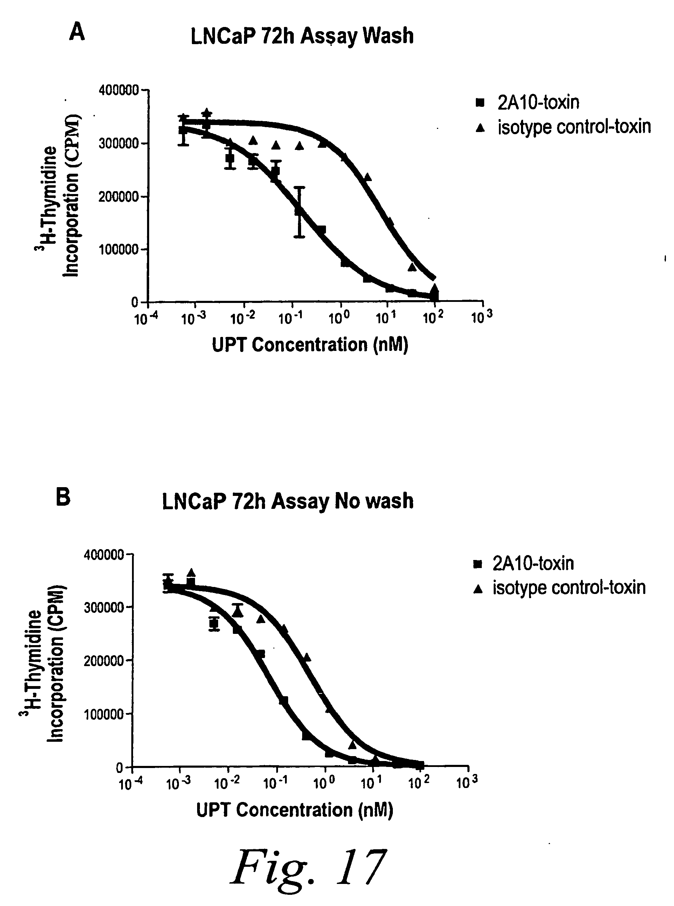

The anti-PSMA HuMAb antibody 2A10 was conjugated to a toxin via a linker, such as a peptidyl, hydrazone or disulfide linker. Examples of toxin compounds that may be conjugated to the antibodies of the current invention are described in the filed application with Attorney Docket No. 04280/100M629US3, filed on September 26, 2005. The PSMA-expressing prostate cancer cell line LNCaP was seeded at 2.5x10⁴ cells/wells in 100 µl wells for 3 hours. An anti-PSMA antibody-toxin conjugate was added to the wells at a starting concentration of 30 nM and titrated down at 1:3 serial dilutions. An isotype control antibody that is non-specific for PSMA was used as a negative control. Plates were allowed to incubate for 72 hours with either a wash at 3 hours or a continuous wash. The plates were then pulsed with 1.0 µCi of ³H-thymidine for 24 hours, harvested and read in a Top Count Scintillation Counter (Packard Instruments, Meriden, CT). The results are shown in Figure 17A (three-hour wash) and 17B (continuous wash). The anti-PSMA antibody 2A10 showed an antibody-toxin concentration dependent decrease in ³H-thymidine incorporation in PSMA-expressing LNCaP prostate cancer cells. The EC₅₀ values for the anti-PSMA antibody 2A10 was 0.157 nM for the wash assay and 0.0643 nM for the continuous wash assay. This data demonstrates that anti-PSMA are cytotoxic to prostate cancer cells when conjugated to a toxin.

### EXAMPLE 9: In Vivo Studies

In this example, anti-PSMA monoclonal antibodies conjugated to a toxin were tested for the ability to kill PSMA+ prostate cancer cell lines *in vivo.*

### A. Treatment of in vivo tumor xenografts

The anti-PSMA HuMAb 2A10 and an isotype control antibody were each buffer exchanged into 0.1M phosphate buffer pH8.0 containing 50mM NaCl and 2mM DTPA, and concentrated to 6mg/ml. Both antibodies were then thiolated by incubation with a 25-fold molar excess of 2-iminothiolane for one hour at room temperature, followed by desalting into 0.1M phosphate buffer pH6.0 containing 50mM NaCl and 2mM DTPA buffer using a Sephadex G-25 column. Thiolated antibodies were then maintained on ice, whilst the number of thiol groups introduced was determined. This was achieved by reaction of a sample of thiolated antibody with dithiodipyridine (DTDP). The absorbance at 280nm was measured to determine the concentration of protein in the samples, and then an aliquot of each sample (0.9ml) was incubated with 0.1ml DTDP (5mM stock solution in ethanol) for 10 minutes at room temperature. Blank samples of buffer alone plus DTDP were incubated alongside. Absorbance at 324nm was measured and the number of thiols present per antibody quantitated using an extinction coefficient for thiopyridine of 19800M⁻¹. In the case of anti-PSMA 5.3 thiols per antibody were introduced, and in the case of the isotype control 6.0.

The thiolated antibodies were then incubated with a 3 fold molar excess of Compound A over the molar concentration of thiol groups. 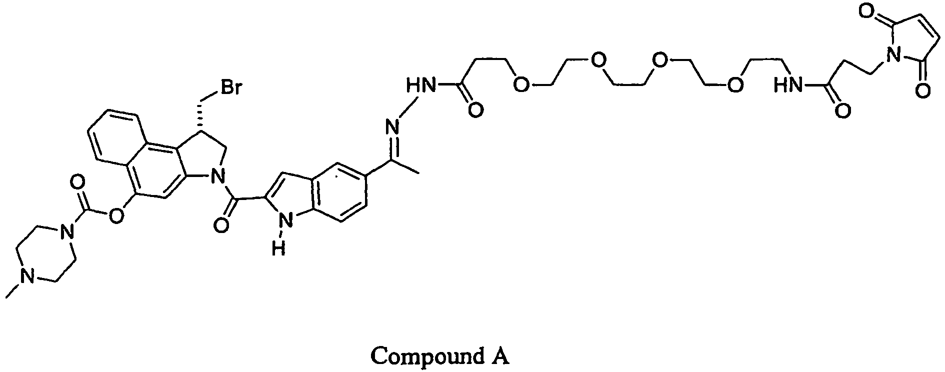 5mM stock solution in DMSO of Compound A was added to the thiolated antibodies along with sufficient DMSO to bring the final concentration of DMSO to 10% (v/v). After incubation at room temperature for 3 hours the pH of the incubation mixture was raised to 7.0 using triethanolamine. The antibody-Compound A conjugates were then purified by size-exclusion chromatography on a Sephacryl S200 column pre-equilibrated with 0.1M phosphate buffer (pH 7.2) containing 50mM NaCl and 5% (v/v) DMSO. Fractions containing monomeric conjugate were collected and pooled. The resulting purified conjugates were then concentrated in a stirred cell under nitrogen, using a 10kDa cut-off membrane. Concentrations and substitution ratios (number of drug molecules attached per antibody molecule) of the conjugates were determined using absorbance at 280nm and 340nm, by reference to the extinction coefficients of both antibody and Compound A at each wavelength as previously measured. Examples of other toxin compounds that may be conjugated to the antibodies of the current invention are described in the co-owned U.S. Patent application with Attorney Docket No. 04280/100M629US3, filed on September 26, 2005.

Anti-tumor efficacy of anti-PSMA (2A10 clone) conjugated to Compound A was tested on LNCaP, which is human prostate carcinoma xenografts, grown in male CB17.SCID mice (available from Taconic, Germantown, NY). LNCaP prostate cancer cells expressing high levels of PSMA were obtained from ATCC (Cat# CRL-1740) and expanded *in vitro* following ATCC instruction. 8 week-old male CB17.SCID mice from Taconic were implanted subcutaneously in the right flank with 2.5 x10⁶ LNCaP cells in 0.2 ml of PBS/Matrigel (1:1) per mouse. Mice were weighed and measured for tumor three dimensionally using an electronic caliper twice weekly starting three weeks post implantation. Individual tumor volume was calculated as height x width x length. Mice with vascularized tumors (determined by appearance of the tumors) of appropriate sizes were randomized into treatment groups and were dosed per individual body weight on Day 0. Mice were monitored for tumor growth around 60 days post dosing and terminated at the end of the study. Mice were euthanized when the tumors reached tumor end point (1500 mm³). The design of this xenograft study is summarized in table 2.

**Table 2. LNCaP Xenograft Study Summary**

| **Treatment** | **Dose (µmole/kg Cytotoxics)** | **N per group** | **Dosing Route** | **Average Tumor Volume at Day-1 (mm³)** |
|---|---|---|---|---|
| **Vehicle** | - | **3** | **ip** | **100** |
| **Isotype Ab-Cmpd A Conjugate** | **0.3** | **3** | **ip** | **100** |
| **2A10-Cmpd A Conjugate** | **0.3** | **3** | **ip** | **100** |

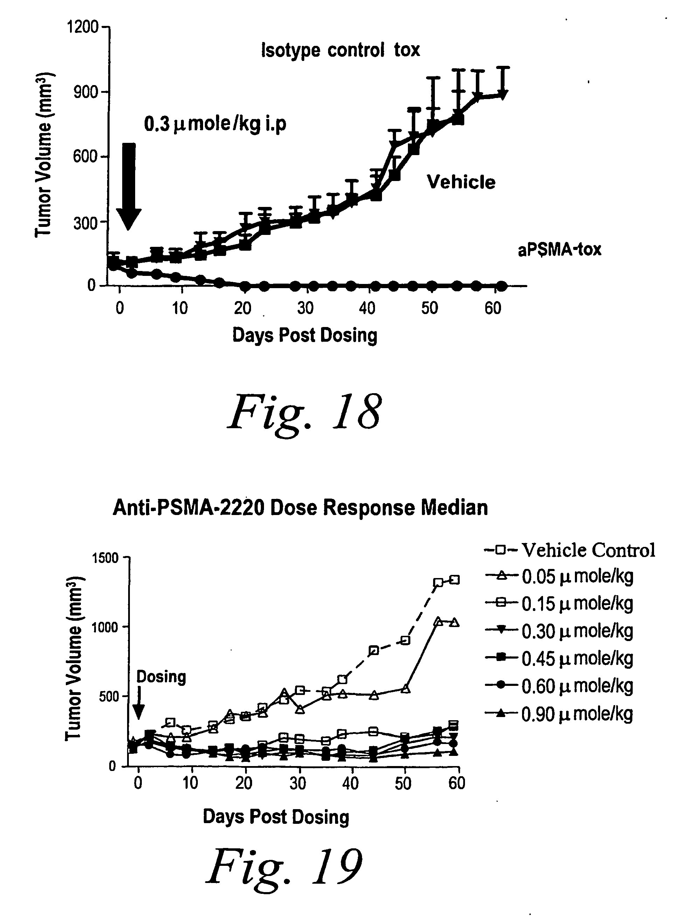

As shown in Fig. 18, 0.3 µmole/kg (referring to the moles of the cytotoxin Compound A) of the 2A10-Compound A conjugate induced complete regression of all three established small LNCaP tumors.

### B. Dose-Response Study

Anti-PSMA (2A10) was buffer exchanged into 0.1M phosphate buffer pH 8.0 containing 50mM NaCl and 2mM DTPA, and concentrated to 5.6mg/ml. Antibody was then thiolated by incubation with a 7.5-fold molar excess of 2-iminothiolane for one hour at room temperature, followed by desalting into 50mM HEPES buffer pH 6.0 containing 5mM glycine, 2mM DTPA and 3% (v/v) glycerol using a Sephadex G-25 column. Thiolated antibody was maintained on ice, whilst the number of thiol groups introduced was determined. This was achieved by reaction of a sample of thiolated antibody with dithiodipyridine (DTDP). The absorbance at 280nm was measured to determine the concentration of protein in the samples, and then an aliquot of each sample (0.9ml) was incubated with 0.1ml DTDP (5mM stock solution in ethanol) for 10 minutes at room temperature. Blank samples of buffer alone plus DTDP were incubated alongside. Absorbance at 324nm was measured and the number of thiols present per antibody quantitated using an extinction coefficient for thiopyridine of 19800 M⁻¹.

The thiolated antibody was then incubated with a 2-fold molar excess of Compound A over the molar concentration of thiol groups. Compound A, 5mM stock solution in 10% (v/v) DMSO/90% (v/v) ethylene glycol dimethyl ether, was added to the thiolated antibody along with sufficient ethylene glycol dimethyl ether to bring the final concentration to 5% (v/v). After incubation at room temperature for 2 hours the antibody-Compound A conjugate was purified by ion-exchange chromatography. Reaction mix was applied to an SP-Sepharose column pre-equilibrated in buffer A (50mM HEPES, 5mM glycine, 3% (v/v) glycerol, pH 6.0). The column was washed with buffer A, then with 95% buffer A, 5% buffer B (50mM HEPES, 1M NaCl, 5mM glycine, 3% (v/v) glycerol, pH 7.2) and then antibody-Compound A conjugate was eluted with 10% buffer B, 90% buffer A. Fractions containing monomeric conjugate were collected and pooled and the pH adjusted to 7.2 by addition of monoethanolamine. The resulting purified conjugate was then dialysed into 50mM HEPES, 100mM NaCl, 5mM glycine, 3% (v/v) glycerol, pH 7.2 and then concentrated in a stirred cell under nitrogen, using a 10kDa cut-off membrane. Concentrations and substitution ratios (number of drug molecules attached per antibody molecule) of the conjugate was determined using absorbance at 280nm and 340nm, by reference to the extinction coefficients of both antibody and Compound A at each wavelength as previously measured. The isotype control (anti-CD70 2H5) conjugate was prepared using the same method except that elution of conjugate from the ion-exchange column was achieved with 15% buffer B, 85% buffer A.

Efficacy and selectivity of the conjugates was determined using LNCaP human prostate carcinoma xenografts grown in male CB17.SCID mice as described above. The design of this xenograft study is summarized in table 3.

**Table 3. LNCaP Xenograft Study Summary**

| **Treatment** | **Dose (µmole/kg Cytotoxin)** | **N per group** | **Dosing Route** | **Average Tumor Volume at Day -1 (mm³)** |
|---|---|---|---|---|
| **Vehicle** | - | **9** | **ip** | **160** |
| **Isotype Ab-CmpdA** | **0.05, 0.15, 0.30, 0.45, 0.60, 0.90** | **9** | **ip** | **160** |
| **2A10-Cmpd A** | **0.05, 0.15, 0.30, 0.45,0.60,0.90** | **9** | **ip** | **160** |

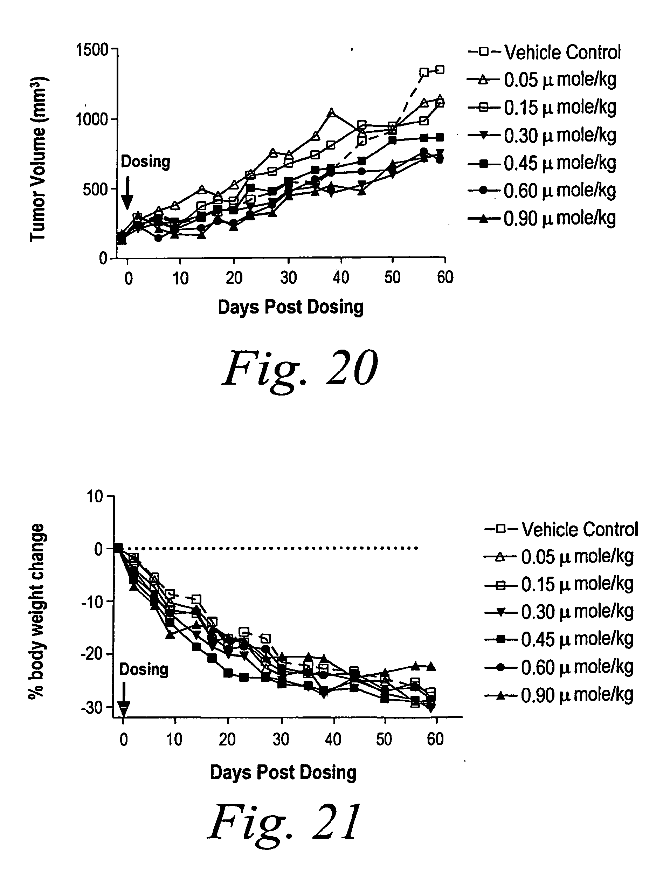

As shown in Table 3 and Figs. 19-20, 0.15 µmole/kg of anti-PSMA-Compound A (Fig. 19) had better anti-tumor efficacy than 0.90 µmole/kg of isotype control-Compound A, indicating at least >6x selectivity (Fig. 20). 0.90 µmole/kg of anti-PSMA-Compound A only showed transient toxicity (Figs. 21-22) and was below the maximum tolerated dose. Therefore, an over 6-fold therapeutic index was identified for anti-PSMA-Compound A in LNCaP-tumor-bearing mice.

### C. Efficacy on Large Tumors

Anti-PSMA (2A10) was buffer exchanged into 0.1M phosphate buffer pH8.0 containing 50mM NaCl and 2mM DTPA, and concentrated to 5.6mg/ml. Antibody was then thiolated by incubation with a 9-fold molar excess of 2-iminothiolane for one hour at room temperature, followed by desalting into 50mM HEPES buffer pH6.0 containing 5mM glycine, 2mM DTPA and 3% (v/v) glycerol using a Sephadex G-25 column. Thiolated antibody was maintained on ice, whilst the number of thiol groups introduced was determined. This was achieved by reaction of a sample of thiolated antibody with dithiodipyridine (DTDP). The absorbance at 280nm was measured to determine the concentration of protein in the samples, and then an aliquot of each sample (0.9ml) was incubated with 0.1ml DTDP (5mM stock solution in ethanol) for 10 minutes at room temperature. Blank samples of buffer alone plus DTDP were incubated alongside. Absorbance at 324nm was measured and the number of thiols present per antibody quantitated using an extinction coefficient for thiopyridine of 19800M⁻¹.

The thiolated antibody was then incubated with a 2-fold molar excess of Compound A over the molar concentration of thiol groups. Compound A, 5mM stock solution in 10% (v/v) DMSO 90% (v/v) ethylene glycol dimethyl ether, was added to the thiolated antibody along with sufficient ethylene glycol dimethyl ether to bring the final concentration to 5% (v/v). After incubation at room temperature for 2 hours the antibody-Compound A conjugate was purified by ion-exchange chromatography. Reaction mix was applied to an SP-Sepharose column pre-equilibrated in 50mM HEPES, 5mM glycine, 3% (v/v) glycerol, pH 6.0 (buffer A). The column was washed with buffer A, then with 95% buffer A, 5% buffer B (50mM HEPES, 1M NaCl, 5mM glycine, 3% (v/v) glycerol, pH 7.2) and then antibody-Compound A conjugate was eluted with 10% buffer B, 90% buffer A. Fractions containing monomeric conjugate were collected and pooled and the pH adjusted to 7.2 by addition of monoethanolamine. The resulting purified conjugate was then dialysed into 50mM HEPES, 100mM NaCl, 5mM glycine, 3% (v/v) glycerol, pH 7.2 and then concentrated in a stirred cell under nitrogen, using a 10kDa cut-off membrane. Concentrations and substitution ratios (number of drug molecules attached per antibody molecule) of the conjugate was determined using absorbance at 280nm and 340nm, by reference to the extinction coefficients of both antibody and Compound A at each wavelength as previously measured. The isotype control (anti-CD70 2H5) conjugate was prepared using the same method except that elution of conjugate from the ion-exchange column was achieved with 15% buffer B, 85% buffer A.

Efficacy and selectivity of the conjugates was determined using LNCaP human prostate carcinoma xenografts grown in male CB17.SCID mice as described above. The design of these xenograft studies is summarized in tables 4 & 5.

**Table 4. LNCaP Xenograft Study Summary**

| **Treatment** | **Dose (µmole/kg Cytotoxin)** | **N per group** | **Dosing Route** | **Average Tumor Volume at Day -1 (mm³)** |
|---|---|---|---|---|
| **Vehicle** | - | **8** | **iv** | **240** |
| **Isotype Ab-Cmpd A** | **0.15** | **8** | **iv** | **240** |
| **2A10-Cmpd A** | **0.15** | **8** | **iv** | **240** |

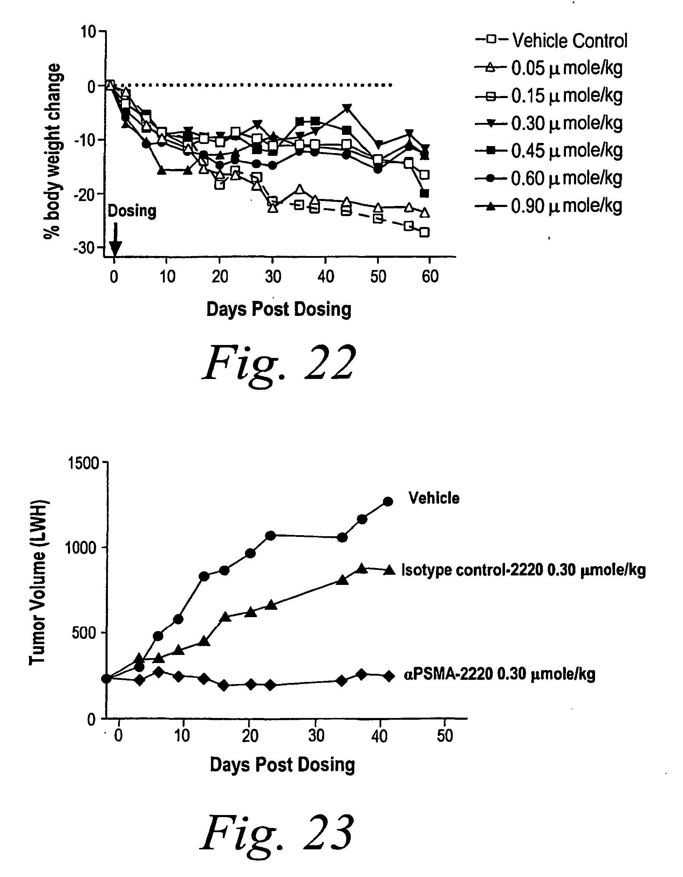

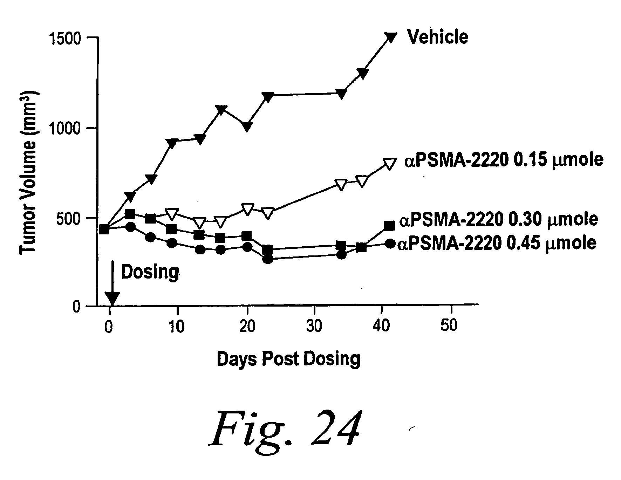

As shown in Table 4 and Fig. 23, a single low dose of 0.15 µmole/kg of anti-PSMA-Compound A greatly inhibited growth of established large LNCaP tumors of average sizes of 240 mm³. In contrast, 0.15 µmole/kg of isotype control-Compound A had minimal anti-tumor efficacy. As shown in Table 5 and Fig. 24, a single dose of 0.30 µmole/lkg of anti-PSMA-Compound A induced regression and inhibited growth of very large LNCaP tumors of average sizes of 430 mm³.

**Table 5. LNCaP Xenograft Study Summary**

| **Treatment** | **Dose (µmole/kg Cytotoxin)** | **N per group** | **Dosing Route** | **Average Tumor Volume at Day -1 (mm³)** |
|---|---|---|---|---|
| **Vehicle** | - | **6** | **ip** | **430** |
| **2A10-Cmpd A** | **0.15, 0.30, 0.45** | **6** | **ip** | **430** |

### SUMMARY OF SEQUENCE LISTING

| SEQ ID NO: | SEQUENCE | SEQ ID NO: | SEQUENCE |
|---|---|---|---|
| 1 | VH a.a. 1C3 | 21 | VK CDR1 a.a. IC3 |
| 2 | VH a.a. 2A10 | 22 | VK CDR1 a.a. 2A10 |
| 3 | VH a.a. 2F5 | 23 | VK CDR1 a.a. 2F5 |
| 4 | VH a.a. 2C6 | 24 | VK CDR1 a.a. 2C6 |
| | | | |
| 5 | VK a.a. 1C3 | 25 | VK CDR2 a.a. 1 C3 |
| 6 | VK a.a. 2A10 | 26 | VK CDR2 a.a. 2A10 |
| 7 | VK a.a. 2F5 | 27 | VK CDR2 a.a. 2F5 |
| 8 | VK a.a. 2C6 | 28 | VK CDR2 a.a. 2C6 |
| | | | |
| 9 | VH CDR1 a.a. 1C3 | 29 | VK CDR3 a.a. 1 C3 |
| 10 | VH CDR1 a.a. 2A10 | 30 | VK CDR3 a.a. 2A10 |
| 11 | VH CDR1 a.a. 2F5 | 31 | VK CDR3 a.a. 2F5 |
| 12 | VH CDR1 a.a. 2C6 | 32 | VK CDR3 a.a. 2C6 |
| | | | |
| 13 | VH CDR2 a.a. 1C3 | 33 | VH n.t. 1C3 |
| 14 | VH CDR2 a.a. 2A10 | 34 | VH n.t. 2A10 |
| 15 | VH CDR2 a.a. 2F5 | 35 | VH n.t. 2F5 |
| 16 | VH CDR2 a.a. 2C6 | 36 | VH n.t. 2C6 |
| | | | |
| 17 | VH CDR3 a.a. 1C3 | 37 | VK n.t. 1C3 |
| 18 | VH CDR3 a.a. 2A10 | 38 | VK n.t. 2A10 |
| 19 | VH CDR3 a.a. 2F5 | 39 | VK n.t. 2F5 |
| 20 | VH CDR3 a.a. 2C6 | 40 | VK n.t. 2C6 |
| | | | |
| 41 | VH 3-30.3 germline a.a. | 43 | VK L18 germline a.a. |
| 42 | VH 5-51 germline a.a. | 44 | VKL6 |
| | | | |
| 45 | JH6b germline a.a. | 47 | JK3 germline a.a. |
| 46 | JK4 germline a.a. | 48 | (Gly₄ -Ser)₃ a.a. |

## Claims

1. An isolated human monoclonal antibody, or antigen-binding portion thereof, that specifically binds prostate specific membrane antigen (PSMA), comprising:
a heavy chain variable region CDR1 comprising SEQ ID NO: 10,
a heavy chain variable region CDR2 comprising SEQ ID NO: 14,
a heavy chain variable region CDR3 comprising SEQ ID NO: 18,
a light chain variable region CDR1 comprising SEQ ID NO: 22,
a light chain variable region CDR2 comprising SEQ ID NO: 26 and
a light chain variable region CDR3 comprising SEQ ID NO: 30.

2. The antibody, or antigen-binding portion thereof, of claim 1, which comprises a heavy chain variable region comprising the amino acid sequence of SEQ ID NO: 2 and a light chain variable region comprising the amino acid sequence of SEQ ID NO: 6.

3. An immunoconjugate comprising the antibody, or antigen-binding portion thereof, of claim 1 or 2, linked to a therapeutic agent.

4. The immunoconjugate of claim 3, wherein the therapeutic agent is a cytotoxin or a radioactive isotope.

5. A bispecific molecule comprising the antibody, or antigen-binding portion thereof, of claim 1 or 2, linked to a second functional moiety having a different binding specificity than said antibody, or antigen binding portion thereof.

6. A composition comprising the antibody, or antigen-binding portion thereof, of claim 1 or 2, the immunoconjugate of claim 3 or 4 or the bispecific molecule of claim 5, and a pharmaceutically acceptable carrier.

7. An isolated nucleic acid molecule encoding the antibody, or antigen-binding portion thereof, of claim 1 or 2.

8. An expression vector comprising the nucleic acid molecule of claim 7.

9. A host cell comprising the expression vector of claim 8.

10. A transgenic mouse comprising human immunoglobulin heavy and light chain transgenes, wherein the mouse expresses the antibody of claim 1 or 2.

11. A hybridoma prepared from the mouse of claim 10, wherein the hybridoma produces said antibody.

12. The antibody, or antigen-binding portion thereof, of claim 1 or 2 for use in a method of inhibiting growth of a tumor in a subject, wherein cells of the tumor or vascular endothelial cells proximate to the tumor express PSMA.

13. The antibody, or antigen-binding portion thereof, of claim 1 or 2, for use in combination with an anti-tumor agent in a method of inhibiting or preventing growth of a tumor in a subject, wherein cells of the tumor or vascular endothelial cells proximate to the tumor express PSMA.

14. The antibody, or antigen-binding portion thereof, for use according to claim 13, wherein administration to said subject of said anti-PSMA antibody, or antigen-binding portion thereof, in combination with said anti-tumor agent, leads to a synergistic effect on the inhibition of the growth of said tumor.

15. The antibody, or antigen-binding portion thereof, for use according to claim 13, wherein said anti-tumor agent causes damage in the tumor mass, thereby leading to a more effective antibody dependent cell-mediated cytotoxicity (ADCC) of the tumor.

16. The antibody, or antigen-binding portion thereof, for use according to any one of claims 12 to 15, wherein the tumor is of a cancer selected from prostate, colon, renal, rectal, urothelial, breast, bladder, liver, pancreas and melanoma.

17. The antibody for use according to claim 13, wherein said anti-tumor agent is a chemotherapeutic agent, an anti-angiogenic agent or an immunomodulatory agent.

18. The antibody for use according to claim 17, wherein, said chemotherapeutic agent is Taxotere® (docetaxel), said anti-angiogenic agent is selected from angiostatin K1-3, Arresten, aaAT, Canstatin, DL-α-Difluoromethyl-ornithine, Endostatin, Fumagillin, Genistein, Minocycline, Staurosporine, Thalidomide, and Tumstatin or said immunomodulatory agent is selected from anti-PD1 antibodies, anti-CTLA-4 antibodies, phosphorothiolate oligodeoxyribonucleotide (1018 ISS), GM-CSF gene vaccines, interleukin-2, interleukin-7 (CYT 99 07), interleukin-12 and interleukin-21.

19. The antibody, or antigen-binding portion thereof, of claim 1 or 2, for use in combination with an anti-tumor agent in a method of inhibiting tumor-related cachexia in a subject or of stimulating antibody dependent cell-mediated cytotoxicity (ADCC) of a tumor in a subject, wherein cells of the tumor or vascular endothelial cells proximate to the tumor express PSMA.

20. A composition comprising the antibody, or antigen-binding portion thereof, of claim 1 or 2and an anti-tumor agent for use in a method of inhibiting or preventing growth of a tumor, or of stimulating antibody dependent cell-mediated cytotoxicity (ADCC) of a tumor.

21. An in vitro method of identifying an anti-tumor agent capable of acting synergistically with the antibody, or antigen-binding portion thereof, of claim 1 or 2in inhibiting or preventing growth of a tumor, wherein cells of the tumor or vascular endothelial cells proximate to the tumor express PSMA, comprising:
contacting an indicator composition with:
(a) a test anti-tumor agent alone,
(b) an anti-PSMA antibody of claims 1 or 2 alone, and
(c) both a test anti-tumor agent and an anti-PSMA antibody of claims 1 or 2; and
comparing the ability of (a) the test anti-tumor agent alone and (b) the anti-PSMA antibody alone to inhibit or prevent growth of a tumor to the ability of (c) both the test anti-tumor agent and the anti-PSMA antibody to inhibit or prevent growth of a tumor, wherein inhibition or prevention of tumor growth by (c) in an amount that is greater than the additive effect of (a) and (b) would lead to the identification of an anti-tumor agent capable of acting synergistically with an anti-PSMA antibody in inhibiting or preventing growth of a tumor.

22. The method of claim 21, wherein the indicator composition is an animal model for a tumor.

## Patentansprüche

1. Isolierter humaner monoklonaler Antikörper oder Antigen-bindender Abschnitt davon, der spezifisch Prostataspezifisches Membranantigen (PSMA) bindet, umfassend:
eine variable CDR1-Region der schweren Kette, umfassend SEQ ID NO:10,
eine variable CDR2-Region der schweren Kette, umfassend SEQ ID NO:14,
eine variable CDR3-Region der schweren Kette, umfassend SEQ ID NO:18,
eine variable CDR1-Region der leichten Kette, umfassend SEQ ID NO:22,
eine variable CDR2-Region der leichten Kette, umfassend SEQ ID NO:26 und
eine variable CDR3-Region der leichten Kette, umfassend SEQ ID NO:30.

2. Antikörper oder Antigen-bindender Abschnitt davon gemäß Anspruch 1, der eine variable Region der schweren Kette umfasst, umfassend die Aminosäuresequenz von SEQ ID NO:2 und der eine variable Region der leichten Kette umfasst, umfassend die Aminosäuresequenz von SEQ ID NO:6.

3. Immunkonjugat, umfassend den Antikörper oder den Antigen-bindenden Abschnitt davon gemäß Anspruch 1 oder 2, verknüpft mit einem therapeutischen Mittel.

4. Immunkonjugat gemäß Anspruch 3, wobei das therapeutische Mittel ein Cytotoxin oder ein radioaktives Isotop ist.

5. Bispezifisches Molekül, umfassend den Antikörper oder den Antigen-bindenden Abschnitt davon gemäß Anspruch 1 oder 2, verknüpft mit einer zweiten funktionalen Einheit, die eine unterschiedliche Bindungsspezifität als der Antikörper oder der Antigen-bindende Abschnitt davon hat.

6. Zusammensetzung, umfassend den Antikörper oder den Antigen-bindenden Abschnitt davon gemäß Anspruch 1 oder 2, das Immunkonjugat gemäß Anspruch 3 oder 4 oder das bispezifische Molekül gemäß Anspruch 5 und einen pharmazeutisch verträglichen Träger.

7. Isoliertes Nukleinsäuremolekül, das den Antikörper oder den Antigen-bindenden Abschnitt davon gemäß Anspruch 1 oder 2 codiert.

8. Expressionsvektor, der das Nukleinsäuremolekül gemäß Anspruch 7 umfasst.

9. Wirtszelle, die den Expressionsvektor gemäß Anspruch 8 umfasst.

10. Transgene Maus, die Transgene der schweren und leichten Kette der humanen Immmunoglobuline umfasst, wobei die Maus den Antikörper gemäß Anspruch 1 oder 2 exprimiert.

11. Hybridom, hergestellt aus der Maus gemäß Anspruch 10, wobei das Hybridom den Antikörper produziert.

12. Antikörper oder Antigen-bindender Abschnitt davon gemäß Anspruch 1 oder 2 zur Verwendung in einem Verfahren zur Inhibition des Wachstums eines Tumors in einem Subjekt, wobei Zellen des Tumors oder vaskuläre Endothelzellen in der Nähe des Tumors PSMA exprimieren.

13. Antikörper oder Antigen-bindender Abschnitt davon gemäß Anspruch 1 oder 2 zur Verwendung in Kombination mit einem Antitumormittel in einem Verfahren zur Inhibition oder Vorbeugung von Wachstum eines Tumors in einem Subjekt, wobei Zellen des Tumors oder vaskuläre Endothelzellen in der Nähe des Tumors PSMA exprimieren.

14. Antikörper oder Antigen-bindender Abschnitt davon zur Verwendung gemäß Anspruch 13, wobei Verabreichung des Anti-PSMA-Antikörpers oder Antigen-bindenden Abschnitts davon in Kombination mit dem Antitumormittel an das Subjekt zu einer synergistischen Wirkung auf die Inhibition des Wachstums des Tumors führt.

15. Antikörper oder Antigen-bindender Abschnitt davon zur Verwendung gemäß Anspruch 13, wobei das Antitumormittel Schaden an der Tumormasse verursacht, und dadurch zu einer wirksameren Antikörper-abhängigen Zell-vermittelten Cytotoxizität (ADCC) des Tumors führt.

16. Antikörper oder Antigen-bindender Abschnitt davon zur Verwendung gemäß einem der Ansprüche 12 bis 15, wobei der Tumor von einem Karzinom ist, ausgewählt aus Prostata, Darm, Niere, Rektal, Urothelial, Brust, Blase, Leber, Bauchspeicheldrüse und Melanom.

17. Antikörper zur Verwendung gemäß Anspruch 13, wobei das Antitumormittel ein chemotherapeutisches Mittel, ein antiangiogenes Mittel oder ein immunmodulatorisches Mittel ist.

18. Antikörper zur Verwendung gemäß Anspruch 17, wobei das chemotherapeutische Mittel Taxotere^{®} (Docetaxel) ist, das anti-angiogene Mittel ausgewählt ist aus Angiostatin K1-3, Arresten, aaAT, Canstatin, DL-α-Difluormethyl-ornithin, Endostatin, Fumagillin, Genistein, Minocyclin, Staurosporin, Thalidomid und Tumstatin oder das immunmodulatorische Mittel ausgewählt ist aus Anti-PD1-Antikörpern, Anti-CTLA-4-Antikörpern, Phosphorothiolatoligodeoxyribonukleotiden (1018 ISS), GM-CSF-Gen-Impfstoffen, Interleukin-2, Interleukin-7 (CYT 99 07), Interleukin-12 und Interleukin-21.

19. Antikörper oder Antigen-bindender Abschnitt davon gemäß Anspruch 1 oder 2 zur Verwendung in Kombination mit einem Antitumormittel in einem Verfahren zur Inhibition Tumorassoziierter Cachexie in einem Subjekt oder zur Stimulierung Antikörper-abhängiger Zell-vermittelter Cytotoxizität (ADCC) eines Tumors in einem Subjekt, wobei Zellen des Tumors oder vaskuläre Endothelzellen in der Nähe des Tumors PSMA exprimieren.

20. Zusammensetzung, umfassend den Antikörper oder den Antigen-bindenden Abschnitt davon gemäß Anspruch 1 oder 2, und ein Antitumormittel zur Verwendung in einem Verfahren zur Inhibition oder Vorbeugung von Wachstum eines Tumors, oder zur Stimulation Antikörper-abhängiger Zell-vermittelter Cytotoxizität (ADCC) eines Tumors.

21. In-vitro-Verfahren zur Identifizierung eines Antitumormittels, das dazu in der Lage ist, synergistisch mit dem Antikörper oder Antigen-bindenden Abschnitt davon gemäß Anspruch 1 oder 2 bei der Inhibition oder Vorbeugung von Wachstum eines Tumors zu wirken, wobei Zellen des Tumors oder vaskuläre Endothelzellen in der Nähe des Tumors PSMA exprimieren, umfassend:
Inkontaktbringen einer Indikatorzusammensetzung mit:
(a) einem Test-Antitumormittel alleine,
(b) einem Anti-PSMA-Antikörper gemäß Anspruch 1 oder 2 alleine und
(c) beidem, einem Test-Antitumormittel und einem Anti-PSMA-Antikörper gemäß Anspruch 1 oder 2; und
Vergleichen der Fähigkeit von (a) dem Test-Antitumormittel alleine und (b) dem Anti-PSMA-Antikörper alleine, Wachstum eines Tumors zu inhibieren oder vorzubeugen, mit der Fähigkeit von (c) beidem, dem Test-Antitumormittel und dem Anti-PSMA-Antikörper, Wachstum eines Tumors zu inhibieren oder vorzubeugen, wobei Inhibition oder Vorbeugung von Tumorwachstum durch (c) in einer Menge, die höher als der additive Effekt von (a) und (b) ist, zu der Identifizierung eines Antitumormittels führt, das dazu in der Lage ist, synergistisch mit einem Anti-PSMA-Antikörper bei der Inhibition oder der Vorbeugung von Wachstum eines Tumors zu wirken.

22. Verfahren gemäß Anspruch 21, wobei die Indikatorzusammensetzung ein Tiermodell für einen Tumor ist.

## Revendications

1. Anticorps monoclonal humain isolé, ou son domaine de liaison à l'antigène, qui se lie spécifiquement à un antigène de membrane spécifique de prostate (antigène PSMA), lequel anticorps comporte :
- une région variable de chaîne lourde CDR1 comprenant la Séquence N° 10,
- une région variable de chaîne lourde CDR2 comprenant la Séquence N° 14,
- une région variable de chaîne lourde CDR3 comprenant la Séquence N° 18,
- une région variable de chaîne légère CDR1 comprenant la Séquence N° 22,
- une région variable de chaîne légère CDR2 comprenant la Séquence N° 26,
- et une région variable de chaîne légère CDR3 comprenant la Séquence N° 30.

2. Anticorps, ou son domaine de liaison à l'antigène, conforme à la revendication 1, qui comprend une région variable de chaîne lourde comprenant la séquence d'acides aminés donnée en tant que Séquence N° 2 et une région variable de chaîne légère comprenant la séquence d'acides aminés donnée en tant que Séquence N° 6.

3. Immuno-conjugué comprenant un anticorps ou son domaine de liaison à l'antigène, conforme à la revendication 1 ou 2, relié à un agent thérapeutique.

4. Immuno-conjugué conforme à la revendication 3, dans lequel l'agent thérapeutique est une cytotoxine ou un isotope radioactif.

5. Molécule bispécifique comprenant un anticorps ou son domaine de liaison à l'antigène, conforme à la revendication 1 ou 2, relié à une deuxième entité fonctionnelle, qui possède une spécificité de liaison différente de celle dudit anticorps, ou au domaine de liaison à l'antigène de cette entité.

6. Composition comprenant un anticorps ou son domaine de liaison à l'antigène, conforme à la revendication 1 ou 2, un immuno-conjugué conforme à la revendication 3 ou 4, ou une molécule bispécifique conforme à la revendication 5, et un véhicule pharmacologiquement admissible.

7. Molécule d'acide nucléique isolé codant un anticorps, ou son domaine de liaison à l'antigène, conforme à la revendication 1 ou 2.

8. Vecteur d'expression comprenant une molécule d'acide nucléique conforme à la revendication 7.

9. Cellule hôte comprenant un vecteur d'expression conforme à la revendication 8.

10. Souris transgénique portant des transgènes de chaîne lourde et de chaîne légère d'immunoglobuline humaine, laquelle souris exprime un anticorps conforme à la revendication 1 ou 2.

11. Hybridome préparé à partir d'une souris conforme à la revendication 10, lequel hybridome produit ledit anticorps.

12. Anticorps ou son domaine de liaison à l'antigène, conforme à la revendication 1 ou 2, pour utilisation dans un procédé visant à inhiber la croissance d'une tumeur chez un sujet où des cellules de la tumeur, ou des cellules endothéliales vasculaires proches de la tumeur, expriment l'antigène PSMA.

13. Anticorps ou son domaine de liaison à l'antigène, conforme à la revendication 1 ou 2, pour utilisation, en association avec un agent antitumoral, dans un procédé visant à inhiber ou prévenir la croissance d'une tumeur chez un sujet où des cellules de la tumeur, ou des cellules endothéliales vasculaires proches de la tumeur, expriment l'antigène PSMA.

14. Anticorps ou son domaine de liaison à l'antigène pour utilisation conforme à la revendication 13, l'administration audit sujet dudit anticorps anti-PSMA ou de son domaine de liaison à l'antigène, en association avec ledit agent antitumoral, conduisant à un effet de synergie sur l'inhibition de la croissance de ladite tumeur.

15. Anticorps ou son domaine de liaison à l'antigène pour utilisation conforme à la revendication 13, ledit agent antitumoral causant des dommages dans la masse de la tumeur et conduisant par là à une cytotoxicité à médiation cellulaire dépendant des anticorps (ADCC) plus efficace au niveau de la tumeur.

16. Anticorps ou son domaine de liaison à l'antigène pour utilisation conforme à l'une des revendications 12 à 15, la tumeur étant un cancer choisi parmi les cancers de la prostate, du côlon, du rein, du rectum, du sein, de la vessie, du foie ou du pancréas, un cancer urothélial et un mélanome.

17. Anticorps pour utilisation conforme à la revendication 13, ledit agent antitumoral étant un agent chimiothérapeutique, un agent anti-angiogenèse ou un agent immuno-modulateur.

18. Anticorps pour utilisation conforme à la revendication 17, ledit agent chimiothérapeutique étant du Taxotère^{®} (docetaxel), ledit agent anti-angiogenèse étant choisi parmi les angiostatine K1-3, arrestène, aaAT, canstatine, DL-α-difluorométhyl-ornithine, endostatine, fumagilline, génistéine, minocycline, staurosporine, thalidomide et tumstatine, ou ledit agent immuno-modulateur étant choisi parmi les anticorps anti-PD1, anticorps anti-CTLA-4, oligodésoxyribonucléotide phosphorothiolate (1018 ISS), vaccins à base de gène de facteur GM-CSF, interleukine-2, interleukine-7 (CYT 9907), interleukine-12 et interleukine-21.

19. Anticorps ou son domaine de liaison à l'antigène, conforme à la revendication 1 ou 2, pour utilisation, en association avec un agent antitumoral, dans un procédé visant à inhiber une cachexie liée à une tumeur chez un sujet ou à stimuler une cytotoxicité à médiation cellulaire dépendant des anticorps (ADCC) au niveau d'une tumeur chez un sujet, où des cellules de la tumeur, ou des cellules endothéliales vasculaires proches de la tumeur, expriment l'antigène PSMA.

20. Composition comprenant un anticorps ou son domaine de liaison à l'antigène, conforme à la revendication 1 ou 2, et un agent antitumoral, pour utilisation dans un procédé visant à inhiber ou prévenir la croissance d'une tumeur ou à stimuler une cytotoxicité à médiation cellulaire dépendant des anticorps (ADCC) au niveau d'une tumeur.

21. Procédé *in vivo* d'identification d'un agent antitumoral capable d'agir en synergie avec un anticorps ou son domaine de liaison à l'antigène, conforme à la revendication 1 ou 2, pour inhiber ou prévenir la croissance d'une tumeur où des cellules de la tumeur, ou des cellules endothéliales vasculaires proches de la tumeur, expriment l'antigène PSMA, lequel procédé comporte le fait de mettre une composition indicatrice en contact avec
a) l'agent antitumoral testé, seul,
b) un anticorps anti-PSMA conforme à la revendication 1 ou 2, seul,
c) et à la fois l'agent antitumoral testé et l'anticorps anti-PSMA conforme à la revendication 1 ou 2,
et le fait de comparer les capacités qu'ont (a) l'agent antitumoral testé, seul et (b) l'anticorps anti-PSMA conforme à la revendication 1 ou 2, seul, à inhiber ou prévenir la croissance d'une tumeur, avec la capacité qu'ont ensemble (c) l'agent antitumoral testé et l'anticorps anti-PSMA conforme à la revendication 1 ou 2 à inhiber ou prévenir la croissance d'une tumeur, étant entendu qu'une inhibition ou une prévention de la croissance d'une tumeur à un degré plus grand, dans le cas (c), que la somme des effets observés dans les cas (a) et (b) conduit à l'identification d'un agent antitumoral capable d'agir en synergie avec un anticorps anti-PSMA pour inhiber ou prévenir la croissance d'une tumeur.

22. Procédé conforme à la revendication 21, dans lequel la composition indicatrice est un modèle animal de tumeur.
